# EUROPEAN PATENT SPECIFICATION

(11) **EP 2 658 527 B1**
(45) Date of publication and mention of the grant of the patent: **30.11.2022**
(21) Application number: 11852627.6
(22) Date of filing: 29.12.2011
(51) Int. Cl.: A61L 29/16

(54) **NANOPARTICLE AND SURFACE-MODIFIED PARTICULATE COATINGS, COATED BALLOONS, AND METHODS THEREFORE**
NANOPARTIKEL UND OBERFLÄCHENMODIFIZIERTE PARTIKELBESCHICHTUNGEN, BESCHICHTETE BALLONS UND VERFAHREN DAFÜR
NANOPARTICULES ET REVÊTEMENTS PARTICULAIRES MODIFIÉS EN SURFACE, BALLONNETS REVÊTUS ET PROCÉDÉS ASSOCIÉS

(30) Priority: 30.12.2010 US 201061428785 P
(43) Date of publication of application: 06.11.2013
(73) Proprietor: Micell Technologies, Inc., Durham, NC 27713 (US)
(72) Inventor: MCCLAIN, James B., Raleigh, NC 27613 (US); TAYLOR, Charles Douglas, Franklinton, NC 27525 (US); ZANI, Brett G., Arlington, MA 02747 (US); KIORPES, Timothy Charles, Doylestown, PA 18902 (US)
(74) Representative: Wichmann, Hendrik
(86) International application number: PCT/US2011/067921
(87) International publication number: WO 2012/092504

(56) References cited:
- WO-A1-2009/051614
- WO-A1-2010/136604
- US-A1- 2004 157 789
- US-A1- 2005 048 121
- US-A1- 2006 160 455
- US-A1- 2009 186 069
- US-A1- 2010 196 482
- US-A1- 2010 241 220

## Description

### BACKGROUND OF THE INVENTION

Drug-coated balloons may be used to address the drawbacks of bare balloons, of bare stents, or of drug coated stents to treat restenosis and to promote healing of the vessel after opening the blockage by PCI/stenting. Such a device is disclosed in WO 2010/136604 A1. Some current drug eluting stents can have physical, chemical and therapeutic legacy in the vessel over time. Others may have less legacy, bur are not optimized for thickness, deployment flexibility, access to difficult lesions, and minimization of vessel wall intrusion. Infusion techniques delivering nanoparticles to a treatment site can have undesired results systemically or fail to be delivered to the treatment site. There is a need for medical device technology that can rapidly, efficiently, reproducibly and safely transfer a drug from the surface of a percutaneous medical device (a coating) onto/into a specific treatment site in the body.

### SUMMARY OF THE INVENTION

Provided herein is a device comprising: a medical device for delivering nanoparticles of an active agent to a treatment site; and a removable coating on the medical device comprising the active agent nanoparticles, wherein the active agent is a macrolide immunosuppressive drug, at least 95% of which is in crystalline form, and which active agent nanoparticles are, on average, at most 1 micrometer, at least a portion of the coating being deliverable to the treatment site which portion releases active agent nanoparticles into the treatment site over at least about 1 day, and wherein preparing the coating comprises depositing poly(lactide-co-glycolide) (PLGA), followed by depositing the active agent nanoparticles, wherein the coating further comprises a cationic binding agent in a target ratio of active agent: cationic binding agent of 1:1 to 10:1, wherein the actual ratio on the device is ±30% of the target ratio.

The active agent devices is a macrolide immunosuppressive drug. The active agent may be selected from sirolimus, a prodrug, a derivative, an analog, a hydrate, an ester, and a salt thereof. The nanoparticles may be, on average, at least one of: at most 1 micrometer, about 1 micrometer, below about 1 micrometer, below about 750 nanometers (nm), below about 500 nanometers, about 100 nm to about 1 micrometer, about 300 nm to about 1 micrometer, about 100 nm to about 300 nm, about 300 nm to about 500 nm, below about 300nm, below about 100nm, and between about 50nm and about 300 nm.

In devices provided herein the polymer comprises PLGA. The PLGA may have at least one of: a MW of about 30KDa and a Mn of about 15KDa, a Mn of about 10KDa to about 25 KDa, and a MW of about 15 KDa to about 40KDa.

In some embodiments of the devices provided herein the coating portion delivered to the treatment site releases nanoparticles to the treatment site over at least one of: about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days.

In some embodiments of the devices provided herein the treatment site is a vessel wall.

In some embodiments of the devices provided herein the medical device comprises a balloon. In some embodiments the medical device is a balloon of a balloon catheter.

A method may comprise depositing a second polymer on the medical device following depositing the nanoparticles. In some embodiments, a second polymer is deposited on the medical device following the deposition of the nanoparticles. In some embodiments, the coating comprises a second polymer. The second polymer may comprise PLGA. The PLGA has at least one of: a MW of about 30KDa and a Mn of about 15KDa, a Mn of about 10KDa to about 25 KDa, and a MW of about 15 KDa to about 40KDa. In some embodiments depositing the second polymer on the device uses at least one of a RESS coating process, an eSTAT coating process, a dip coating process, and a spray coating process.

In some embodiments of the devices provided herein the coating comprises a positive surface charge on a surface of the coating configured to contact the treatment site.

In some embodiments of the devices provided herein the coating comprises a surfactant. In some embodiments the surfactant is cationic. In some embodiments the surfactant comprises at least one of a primary amine having pH < 10, and a secondary amine having pH < 4. In some embodiments surfactant comprises octenidine dihydrochloride. In some embodiments the surfactant comprises a permanently charged quaternary ammonium cation. In some embodiments the permanently charged quaternary ammonium cation comprises at least one of: an Alkyltrimethylammonium salt such as cetyl trimethylammonium bromide (CTAB), hexadecyl trimethyl ammonium bromide, cetyl trimethylammonium chloride (CTAC); Cetylpyridinium chloride (CPC); Polyethoxylated tallow amine (POEA); Benzalkonium chloride (BAC); Benzethonium chloride (BZT); 5-Bromo-5-nitro-1,3-dioxane; Dimethyldioctadecylammonium chloride; and Dioctadecyldimethylammonium bromide (DODAB). In some embodiments the surfactant comprises at least one of: didodecyldimethylammonium bromide (DMAB), linear isoform Polyethylenimine (linear PEI), Branched Low MW Polyethylenimine (PEI) (of about <25KDa), Branched Low MW Polyethylenimine (PEI) (of about <15KDa), Branched Low MW Polyethylenimine (PEI) (of about <10KDa), Branched High MW Polyethylenimine (of about >/=25 KDa), Poly-L-Arginine (average or nominal MW of about 70,000 Da), Poly-L-Arginine (average or nominal MW > about 50,000 Da), Poly-L-Arginine (average or nominal MW of about 5,000 to about 15,000 Da), Poly-L-Lysine (average or nominal MW of about 28,200 Da), Poly-L-Lysine (average or nominal MW of about 67,000 Da), Poly Histidine, Ethylhexadecyldimethylammonium Bromide, Dodecyltrimethyl Ammonium Bromide, Tetradodecylammonium bromide, Dimethylditetradecyl Ammonium bromide, Tetrabutylammonium iodide, DEAE-Dextran hydrochloride, and Hexadimethrine Bromide.

In some embodiments of the devices provided herein the surfactant and the nanoparticles are mixed, lyophilized, and deposited together on the device.

In some embodiments of the devices provided herein the surfactant is deposited on a balloon after the nanoparticles are deposited thereon.

The positive surface charge may be about 20 mV to about 40mV. The positive surface charge may be at least one of: at least about 1 mV, over about 1 mV, at least about 5 mV, at least about 10 mV, about 10 mV to about 50 mV, about 20 mV to about 50 mV, about 10 mV to about 40 mV, about 30 mV to about 40 mV, about 20 mV to about 30 mV, and about 25 mV to about 35 mV.

A method may comprise forming a coating on a medical device comprising depositing a polymer on the medical device using an RESS process mixing a surfactant and nanoparticles of an active agent to prepare a agent-surfactant mixture, lyophilizing the agent-surfactant mixture and depositing the agent-surfactant mixture on the medical device using an eSTAT process.

The coating may release the nanoparticles into a treatment site over at least one of: about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days.

The devices provided herein may comprise depositing a second polymer on the medical device following depositing the agent-surfactant mixture on the device. The second polymer may comprise PLGA. The PLGA may have at least one of: a MW (weight average molecular weight) of about 30KDa and a Mn (number average molecular weight) of about 15KDa, a Mn of about 10KDa to about 25 KDa, and a MW of about 15 KDa to about 40KDa. Depositing the second polymer on the medical device may use at least one of a RESS coating process, an eSTAT coating process, a dip coating process, and a spray coating process.

Provided herein is a coated medical device as defined in the claims comprising a medical device for delivering nanoparticles of an active agent to a treatment site; and a coating on the medical device comprising a polymer, a surfactant, and the nanoparticles, wherein the coating is prepared by forming a nanoemulsion comprising the nanoparticles, PLGA, and a surfactant (wherein this method is not according to the invention), and wherein the coated medical device delivers a coating portion to the treatment site, which portion releases the nanoparticles into the treatment site over at least about 1 day.

Provided herein is a coating for a medical device, which is not according to the invention, comprising a polymer, a surfactant, and nanoparticles of an active agent, wherein the coating is prepared by forming a nanoemulsion comprising the nanoparticles, polymer, and a surfactant, wherein the device delivers at least a portion of the coating to a treatment site which releases the nanoparticles into the treatment site over at least about 1 day.

A method (not according the the invention) may comprise forming a coating on a medical device comprising providing an emulsion of a polymer, nanoparticles of an active agent, and a surfactant, depositing the emulsion on the medical device, wherein the coating delivers the nanoparticles to a treatment site over at least about 1 day.

The active agent in the embodiments of the devices provided herein comprises a macrolide immunosuppressive drug. The active agent may be selected from sirolimus, a prodrug, a derivative, an analog, a hydrate, an ester, and a salt thereof. A portion of the nanoparticles may be in crystalline form. The nanoparticles may be, on average, at least one of: at most 1 micrometer, about 1 micrometer, below about 1 micrometer, below about 750 nanometers (nm), below about 500 nanometers, about 100 nm to about 1 micrometer, about 300 nm to about 1 micrometer, about 100 nm to about 300 nm, about 300 nm to about 500 nm, below about 300nm, below about 100nm, and between about 50nm and about 300 nm.

In the embodiments of the devices provided herein the polymer comprises PLGA. The PLGA may have at least one of: a MW of about 30KDa and a Mn of about 15KDa, a Mn of about 10KDa to about 25 KDa, and a MW of about 15 KDa to about 40KDa.

In some embodiments of the devices provided herein the coating portion delivered to the treatment site releases nanoparticles to the treatment site over at least one of: about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days.

In some embodiments of the devices provided herein the treatment site is a vessel wall.

In some embodiments of the devices provided herein the medical device comprises a balloon. In some embodiments the medical device is a balloon of a balloon catheter.

A method may comprise depositing a second polymer on the medical device following depositing the nanoparticles. In some embodiments, a second polymer is deposited on the medical device following the deposition of the nanoparticles. In some embodiments, the coating comprises a second polymer. The second polymer may comprise PLGA. The PLGA has at least one of: a MW of about 30KDa and a Mn of about 15KDa, a Mn of about 10KDa to about 25 KDa, and a MW of about 15 KDa to about 40KDa. In some embodiments depositing the second polymer on the device uses at least one of a RESS coating process, an eSTAT coating process, a dip coating process, and a spray coating process.

In some embodiments of the devices provided herein the coating comprises a positive surface charge on a surface of the coating configured to contact the treatment site.

In some embodiments of the devices provided herein the coating comprises a surfactant. In some embodiments the surfactant is cationic. In some embodiments the surfactant comprises at least one of a primary amine having pH < 10, and a secondary amine having pH < 4. In some embodiments surfactant comprises octenidine dihydrochloride. In some embodiments the surfactant comprises a permanently charged quaternary ammonium cation. In some embodiments the permanently charged quaternary ammonium cation comprises at least one of: an Alkyltrimethylammonium salt such as cetyl trimethylammonium bromide (CTAB), hexadecyl trimethyl ammonium bromide, cetyl trimethylammonium chloride (CTAC); Cetylpyridinium chloride (CPC); Polyethoxylated tallow amine (POEA); Benzalkonium chloride (BAC); Benzethonium chloride (BZT); 5-Bromo-5-nitro-1,3-dioxane; Dimethyldioctadecylammonium chloride; and Dioctadecyldimethylammonium bromide (DODAB). In some embodiments the surfactant comprises at least one of: didodecyldimethylammonium bromide (DMAB), linear isoform Polyethylenimine (linear PEI), Branched Low MW Polyethylenimine (PEI) (of about <25KDa), Branched Low MW Polyethylenimine (PEI) (of about <15KDa), Branched Low MW Polyethylenimine (PEI) (of about <10KDa), Branched High MW Polyethylenimine (of about >/=25 KDa), Poly-L-Arginine (average or nominal MW of about 70,000 Da), Poly-L-Arginine (average or nominal MW > about 50,000 Da), Poly-L-Arginine (average or nominal MW of about 5,000 to about 15,000 Da), Poly-L-Lysine (average or nominal MW of about 28,200 Da), Poly-L-Lysine (average or nominal MW of about 67,000 Da), Poly Histidine, Ethylhexadecyldimethylammonium Bromide, Dodecyltrimethyl Ammonium Bromide, Tetradodecylammonium bromide, Dimethylditetradecyl Ammonium bromide, Tetrabutylammonium iodide, DEAE-Dextran hydrochloride, and Hexadimethrine Bromide.

A method may comprise depositing the emulsion on the medical device uses at least one of a RESS coating process, an eSTAT coating process, a dip coating process, and a spray coating process.

Provided herein is a coated medical device comprising: a medical device for delivering encapsulated active agent nanoparticles to a treatment site; and a coating on the medical device comprising the encapsulated active agent nanoparticles wherein the encapsulated active agent nanoparticles comprise active agent nanoparticles encapsulated in a polymer, and wherein the encapsulated active agent nanoparticles have a positive surface charge.

Provided herein is a coating for a medical device comprising encapsulated active agent nanoparticles comprising active agent nanoparticles of encapsulated in a polymer, wherein the encapsulated active agent nanoparticles have a positive surface charge, and wherein the coating delivers active agent nanoparticles to a treatment site over at least about 1 day.

A method may comprise forming a coating on a medical device comprising providing encapsulated active agent nanoparticles comprising a polymer and active agent nanoparticles, wherein the encapsulated active agent nanoparticles have a positive surface charge, depositing the encapsulated active agent nanoparticles on the medical device, wherein the coating delivers the active agent nanoparticles to the treatment site over at least about 1 day.

In the embodiments of the devices according to the invention the polymer comprises PLGA. The PLGA may have at least one of: a MW of about 30KDa and a Mn of about 15KDa, a Mn of about 10KDa to about 25 KDa, and a MW of about 15 KDa to about 40KDa.

In some embodiments of the devices provided herein the coating comprises a positive surface charge. The positive surface charge may be about 20 mV to about 40mV. The positive surface charge may be at least one of: at least about 1 mV, over about 1 mV, at least about 5 mV, at least about 10 mV, about 10 mV to about 50 mV, about 20 mV to about 50 mV, about 10 mV to about 40 mV, about 30 mV to about 40 mV, about 20 mV to about 30 mV, and about 25 mV to about 35 mV.

In some embodiments of the devices provided herein, the w/w percent of active agent in the encapsulated active agent nanoparticles is about 5%. In some embodiments of the devices provided herein, the w/w percent of active agent in the encapsulated active agent nanoparticles is about 10-25%.

In some embodiments of the devices provided herein, at least a portion of the encapsulated active agent nanoparticles are nanospheres. At least a portion of the encapsulated active agent nanoparticles may be at least one of: a discoidal shape, a hemispherical shape, a cylindrical shape, a conical shape, a nanoreef shape, a nanobox shape, a cluster shape, a nanotube shape, a whisker shape, a rod shape, a fiber shape, a cup shape, a jack shape, a hexagonal shape, an ellipsoid shape, an oblate ellipsoid shape, a prolate ellipsoid shape, a torus shape, a spheroid shape, a taco-like shape, a bullet shape, a barrel shape, a lens shape, a capsule shape, a pulley wheel shape, a circular disc shape, a rectangular disc shape, a hexagonal disc shape, a flying saucer-like shape, a worm shape, a ribbon-like shape, and a ravioli-like shape.

The active agent the devices provided herein comprises a macrolide immunosuppressive drug. The active agent may be selected from sirolimus, a prodrug, a derivative, an analog, a hydrate, an ester, and a salt thereof. A portion of at least 95% of the nanoparticles is in crystalline form. The active agent nanoparticles are at most 1 micrometer, about 1 micrometer, below about 1 micrometer, below about 750 nanometers (nm), below about 500 nanometers, about 100 nm to about 1 micrometer, about 300 nm to about 1 micrometer, about 100 nm to about 300 nm, about 300 nm to about 500 nm, below about 300nm, below about 100nm, and between about 50nm and about 300 nm. The encapsulated active agent nanoparticles are on average, at least one of: at most 1 micrometer, about 1 micrometer, below about 1 micrometer, below about 750 nanometers (nm), below about 500 nanometers, about 100 nm to about 1 micrometer, about 300 nm to about 1 micrometer, about 100 nm to about 300 nm, about 300 nm to about 500 nm, below about 300nm, below about 100nm, and between about 50nm and about 300 nm.

In the devices provided herein the coating delivers the active agent nanoparticles to the treatment site over at least about 1 day. In some embodiments of the devices provided herein the coating delivers the active agent nanoparticles to the treatment site over at least one of: about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days.

In some embodiments of the devices provided herein the treatment site is a vessel wall.

In some embodiments of the devices provided herein the coating comprises a positive surface charge on a surface of the coating configured to contact the treatment site.

In some embodiments of the devices provided herein the encapsulated active agent nanoparticles are micelles.

In some embodiments of the devices provided herein the medical device comprises a balloon. In some embodiments the medical device is a balloon of a balloon catheter.

In some embodiments of the devices provided herein depositing the encapsulated active agent nanoparticles comprises using an eSTAT process. In some embodiments of the devices provided herein depositing a second polymer on the medical device following depositing the encapsulated active agent nanoparticles on the medical device.

In some embodiments of the devices provided herein the second polymer comprises PLGA. The PLGA may have at least one of: a MW of about 30KDa and a Mn of about 15KDa, a Mn of about 10KDa to about 25 KDa, and a MW of about 15 KDa to about 40KDa. Depositing the second polymer on the medical device may use at least one of a RESS coating process, an eSTAT coating process, a dip coating process, and a spray coating process.

Provided herein is a coated medical device comprising: a medical device for delivering nanoparticles of an active agent to a treatment site; and a coating on the device comprising the active agent nanoparticles, wherein the coated medical device delivers at least a portion of the coating to the treatment site which portion releases active agent nanoparticles into the treatment site over at least about 1 day.

Provided herein is a coating for a medical device comprising nanoparticles of an active agent, wherein the coating delivers the nanoparticles into a treatment site over at least about 1 day.

A method may comprise forming coating on a medical device with nanoparticles of an active agent comprising depositing the nanoparticles on the medical device using an eSTAT process.

The active agent in the embodiments of the devices provided herein comprises a macrolide immunosuppressive drug. The active agent may be selected from sirolimus, a prodrug, a derivative, an analog, a hydrate, an ester, and a salt thereof. A portion of at least 95% of the nanoparticles is in crystalline form. The nanoparticles may be, on average, at least one of: at most 1 micrometer, about 1 micrometer, below about 1 micrometer, below about 750 nanometers (nm), below about 500 nanometers, about 100 nm to about 1 micrometer, about 300 nm to about 1 micrometer, about 100 nm to about 300 nm, about 300 nm to about 500 nm, below about 300nm, below about 100nm, and between about 50nm and about 300 nm.

In some embodiments of the devices provided herein the coating portion delivered to the treatment site releases nanoparticles into the treatment site over at least one of: about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days.

In some embodiments of the devices provided herein the treatment site is a vessel wall.

In some embodiments of the devices provided herein the medical device comprises a balloon. In some embodiments the medical device is a balloon of a balloon catheter.

Some embodiments of the devices provided comprise depositing a polymer on the medical device following depositing the nanoparticles. The polymer may comprise PLGA. The PLGA has at least one of: a MW of about 30KDa and a Mn of about 15KDa, a Mn of about 10KDa to about 25 KDa, and a MW of about 15 KDa to about 40KDa. In some embodiments depositing the polymer on the device uses at least one of a RESS coating process, an eSTAT coating process, a dip coating process, and a spray coating process.

In some embodiments of the devices provided herein the coating comprises a positive surface charge on a surface of the coating configured to contact the treatment site.

In some embodiments of the devices provided herein the coating comprises a surfactant. In some embodiments the surfactant is cationic. In some embodiments the surfactant comprises at least one of a primary amine having pH < 10, and a secondary amine having pH < 4. In some embodiments surfactant comprises octenidine dihydrochloride. In some embodiments the surfactant comprises a permanently charged quaternary ammonium cation. In some embodiments the permanently charged quaternary ammonium cation comprises at least one of: an Alkyltrimethylammonium salt such as cetyl trimethylammonium bromide (CTAB), hexadecyl trimethyl ammonium bromide, cetyl trimethylammonium chloride (CTAC); Cetylpyridinium chloride (CPC); Polyethoxylated tallow amine (POEA); Benzalkonium chloride (BAC); Benzethonium chloride (BZT); 5-Bromo-5-nitro-1,3-dioxane; Dimethyldioctadecylammonium chloride; and Dioctadecyldimethylammonium bromide (DODAB). In some embodiments the surfactant comprises at least one of: didodecyldimethylammonium bromide (DMAB), linear isoform Polyethylenimine (linear PEI), Branched Low MW Polyethylenimine (PEI) (of about <25KDa), Branched Low MW Polyethylenimine (PEI) (of about <15KDa), Branched Low MW Polyethylenimine (PEI) (of about <10KDa), Branched High MW Polyethylenimine (of about >/=25 KDa), Poly-L-Arginine (average or nominal MW of about 70,000 Da), Poly-L-Arginine (average or nominal MW > about 50,000 Da), Poly-L-Arginine (average or nominal MW of about 5,000 to about 15,000 Da), Poly-L-Lysine (average or nominal MW of about 28,200 Da), Poly-L-Lysine (average or nominal MW of about 67,000 Da), Poly Histidine, Ethylhexadecyldimethylammonium Bromide, Dodecyltrimethyl Ammonium Bromide, Tetradodecylammonium bromide, Dimethylditetradecyl Ammonium bromide, Tetrabutylammonium iodide, DEAE-Dextran hydrochloride, and Hexadimethrine Bromide.

In some embodiments of the devices provided herein the surfactant and the nanoparticles are mixed, lyophilized, and deposited together on the device. In some embodiments of the devices provided herein the surfactant is deposited on the medical device after the nanoparticles are deposited thereon.

A method may comprise forming a coating on a medical device comprising mixing a surfactant and nanoparticles of an active agent to prepare a agent-surfactant mixture, lyophilizing the agent-surfactant mixture, and depositing the agent-surfactant mixture on the device using an eSTAT process.

The coating may release the nanoparticles into a treatment site over at least one of: about 1 day, about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 1 day, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days.

In some embodiments of the devices provided herein the coating on the medical device comprises a positive surface charge. In some embodiments, the surfactant of the agent-surfactant mixture is cationic. In some embodiments, the surfactant comprises a primary amines having pH < 10, and a secondary amines having pH < 4.

In some embodiments, the surfactant comprises octenidine dihydrochloride. In some embodiments, the surfactant comprises a permanently charged quaternary ammonium cation. In some embodiments, the permanently charged quaternary ammonium cation comprises at least one of: an Alkyltrimethylammonium salt such as cetyl trimethylammonium bromide (CTAB), hexadecyl trimethyl ammonium bromide, cetyl trimethylammonium chloride (CTAC); Cetylpyridinium chloride (CPC); Polyethoxylated tallow amine (POEA); Benzalkonium chloride (BAC); Benzethonium chloride (BZT); 5-Bromo-5-nitro-1,3-dioxane; Dimethyldioctadecylammonium chloride; and Dioctadecyldimethylammonium bromide (DODAB). In some embodiments, the surfactant comprises at least one of: didodecyldimethylammonium bromide (DMAB), linear isoform Polyethylenimine (linear PEI), Branched Low MW Polyethylenimine (PEI) (of about <25KDa), Branched Low MW Polyethylenimine (PEI) (of about <15KDa), Branched Low MW Polyethylenimine (PEI) (of about <10KDa), Branched High MW Polyethylenimine (of about >/=25 KDa), Poly-L-Arginine (average or nominal MW of about 70,000 Da), Poly-L-Arginine (average or nominal MW > about 50,000 Da), Poly-L-Arginine (average or nominal MW of about 5,000 to about 15,000 Da), Poly-L-Lysine (average or nominal MW of about 28,200 Da), Poly-L-Lysine (average or nominal MW of about 67,000 Da), Poly Histidine, Ethylhexadecyldimethylammonium Bromide, Dodecyltrimethyl Ammonium Bromide, Tetradodecylammonium bromide, Dimethylditetradecyl Ammonium bromide, Tetrabutylammonium iodide, DEAE-Dextran hydrochloride, and Hexadimethrine Bromide.

A method may comprise depositing a polymer on the medical device following depositing the agent-surfactant mixture on the device. The polymer may comprise PLGA. In some embodiments, depositing the polymer on the medical device uses at least one of a RESS coating process, an eSTAT coating process, a dip coating process, and a spray coating process.

In some embodiments, the medical device comprises a balloon.

A method may comprise coating at least a portion of a medical device thereby forming on the medical device a coating comprising an active agent and a binding agent, wherein the method comprises: dissolving the binding agent to form a binding agent solution, combining the binding agent solution and the active agent, mixing the combined binding agent and active agent using a high shear mixer, forming a suspension comprising the combined mixed active agent and binding agent, lyophilising the suspension to form a lyophilisate of the active agent and the binding agent, and coating the medical device with the lyophilisate in powder form using an eSTAT process, wherein the active agent coated on the medical device comprises active agent in crystalline form.

In some embodiments, the high shear mixer is a mechanical mixer. In some embodiments, the mechanical mixer comprises an impeller, propeller, and/or a high speed saw tooth disperser. In some embodiments, the mechanical mixer comprise a high pressure pump. In some embodiments, the high shear mixer comprises a sonic mixer. In some embodiments, the sonic mixer comprises a sonicator. In some embodiments, the sonic mixer comprises a benchtop bath based sonicator. In some embodiments, the sonic mixer comprises an ultrasonic mixer. In some embodiments, the sonic mixer comprises an megasonic mixer.

The ratio of the active agent to the binding agent is 1:1, 2:1, 3:1, 4:1, 5:1, 10:1, 3:2,52, or 5:3, as a target ratio. The actual ratio of the active agent to the binding agent is +/-30% of the target ratio. In some embodiments, the actual ratio is calculated based on UV-Vis testing of the medical device.

In some embodiments, when the device is delivered to a treatment site in vivo, at least 3%, at least 5%, or at least 10% of the active agent is transferred to tissue of the treatment site.

In some embodiments, the binding agents comprises at least one of: Polyarginine, Polyarginine 9-L-pArg, DEAE-Dextran (Diethylaminoethyl cellulose- Dextran), DMAB (Didodecyldimethylammonium bromide), PEI (Polyethyleneimine), TAB (Tetradodecylammonium bromide), and DMTAB (Dimethylditetradecylammonium bromide).

In some embodiments, an average molecular weight of the binding agent is controlled.

In some embodiments, a size of the active agent in the coating is controlled.

In some embodiments, the active agent is sirolimus and wherein the sirolimus has have an average size of at least one of: about 1.5 µm, about 2.5 µm, about 645nm, about 100-200 nm, another controlled size, or a combination thereof.

In some embodiments, the active agent is sirolimus and wherein sirolimus at least 50%, at least 75% and/or at least 90% of the sirolimus as is 1.5 µm, 2.5 µm, 645nm, 100-200 nm, or another controlled size.

In some embodiments, the coating may comprise nanoparticles, and the nanoparticles may comprise an active agent and a polymer.

In some embodiments, the coating comprises PLGA comprising about 50:50 Lactic acid: Glycolic acid.

In some embodiments, the coating comprised and about a 10:1 ratio of the active agent to the binding agent, wherein the active agent comprises sirolimus wherein the binding agent comprises Polyarginine.

In some embodiments, the sirolimus has an average size of 1.5 µm or 2.5 µm (not according to the invention)

In some embodiments, the Polyarginine average molecular weight is 70kDa. In some embodiments, the Polyarginine average molecular weight is 5-15kDa.

In some embodiments, the active agent and the binding agent are lyophilized prior to deposition on the medical device.

In some embodiments, at least about 2 ng/mg of active agent, at least about 3 ng/mg of active agent, at least about 5 ng/mg of active agent, at least about 10 ng/mg of active agent, at least about 20 ng/mg of active agent, at least about 30 ng/mg of active agent, and/or at least about 40 ng/mg of active agent are found in tissue 72 hours after delivery of the medical device to the treatment site.

In some embodiments, the device releases at least one of: at least 5% of the active agent to tissue upon delivery of the medical device to the treatment site, at least 7% of the active agent to tissue upon delivery of the medical device to the treatment site, at least 10 % of the active agent to tissue upon delivery of the medical device to the treatment site, at least 15 % of the active agent to tissue upon delivery of the medical device to the treatment site, at least 20 % of the active agent to tissue upon delivery of the medical device to the treatment site, at least 25 % of the active agent to tissue upon delivery of the medical device to the treatment site, at least 25 % of the active agent to tissue upon delivery of the medical device to the treatment site, at least 30 % of the active agent to tissue upon delivery of the medical device to the treatment site, at least 40 % of the active agent to tissue upon delivery of the medical device to the treatment site, at least 50 % of the active agent to tissue upon delivery of the medical device to the treatment site, between 2% and 50% of the active agent to tissue upon delivery of the medical device to the treatment site, between 3% and 50% of the active agent to tissue upon delivery of the medical device to the treatment site, between 5% and 50% of the active agent to tissue upon delivery of the medical device to the treatment site, between 3% and 30% of the active agent to tissue upon delivery of the medical device to the treatment site, between 3% and 25% of the active agent to tissue upon delivery of the medical device to the treatment site, between 3% and 20 of the active agent to tissue upon delivery of the medical device to the treatment site, between 3% and 15% of the active agent to tissue upon delivery of the medical device to the treatment site, between 1% and 15% of the active agent to tissue upon delivery of the medical device to the treatment site, between 1% and 10% of the active agent to tissue upon delivery of the medical device to the treatment site, between 3% and 10% of the active agent to tissue upon delivery of the medical device to the treatment site, and between 1% and 5% of the active agent to tissue upon delivery of the medical device to the treatment site.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1 depicts an example eSTAT process for coating 12 angioplasty balloons with sirolimus.
Figure 2 depicts coating balloons according to an RESS process.
Figure 3 shows the sirolimus concentrations in rabbit iliac blood (systemic) as tested from 0 to 24 hrs.
Figure 4 shows zeta potentials of DMAB in solution with rapamycin.
Figure 5 depicts a method of preparing a coating formulation according to an embodiment herein.

### DETAILED DESCRIPTION OF THE INVENTION

Provided herein is a device as defined in the claims.

Electrostatic Capture may be used for depositing a coating on a device (e.g. a balloon), and may be referred to as "eSTAT" herein. Coating is applied to the balloons via eSTAT attraction, where the positively charged coating coat a negatively charged device. For example, in some embodiments, sirolimus in crystalline form is applied to the balloons via eSTAT attraction where the positively charged drug particles coat the negatively charged balloons. The sirolimus coated on the balloon, in some embodiments, has an inherently positive charge.

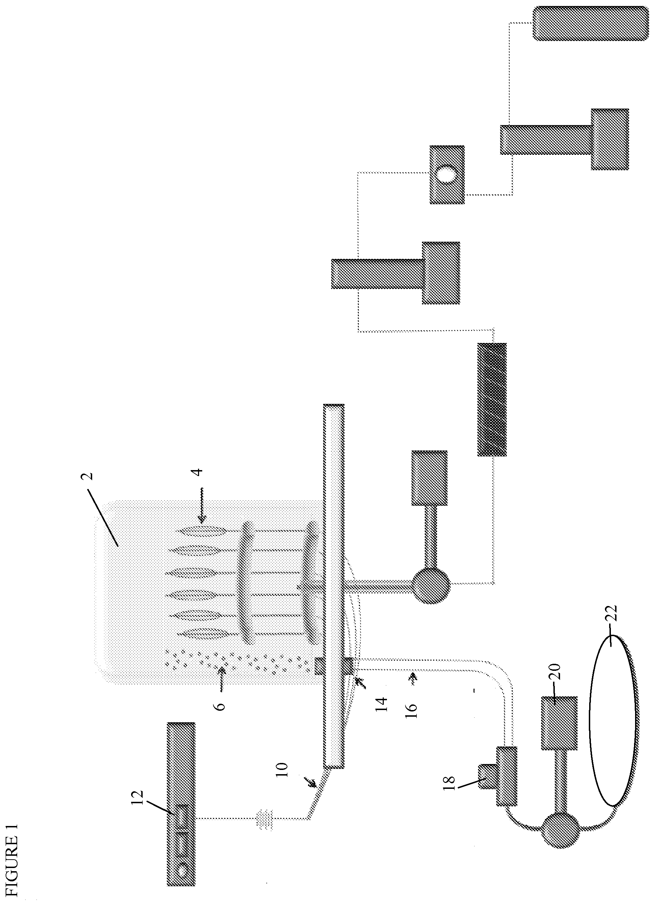

Figure 1 depicts an example eSTAT process for coating 12 angioplasty balloons with sirolimus. In this example process, an eight liter aluminum foil coated bell jar 2 is kept in place, but is not electrically grounded. Milled sirolimus (15.5 mg) is placed in a Swagelok 1/2" tee filter 18 (Swagelok, Inc., Supplemental Figure S15) connected to a pulsed pneumatic valve 20 (Swagelok, Inc., Supplemental Figure S16) attached to a cylinder of compressed nitrogen 22. The tee filter is connected on the other end to the eSTAT nozzle 14 , a 1/2" × 3/8" Swagelok reducing union fitted to a modified 3/8" Swagelok bulkhead union (Swagelok, Inc., Supplemental Figure S17) via 1/2" (outer diameter) polypropylene tubing 16. Balloon(s) 4 are mounted in place under the bell jar 2. In this example, twelve 3.0 mm width balloons at a time are coated with the positively charged milled sirolimus 6. The balloons 4 may be of various lengths, such as lengths ranging from 17 mm to 23 mm, however, in other embodiments, other sizes may be used. In some embodiments, fewer or more balloons may be coated at a time. The balloons 4 used during the coating process are typically mounted on catheters having wires 10 disposed therein 8 which are coupled to a high voltage power supply 12 (such as a Spellman SL30 high voltage power supply), which may be set at -15kV, for example.

In some embodiments, the lengths of the balloons may be any length from 5 mm to 35 mm, or any of the following lengths, for example: about 5 mm, about 7 mm, about 8 mm, about 10 mm, about 12 mm, about 13 mm, about 15 mm, about 18 mm, about 20 mm, about 21 mm, about 23 mm, about 25 mm, about 28 mm, about 30 mm, about 32 mm, about 33 mm, and about 35 mm. The term "about" when used in the context of balloon length, can mean variations of for example, 10%, 25%, 50%, 0.1 mm, 0.25 mm, 0.5 mm, 1 mm, 2 mm, and 5 mm, depending on the embodiment.

In some embodiments, the diameters (i e. widths) of the balloons may be any diameter from 1.5 mm to 6.0 mm, or any of the following diameters, for example: about 1.5 mm, about 1.8 mm, about 2.0 mm, about 2.25 mm, about 2.5 mm, about 2.75 mm, about 3.0 mm, about 3.25 mm, about 3.5 mm, about 3.75 mm, about 4.0 mm, about 4.25 mm, about 4.5 mm, about 4.75 mm, about 5.0 mm, about 5.25 mm, and about 5.5 mm. The term "about" when used in the context of balloon diameter (or width), can mean variations of for example, 10%, 25%, 50%, 0.1 mm, 0.25 mm, 0.3 mm, 0.4 mm, 0.5 mm, 0.75 mm, 1 mm, and 2 mm, depending on the embodiment.

In some embodiments a minimum of one balloon is coated at a time. In some embodiments, at least one of: at least 3 balloons, at least 5 balloons, at least 6 balloons, at least 8 balloons, at least 10 balloons, at least 12 balloons, at least 15 balloons, at least 16 balloons, at least 20 balloons, at least 24 balloons, and at least 30 balloons are coated at a time.

These balloons may or may not be pre-coated with a polymer, such as PLGA. Coating of the balloons may be achieved by various means, such as dip coating, spray coating, or coating using an RESS method. For example, a polymer (e.g. PLGA) is applied to the balloons via rapid expansion of supercritical solutions (RESS), where the solute (e.g. PLGA) is dissolved in a supercritical fluid then rapidly expanded with sudden decompression by passing through a short nozzle into an area of low temperature and pressure. These conditions cause the dissolved PLGA to rapidly precipitate as a fine powder with a narrow distribution of particle size resulting in a uniform coating on the angioplasty balloons.

"Rapid Expansion of Supercritical Solutions" or "RESS" as used herein involves the dissolution of a polymer into a compressed fluid, typically a supercritical fluid, followed by rapid expansion into a chamber at lower pressure, typically near atmospheric conditions. The rapid expansion of the supercritical fluid solution through a small opening, with its accompanying decrease in density, reduces the dissolution capacity of the fluid and results in the nucleation and growth of polymer particles. The atmosphere of the chamber is maintained in an electrically neutral state by maintaining an isolating "cloud" of gas in the chamber. Carbon dioxide, nitrogen, argon, helium, or other appropriate gas is employed to prevent electrical charge is transferred from the substrate to the surrounding environment.

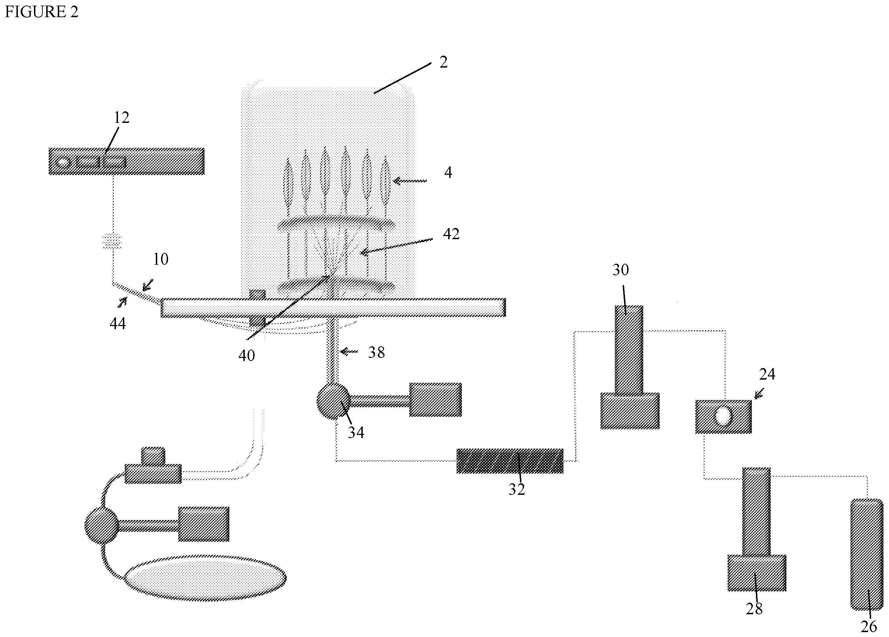

Figure 2 depicts an example of coating balloons according to an RESS (rapid expansion of supercritical solution) process. Figure 2 depicts an example RESS process for coating balloons 4 with PLGA. The PLGA is loaded into a vessel 24 in which it is dissolved in HFC236ea from a HFC236ea cylinder 26 which is sent to the vessel 24 through a syringe pump 28 (for example, an Isco 260D syringe pump). The PLGA thus forms a supercritical solution with the HFC236 ea, which is stirred at a high pressure (5500 psi) in mixing view cells (50cc). The PLGA solution is sent through a syringe pump 30 (for example, an Isco 260D syringe pump) which sends the solution through a heater block 32 (with temperature control feedback) and then through a timed pneumatic valve 34 which is heated at 137C. The PLGA solution is then sent through a capillary tube 36 (e.g. PEEKsil capillary tube 1/16" outer diameter by 100 micron inner diameter by 10 cm long) which is surrounded by a stainless steel sheath (e.g. ¼ inches thick stainless steel sheath). The PLGA is then ejected through a nozzle 40 which is electrically grounded (for example, via a stainless steel sheath). When the PLGA solution exits the nozzle 40, the PLGA is ejected as dry PLGA particles 42, as the solution comprising PLGA and HFC236ea rapidly expands. The balloons 4 used during the coating process are typically mounted on catheters having wires 10 disposed therein 8 which are electrically grounded 44. The wires 10 may be coupled to a high voltage power supply 12 (such as a Spellman SL30 high voltage power supply), in order to facilitate the eSTAT coating of the balloons with the active agent, however, during the RESS process described in this embodiment, the balloons are electrically grounded and no current flows from the power supply 12.

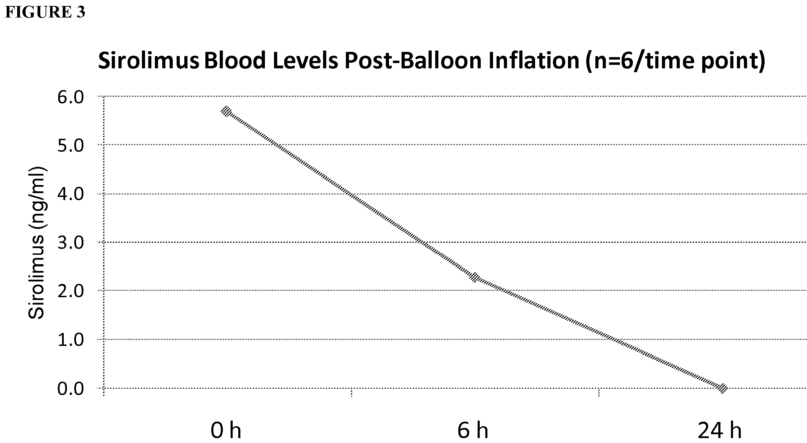

When viewed in combination, Figure 1 and 2 indicate a single apparatus that can both coat according to an RESS process and an eSTAT process. Elements called out and depicted in Figure 2 may similarly be called out in Figure 3, and vice versa. Alternatively, separate coating apparatuses may be used to separately coat according to an RESS process and an eSTAT process.

In one embodiment (not according to the invention), PLGA (30KDa MW, 15 KDa Mn) is coated on 12 GHOST rapid exchange nylon balloon catheters (in this example, 3.0 mm width × 18 mm length balloons were coated, however, other sizes may be similarly coated as noted herein). An 8 L bell jar lined on the outside with aluminum foil and electrically grounded to a stainless steel post of the coating platform is placed over the 12 balloons. The stylus wires inserted in the balloon catheters are combined in 2 bundles of 6 in plastic insulation tubing and electrically grounded to the lead from a Spellman SL30 high voltage power supply outside of the bell jar. No current is passed through the power supply during the process. The RESS restrictor nozzle composed of PEEKsil 1/16" tubing and surrounded by 1/4" stainless steel tubing is also electrically grounded via a plastic insulation tube between the inside diameter of the stainless steel sheath and the outside diameter of the PEEKsil tubing that is connected to a lead attached to a stainless steel post of the coating platform. PLGA is dissolved in HFC236ea at 150C and 5500 psi in a high pressure mixing view cell. Balloons are sprayed with 1 coating of ~2mg/ml PLGA in HFC236ea for 1 minute by a Teledyne 260D Isco pump through a temperature controlled heater block and a timed pneumatically operated high pressure purge valve for 1/16th tubing connected to the RESS restrictor nozzle.

A potential of -15 kV is applied to the bundled stylus wires of the balloon catheters connected to the lead from the Spellman SL30 high voltage power supply. The negative charge applied to the balloons is done to electrostatically attract the sirolimus particles, which have an inherent positive charge, in some embodiments. An eSTAT pulse of 500 pounds per square inch gauge (psig) from the compressed nitrogen is then initiated. Sirolimus is deposited onto the balloons over 90 seconds. The high voltage current during the run starts at 3 uA and ends at 2 uA.

In one test, Sirolimus particles of ~2.5 microns particle size, on average, were coated according to an eSTAT process onto 12 balloons (3.0 mm width × 18 mm length balloons). 15mg (15000 ug) of Sirolimus ejected into the eSTAT deposition chamber. The average amount of sirolimus coated per balloon was 75ug. The total amount of sirolimus that detected on all 12 balloons was 900ug (i.e. 12x75). Thus, the efficiency of such an eSTAT process to deposit sirolimus (of ~2.5 microns average particle size) onto a balloon is about 6% (900/15000).

In one test, (not according to the invention; referred to as Study 1, herein) a 1:1 ratio of PLGA to sirolimus ratio (w/w) was coated on Ghost 3.0x18 mm Rx catheter balloons. An RESS process as noted above was used to coat the balloons with PLGA, followed by an eSTAT (electrostatic) process as noted above to coat the nylon balloons with sirolimus. The coated balloons were then sintered. The amount of sirolimus coated on each balloon was targeted to be 50 micrograms or more, or 50-100 micrograms. Coating on the balloons averaged between ~50-80 µg of Sirolimus per balloon. The target mass for the PLGA was also 50 micrograms or more, or 50-100 micrograms (hence the 1:1 PLGA to sirolimus ratio (w/w) noted above). Following coating of the balloon with the PLGA and sirolimus, the coated balloons were sintered. The coated balloons were sterilized and then used in a rabbit animal study. The balloons were deployed at the target sites (iliac arteries) in the rabbits in the study, two balloons per animal. The animal study resulted in 197.2 ± 85.3 ng/mg of sirolimus embedded in artery walls at 0h. The amount of drug per tissue was determined using a LC-MS test method. Liquid chromatography-mass spectrometry (LC-MS) was used to determine the amount of drug per tissue. Prior to LC-MS, artery samples were homogenized using an Omni hand held probe (1:99 w:v in 80:20 MeOH:2mM Ammonium Acetate in water with 0.1% Formic Acid). The density of the artery was assumed to be 1g/mL

Efficiency of sirolimus transferred from balloons to artery walls was 7.8 ± 2.8%. 5.4 ± 2.3 µg of sirolimus washed away into circulation at 0h. 78.9 ± 6.8% of sirolimus removed from balloons after inflation in arteries. 65.6 ± 1.3% of sirolimus removed from balloons before inflation. 1-5% of the drug (sirolimus) transferred to artery; 1 ng of drug per mg of tissue was detected.

Variables which can affect the amount of drug that is retained on a drug coated balloon upon inflation include: balloon insertion to the target location (e.g. distance of travel, friction along path, relative diameters of path and balloon, etc.), blood flow, balloon processing (e.g. pleating, folding, sheathing, sterilization, handling generally etc.), shipping, balloon inflation and/or contact with artery wall (e.g. physical contact of coating to artery wall). If, for example, the balloon insertion to the target location goes along a long or tortuous path, this can result in coating lost in transit. The relative diameters of the path and the balloon can also result in more coating lost where the diameters are close such that there is friction in transit. On the other hand, blood flow can wash off the balloon coating, an effect that may increase where the ratio of diameter of vessel to diameter of balloon along the delivery path increases, or when the time that the balloon is in the vascular system increases during the procedure. Furthermore, balloon processing and handling can remove coating during these processes, as can friction and shipping. Nevertheless, balloon inflation and/or contact with artery wall (e.g. physical contact of coating to artery wall) is where the coating is designed to be removed from the balloon in large part, such that the drug is delivered to the target treatment site.

A test was performed to show how blood flow affected the coating on balloons from Study 1. From Study 1, four balloons were left uninflated for two minutes in an aorta of a rabbit, and exposed to blood flow therein. The percent drug (sirolimus) lost was based on balloon batch average from UV-Visometry testing. The Study 1 average percent lost was 65.6% (St. dev. 1.3 %) where the drug loaded was on average 21.3 ug per balloon (St.dev. 2.0ug).

A test was performed to show how folding affected the coating on balloons. Balloons were coated according to an RESS process for depositing a polymer, and an eSTAT process to deposit crystalline drug (sirolimus), in accord with Study 1 as noted above. The amount of drug on each balloon following the pleating (i.e. folding) process was compared to the batch average for each balloon size to determine a percent of drug lost in the pleating process. Table 1 shows the results, which shows that, as compared to the batch average amount of drug for the balloon size, that the amount of drug lost on average was 8% (St. dev. 16%), with a range of 0 to 25.8% lost in the process.

**TABLE 1**

| Balloon Size, Batch Average drug amount | Total ug drug on Balloon (Post Pleat/Fold) | % of Batch Average retained |
|---|---|---|
| 3x17 mm | 59.0 | 95.9% |
| 61.3 ug | 54.5 | 88.5% |
| | 45.9 | 75.5% |
| 3x18 mm | 95.4 | 123.4% |
| 76 ug | 84.6 | 109.4% |
| | 65.4 | 89.3% |
| | 37.9 | 78.3% |
| 3x23 mm | 68.8 | n/a |
| 62.8 ug | 58.0 | 93.7% |
| | 47.2 | 74.2% |
| Average percent coating retained | | 92.0% |
| Stdev | | 16.0% |

A test was performed to show how folding in combination with sterilization affected the coating on balloons. The balloons were coated according to an RESS process for depositing a polymer, and an eSTAT process to deposit crystalline drug (sirolimus) in accord with Study 1 as noted above. The amount of drug on each balloon following the pleating (i.e. folding) and sterilization processes were compared to the batch average for each balloon size to determine a percent of drug lost in the processes. Table 2 shows the results, which shows that, as compared to the batch average amount of drug for the balloon size, that the amount of drug lost on average was 16.2% (St. dev. 11.9%), with a range of 0 to 34.2% lost in the processes.

**TABLE 2**

| Balloon Size, Batch Average drug amount | Total ug drug on Balloon (Post Pleat/Fold) | % of Batch Average retained |
|---|---|---|
| 3x17 mm | 53.0 | 88.3% |
| 57.6 ug | 39.5 | 65.8% |
| | 42.1 | 79.9% |
| 3x18 mm | 59.0 | 80.6% |
| 73.9 ug | 70.2 | 95.9% |
| | 65.1 | 86.3% |
| | 61.6 | 81.5% |
| 3x23 mm | 60.2 | n/a |
| 62.5 ug | 64.9 | 104.8% |
| | 44.7 | 70.8% |
| Average percent coating retained | | 83.8% |
| Stdev | | 11.9% |

A test was performed to show transfer efficiency of drug (sirolimus) to rabbit iliac arteries using balloons coated in accord with Study 1 as noted above. Balloons were inflated for a minimum of one minute. The percent of drug (sirolimus) transferred to the artery based on either balloon batch average amount of drug expected to be on the balloon from UV-Visometry data, or based on the amount of drug released from the respective balloons. The estimated time the artery was exposed to blood flow was also tracked, and refers to the amount of time after the balloon was inflated. Table 3 shows the results of this study, which indicates that the Average percent of drug (sirolimus) transferred to the artery was 8.5% with a standard deviation of 2.5% (based on balloon batch average). Based on the amount of drug released from the respective balloons, the average amount of drug transferred to the artery was 23.0 % with a standard deviation of 8.5%.

**TABLE 3**

| Rabbit # (0 h) | Total Sirolimus per Artery (µg) | % Sirolimus Transferred to Artery (based on Batch Average UV-Vis) | % Sirolimus Transferred to Artery Relative to Sirolimus Released from Respective Balloon | Estimated Time Artery Exposed to Blood Flow (min) |
|---|---|---|---|---|
| Denuded Arteries | 7.36 | 10.06% | 23.30% | 10 |
| | 4.62 | 6.32% | 22.22% | 5 |
| | 5.75 | 8.50% | 27.89% | 10 |
| | 4.46 | 6.60% | 16.78% | 5 |
| | 3.92 | 5.18% | 12.25% | 10 |
| | 4.27 | 6.12% | 13.54% | 5 |
| Average SD | 5.06 | 7.13% | 19.33% | - |
| | 1.29 | 1.80% | 6.13% | - |
| Uninjured Arteries | 6.87 | 10.45% | 25.65% | 10 |
| | 6.47 | 9.83% | 20.35% | 5 |
| | 6.26 | 9.51% | 34.00% | 10 |
| | 3.83 | 5.21% | 11.71% | 5 |
| | 8.20 | 11.20% | 31.52% | 10 |
| | 8.45 | 12.50% | 36.73% | 5 |
| Average SD | 6.68 | 9.78% | 26.66% | - |
| | 1.66 | 2.48% | 9.42% | - |

A test was performed to show transfer efficiency of drug (sirolimus) to rabbit iliac arteries using balloons coated according to Study 1 noted above. After a 2d balloon inflation, blood samples were taken at the given time points (0h, 6h, 24h). The results show the cumulative drug concentration from the inflation of two coated balloons per animal. The total drug (sirolimus) in the blood was based on 56 mL per kg in rabbits. This was normalized to humans, based on 5 L of blood in the average human. Figure 3 shows the sirolimus concentrations in rabbit iliac blood (systemic) as tested from 0 to 24 hrs. These results show that the drug (sirolimus) released from the balloon and/or the artery following inflation of the balloon was cleared from the blood within 24hrs, see Table 4.

**TABLE 4**

| Time | Post-Inflation Conc. (ng/mL) | Est. Total Sirolimus in Blood (µg) | Est. Total Sirolimus in Blood Normalized to Human (ng/mL) |
|---|---|---|---|
| 0 Hr | 4.63 | 0.7 | 0.14 |
| | 6.90 | 1.2 | 0.23 |
| | 4.77 | 0.8 | 0.16 |
| | 8.35 | 1.5 | 0.30 |
| | 5.01 | 0.9 | 0.18 |
| | 4.49 | 0.8 | 0.16 |
| Average 0hr | 5.7 | 1.0 | 0.20 |
| SD | 1.6 | 0.3 | 0.06 |
| 6hr | 2.82 | 0.5 | 0.10 |
| | 2.34 | 0.4 | 0.08 |
| | 2.07 | 0.4 | 0.07 |
| | 1.82 | 0.3 | 0.06 |
| | 2.25 | 0.4 | 0.08 |
| | 2.36 | 0.4 | 0.08 |
| Average 6hr | 2.3 | 0.4 | 0.08 |
| SD | 0.3 | 0.1 | 0.01 |
| 24hr | BQL* | - | - |
| | BQL | - | - |
| | BQL | - | - |
| | BQL | - | - |
| | BQL | - | - |
| | BQL | - | - |

| | | | |
|---|---|---|---|
| ^{∗}BQL stands for Below Quantifiable Limit | | | |

### Nanoparticles of crystalline sirolimus

In some embodiments, a coating formulation uses supercritical fluids and electrostatics to coat a balloon with crystalline sirolimus and the polymer PLGA. Crystalline sirolimus particle generally speaking is ~2.5 microns, however, to increase arterial uptake, Sirolimus having an average particle size in the nanometer range (particle sizes below 300 nm, or below 100 nm, for non-limiting example) are prepared and coated on the balloon. Nanoparticles of drug are useful since where the size of the drug particle is reduced (to nanoparticle size, e.g.), the tissue retention of the drug increases.

Particle size and shape affects efficiency of drug localization. Spherical nanoparticles generally speaking, are taken up more efficiently vs. rod shaped nanoparticles. Cells incorporate nanoparticles up to 300 nm via internalization pathways: nonspecific or receptor-mediated endocytosis: nanoparticles that are <100 nm deposit in the vessel wall, whereas nanoparticles that are >100 nm tend to deposit at luminal surface. See, e.g. Small 2010, 6, No. 1, 12-21*.*

Nanoparticles come in various sizes: nanometers (e.g. dendrimers) to hundreds of nanometers (e.g. polymeric, lipid-based particles) to micron-sized particles. Nanoparticles come in various shapes, from the classical spherical particles to discoidal, hemispherical, cylindrical, conical, nanoreefs, nanoboxes, clusters, nanotubes, whiskers, rods, fibers, cups, ellipsoids, oblate ellipsoids, prolate ellipsoids, torus shaped, spheroids, taco-like, bullets, barrels, lenses, capsules, pulley wheels, circular discs, rectangular discs, hexagonal discs, flying saucer-like, worms, ribbon-like particles, and ravioli-like, for non-limiting example. Nanoparticles can have various surface functionalizations, with a broad range of electrostatic charges and bio-molecule conjugations. Investigation of nanoparticle surface modifications for arterial uptake may be done using an ex-vivo dog femoral artery model. Selected formulations may be tested in vivo in acute dog femoral artery and pig coronary artery models. See, e.g. Journal of Pharmaceutical Sciences, Vol. 87, No. 10, October 1998; 1229-1234*.* Nanoparticles surface modified with cationic DMAB, demonstrated 7 to 10-fold greater arterial uptake compared to the unmodified nanoparticles in different ex-vivo and in-vivo studies. Increasing nanoparticle concentration in the infusion solutions increased uptake. *Id.*

Didodecyldimethylammonium bromide (DMAB)-modified paclitaxel-loaded PCL/Pluronic F68 nanoparticles (250-300 nm), positively charged (25 mV), 3.5% drug loading (w/w) were prepared, and these nanoparticles were locally infused into injury vessel. See, e.g. JOURNAL OF PHARMACEUTICAL SCIENCES, VOL. 98, NO. 6, JUNE 2009. These nanoparticles inhibited neointimal hyperplasia in rabbit model.

Water-dispersible gel-like nanoparticles (54 nm), negatively charged (-8.45 mV), 4.2% drug loading (w/w) were synthesized via random free-radical polymerization of N-isopropylacrylamide, N-vinyl pyrrolidone, pegylated maleic. The nanoparticles were lyophilized then dispersed in distilled water by vortexing, to which 250 L of methanolic solution of sirolimus added with constant stirring allowing drug entrapment into nanoparticles. 60 ug of these sirolimus nanoparticles were infused locally in arterial wall immediately after balloon angioplasty via infusion catheter, which showed 9% (5 ug) arterial uptake. See, e.g. Circ Cardiovasc Interv 2008;1;209-26.

Intramural proteins, such as αvβ3-integrins expressed by stretch activated smooth muscle cells exposed along the sheared tissue planes of the arterial wall, provide ample targets for the drug-laden Nanoparticles. Paramagnetic perfluorocarbon NP emulsions: Nanoparticles comprised of 20% perfluorooctylbromide, 1.5% surfactant comixture, 1.7% glycerin, and water for the balance. Sirolimus incorporated into the surfactant layer at a concentration of 0.4 mol%. Infused locally in the arterial wall immediately after balloon stretch injury. See, e.g. Arterioscler Thromb Vasc Biol 2008;28820-826.

Chondroitin sulfate proteoglycans (CSPGs) expressed within the subendothelial matrix but not vascular endothelial cells. Prednisolone phosphate encapsulated in pegylated 3,5-dipentadecyloxybenzamidine hydrochloride (TRX-20) liposomes. Cationic lipid component of TRX-20 is subsequently endocytosed by cellular mechanisms resulting in directed drug release inside the cell. Lipid mixture of HSPC, cholesterol, and TRX-20 at a molar ratio of 50:42:8 and is hydrated with prednisolone disodium phosphate (PSLP) to obtain an expected diameter of around 100 nm. See, e.g. Arterioscler Thromb Vasc Biol. 2008;28:1960·1966.

Local infusion or systemic infusion is that nanoparticles that do not have targeting molecules attached can selectively enter certain organs solely on the basis of their charge and size. See, e.g. Nature Nanotechnology | VOL 3 | JANUARY 2008, 12-13.

A balloon having a coating comprising nanoparticles of sirolimus has been developed as described herein for localized delivery of the sirolimus to a vessel for arterial uptake that occurs over a period of time.

Sirolimus nanoparticle production is done according to a high shear fluid process that aids in particle size reduction (e.g. Microfluidics process and equipment for preparing nanoparticles). In such a process, a high pressure pump (up to 40,000 psi (2578 bar) forces particles into engineered microchannels within an interaction chamber. Inside the chamber, the product is exposed to consistent impact and shear forces and then immediately cooled, results in particles that are 50% smaller than homogenizers, uniform product output due to consistent shear. In some embodiments, the sirolimus nanoparticles are, at least in part, in crystalline form.

The balloon coating procedure follows, and in some embodiments, involves Supercritical fluid (SCF) polymer coating (an RESS process) onto a balloon followed by nanoparticle Sirolimus coating of the balloon, which may be achieved by various processes, noted herein.

For example, a polymer (e.g. PLGA) is applied to the balloons via rapid expansion of supercritical solutions (RESS), where the solute (e.g. PLGA) is dissolved in a supercritical fluid then rapidly expanded with sudden decompression by passing through a short nozzle into an area of low temperature and pressure. These conditions cause the dissolved PLGA to rapidly precipitate as a fine powder with a narrow distribution of particle size resulting in a uniform coating on the angioplasty balloons.

"Rapid Expansion of Supercritical Solutions" or "RESS" as used herein involves the dissolution of a polymer into a compressed fluid, typically a supercritical fluid, followed by rapid expansion into a chamber at lower pressure, typically near atmospheric conditions. The rapid expansion of the supercritical fluid solution through a small opening, with its accompanying decrease in density, reduces the dissolution capacity of the fluid and results in the nucleation and growth of polymer particles. The atmosphere of the chamber is maintained in an electrically neutral state by maintaining an isolating "cloud" of gas in the chamber. Carbon dioxide, nitrogen, argon, helium, or other appropriate gas is employed to prevent electrical charge is transferred from the substrate to the surrounding environment.

Figure 2 depicts an example of coating balloons according to an RESS (rapid expansion of supercritical solution) process. In this example, which is not according to the invention, PLGA (30KDa MW, 15 KDa Mn) is coated on 12 GHOST rapid exchange nylon balloon catheters (in this example, 3.0 mm width × 18 mm length balloons were coated, however, other sizes may be similarly coated as noted herein). An 8 L bell jar lined on the outside with aluminum foil and electrically grounded to a stainless steel post of the coating platform is placed over the 12 balloons. The stylus wires inserted in the balloon catheters are combined in 2 bundles of 6 in plastic insulation tubing and electrically grounded to the lead from a Spellman SL30 high voltage power supply outside of the bell jar. No current is passed through the power supply during the process. The RESS restrictor nozzle composed of PEEKsil 1/16" tubing and surrounded by 1/4" stainless steel tubing is also electrically grounded via a plastic insulation tube between the inside diameter of the stainless steel sheath and the outside diameter of the PEEKsil tubing that is connected to a lead attached to a stainless steel post of the coating platform. PLGA is dissolved in HFC236ea at 150°C and 5500 psi in a high pressure mixing view cell. Balloons are sprayed with 1 coating of ~2mg/ml PLGA in HFC236ea for 1 minute by a Teledyne 260D Isco pump through a temperature controlled heater block and a timed pneumatically operated high pressure purge valve for 1/16th tubing connected to the RESS restrictor nozzle.

In this example, nanoparticle Sirolimus coating of the balloon by an eSTAT process follows PLGA coating of the balloon by RESS process. Figure 1 depicts an example of coating balloons according to an eSTAT process. The sirolimus in this example remains (at least in part) in its crystalline form, however the particle size is in the nanoparticle range-i.e. at or below 1000 nm, at or below 300 nm, at or below 100 nm, for non-limiting example. eSTAT stands for electrostatic (eSTAT) attraction, and the process is described elsewhere herein. Sirolimus in crystalline form is applied to the balloons via eSTAT attraction where the positively charged drug particles coat the negatively charged balloons.

Provided herein is a coated medical device comprising a medical device for delivering nanoparticles of an active agent to a treatment site; and a coating on the medical device comprising a polymer, and the active agent nanoparticles, wherein the device delivers at least a portion of the coating to the treatment site which portion releases active agent nanoparticles into the treatment site over at least about 1 day. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, and/or 1-12 hrs, depending on the embodiment.

Provided herein is a coating for a medical device comprising a polymer and nanoparticles of an active agent, wherein the coating delivers the nanoparticles into a treatment site over at least about 1 day. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, and/or 1-12 hrs, depending on the embodiment.

A method may comprise forming coating on a medical device with nanoparticles of an active agent comprising depositing a polymer on the medical device using an RESS process, and depositing the nanoparticles on the medical device wherein depositing the nanoparticles comprises using an eSTAT process.

When referring to a device that delivers at least a portion of the coating to the treatment site, and the portion (or the device) delivers (or releases) the active agent nanoparticles into (or to) the treatment site over a certain period of time, the following is meant: First, the device deposits some amount of coating at the treatment site. The device itself (minus the coating that was delivered) may or may not be removed from the treatment site thereafter; however, some amount of coating is left behind at the site. This first process may take a minute, less than a minute, five minutes, a half hour, or another amount of time depending on the embodiment. For non-limiting example delivery of the coating to the treatment site may last as long as the time it takes to inflate a balloon, hold inflation for a short period, and then deflate and withdraw the balloon.

The portion of the coating that is left behind at the treatment site has an amount of active agent in it, for example, nanoparticles of active agent, and the tissue of the treatment site uptakes the active agent nanoparticles over a period of time-i.e. a second process and second timing. This may be done in various ways, as noted herein and/or known to one of skill in the art, according to various cellular uptake processes and/or according to release of the active agent nanoparticles from a carrier such as a polymer by various degradation processes. The time of this uptake may be 1 day or longer, depending on the embodiment. This second process may be described as the device delivering the active agent (or nanoparticles thereof) to the treatment site over a period of time, or the coating delivering the active agent (or nanoparticles thereof) to the treatment site over a period of time, or the device releasing the active agent (or nanoparticles thereof) to the treatment site over a period of time, or the coating releasing the active agent (or nanoparticles thereof) to the treatment site over a period of time, depending on the embodiment. The second timing, may occur, over at least one of: about 1 day, about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

In some embodiments of the devices provided herein the coating portion delivered to the treatment site releases nanoparticles to the treatment site over at least one of: about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

The active agent in the embodiments of the devices provided herein comprises a macrolide immunosuppressive drug. The active agent may be selected from sirolimus, a prodrug, a derivative, an analog, a hydrate, an ester, and a salt thereof. A portion of the nanoparticles may be in crystalline form. The nanoparticles may be, on average, at least one of: at most 1 micrometer, about 1 micrometer, below about 1 micrometer, below about 750 nanometers (nm), below about 500 nanometers, about 100 nm to about 1 micrometer, about 300 nm to about 1 micrometer, about 100 nm to about 300 nm, about 300 nm to about 500 nm, below about 300nm, below about 100nm, and between about 50nm and about 300 nm. The term "about" when used in the context of nanoparticle size can mean variations of, for example, 5%, 10%, 25%, 50%, 75%, 0 % to 25%, 0% to 50%, 10%-50%, 50nm, 100nm, 10 nm to 100nm, 0 nm to 100 nm, 250nm, 300nm, and/or 0nm to 250nm, depending on the embodiment.

In the devices provided herein the polymer comprises PLGA. The PLGA may have at least one of: a MW of about 30KDa and a Mn of about 15KDa, a Mn of about 10KDa to about 25 KDa, and a MW of about 15 KDa to about 40KDa. The term "about" when used in the context of polymer MW or Mn can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment.

In some embodiments of the devices provided herein the treatment site is a vessel wall. The treatment site may be in or on the body of a subject. The treatment site may be a vascular wall. The treatment site may be a non-vascular lumen wall. The treatment site may be a vascular cavity wall. The treatment site may be a wall of a body cavity. In some embodiments, the body cavity is the result of a lumpectomy. In some embodiments, the treatment site is a cannulized site within a subject. In some embodiments, the treatment site is a sinus wall. In some embodiments, the treatmetn site is a sinus cavity wall. In some embodiments, the active agent comprises a corticosteroid. "Treatment site" or "intervention site" as used herein refers to the location in the body where the coating is intended to be delivered. The treatment site can be any substance in the medium surrounding the device, e.g., tissue, cartilage, a body fluid, etc. The treatment site can be tissue that requires treatment.

In some embodiments of the devices provided herein the medical device comprises a balloon. In some embodiments the medical device comprises a balloon of a balloon catheter. In some embodiments the medical device comprises at least one of a catheter, a balloon, a cutting balloon, a wire guide, a cannula, tooling, an orthopedic device, a structural implant, stent, stent-graft, graft, vena cava filter, a heart valve, cerebrospinal fluid shunts, pacemaker electrodes, axius coronary shunts, endocardial leads, an artificial heart, any implant for insertion into the body of a human or animal subject, including but not limited to stents (e.g., coronary stents, vascular stents including peripheral stents and graft stents, urinary tract stents, urethral/prostatic stents, rectal stent, oesophageal stent, biliary stent, pancreatic stent), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, vascular supports, and a medical device that is not permanantly implanted.

A method may comprise depositing a second polymer on the medical device following depositing the nanoparticles. In some embodiments, a second polymer is deposited on the medical device following the deposition of the nanoparticles. In some embodiments, the coating comprises a second polymer. The second polymer may comprise PLGA. The PLGA has at least one of: a MW of about 30KDa and a Mn of about 15KDa, a Mn of about 10KDa to about 25 KDa, and a MW of about 15 KDa to about 40KDa. The term "about" when used in the context of polymer MW or Mn can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment. In some embodiments depositing the second polymer on the device uses at least one of a RESS coating process, an eSTAT coating process, a dip coating process, and a spray coating process.

### Positive surface charging of coating

Positive surface charging of a coating is done to improve arterial uptake of nanoparticles. Such a charging process is incorporated as part of a coating process noted herein.

One method of applying a positive surface charge to the coating is to add DMAB to the coating such that it is on its surface, at least. In one example, the method comprising using RESS to coat a polymer to the balloon, followed by coating by eSTAT, or by RESS sirolimus nanoparticles to the balloon, followed by RESS coating a mixture of PLGA and DMAB to the balloon according to an RESS process. The coating is then sintered according to the RESS process. This method coats about 60-70 ug drug on twelve 3.0 mm width × 18 mm length sized balloons, for example. Care should be taken in certain embodiments to optimize the DMAB's application to the balloon and to handle the balloon thereafter to keep the DMAB from washing off.

In a first set of studies, rapamycin (sirolimus) coating affinity testing was performed, wherein rapamycin was dispersed with a charge carrier in water. Table 5 shows the distribution of components between solid and liquid phases, wherein the surfactant to rapamycin ratio is 1:1 (w/w) in a 10x dilution of water. Table 5 shows the distribution of sirolimus (rapamycin) between the liquid and solid phases in a 10x dilution and the percent recovery of the initial rapamycin loading (shown in the column "total"). The initial mass of rapamycin is shown in the column labeled "Original rapa." The supernatant (liquid phase) contains the material of interest as it is drug particles coated with the surfactant modifier. The values presented in the columns labeled "Cal. Rapa" are calculated from the UV-Vis absorbance spectrum of the sediment or supernatant respectively. The ratio of these two values determined the partition coefficient between the sediment and supernatant (reported in the column labeled "ratio"). For the trial noted in Table 5, the top performers as surfactants include High MW PEI, DMAB, 70K MW polyarginine.

**TABLE 5: Distribution of components between solid and liquid phases-- 1:1 w/w surfactant:drug matrix**

| 1:1 Surfactant to Rapa 10× dilution | | | Cal. Rapa | Cal. Rapa | ratio | total |
|---|---|---|---|---|---|---|
| | Orginal rapa (mg) | Sediment wt (mg) | (Sediment) | (Supernate) | super/sed | (% original) |
| DMAB | 1.9 | 2.2 | 1.23 | 0.64 | 0.52 | 98.4% |
| PEI Linear | 2.13 | 6.62 | 1.73 | 0.51 | 0.29 | 105.2% |
| PEI Low MW | 1.97 | 1.33 | 1.2 | 0.54 | 0.45 | 88.3% |
| PEI High MW | 1.97 | 2.48 | 0.88 | 1.09 | 1.24 | 100.0% |
| Poly Arginine 70,000 | 2.2 | 11.7 | 1.49 | 0.73 | 0.49 | 100.9% |
| Poly Arginine 5,000-15,000 | 2.12 | 3.72 | 1.24 | 0.3 | 0.24 | 72.6% |
| Poly Lysin 28,200 | 1.99 | 4.89 | 1.36 | 0.35 | 0.26 | 85.9% |
| Poly Histidine | 2.08 | 7.21 | 1.61 | 0.32 | 0.20 | 92.8% |
| Ethylhexadecyldimeth ylammonium Bromide | 1.9 | 3.85 | 1.37 | 0.33 | 0.24 | 89.5% |
| Dodecyltrimethylam monium Bromide | 1.98 | 1.99 | 1.65 | 0.5 | 0.30 | 108.6% |
| Poly Lisine 66,700 | 1.88 | 1.85 | 1.43 | 0.33 | 0.23 | 93.6% |
| Tetradodecylammoni umbromid | 2 | 6.31 | 1.51 | 0.25 | 0.17 | 88.0% |
| Dimethylditetradecyla mmonium bromide | 2.2 | 2.28 | 1.9 | 0.59 | 0.31 | 113.2% |
| Tetrabutylammonium iodide | 2.25 | 2.1 | 1.97 | 0.6 | 0.30 | 114.2% |
| Dextran | 2.1 | 1.49 | 1.6 | 0.42 | 0.26 | 96.2% |
| Hexadimethrine Bromide | 2.4 | 4.3 | 2.02 | 0.61 | 0.30 | 109.6% |

Table 6 shows the distribution of components between solid and liquid phases, wherein the surfactant to rapamycin ratio is 1:10 (w/w) in a 100x dilution of water. Table 6 shows the distribution of sirolimus (rapamycin) between the liquid and solid phases in a 100× dilution and the percent recovery of the initial rapamycin loading (shown in the column "total"). The initial mass of rapamycin is shown in the column labeled "Original rapa." The supernatant (liquid phase) contains the material of interest as it is drug particles coated with the surfactant modifier. The values presented in the columns labeled "Cal. Rapa" are calculated from the UV-Vis absorbance spectrum of the sediment or supernatant respectively. The ratio of these two values determined the partition coefficient between the sediment and supernatant (reported in the column labeled "ratio"). For the trial noted in Table 6, no surfactants necessarily stand out as promising candidates to be put in a 1:10 surfactant to rapamycin at 100x dilution with water to be used as a coating.

**TABLE 6: Distribution of components between solid and liquid phases-- 1:10 w/w surfactant:drug matrix**

| 1:10 Surfactant to Rapa 100x dilution | | | Cal. Rapa | Cal. Rapa | ratio | total |
|---|---|---|---|---|---|---|
| | Orginal rapa (mg) | Sediment wt (mg) | (Sediment) | (Supernate) | super/sed | (% of original) |
| DMAB | 9.6 | 8.52 | 7.23 | 0.4 | 0.06 | 79.5% |
| PEI Linear | 9.93 | 7.53 | 6.26 | 1.58 | 0.25 | 79.0% |
| PEI Low MW | 9.6 | 7.69 | 6.09 | 0.91 | 0.15 | 72.9% |
| PEI High MW | 9.7 | 8.2 | 6.32 | 1.11 | 0.18 | 76.6% |
| Poly Arginine 70,000 | 10.22 | 7.9 | 6.95 | 1.01 | 0.15 | 77.9% |
| Poly Arginine 5,000-15,000 | 9.84 | 6.5 | 5.36 | 1.15 | 0.21 | 66.2% |
| Poly Lysin 28,200 | 9.65 | 7.4 | 7 | 0.74 | 0.11 | 80.2% |
| Poly Histidine | 10.59 | 7.9 | 7.06 | 1.29 | 0.18 | 78.8% |
| Ethylhexadecyldimeth ylammonium Bromide | 9.86 | 8.6 | 6.86 | 1.05 | 0.15 | 80.2% |
| Dodecyltrimethylamm onium Bromide | 9.68 | 7.7 | 6.6 | 1.33 | 0.20 | 81.9% |

Table 7 shows the distribution of components between solid and liquid phases, wherein the surfactant to rapamycin ratio is 10:1 (w/w) in a 6x dilution of water. Table 7 shows the distribution of sirolimus (rapamycin) between the liquid and solid phases in a 6x dilution and the percent recovery of the initial rapamycin loading (shown in the column "total"). The initial mass of rapamycin is shown in the column labeled "Original rapa." The supernatant (liquid phase) contains the material of interest as it is drug particles coated with the surfactant modifier. The values presented in the columns labeled "Cal. Rapa" are calculated from the UV-Vis absorbance spectrum of the sediment or supernatant respectively. The ratio of these two values determined the partition coefficient between the sediment and supernatant (reported in the column labeled "ratio"). For the trial noted in Table 7, promising candidates to be put in a 10:1 surfactant to rapamycin mixture at 6x dilution with water to be used as a coating include Ethylhexadecyldimethylammonium Bromide, DMAB, Dimethylditetradecylammonium bromide, Dodecyltrimethylammonium Bromide.

**TABLE 7: Distribution of components between solid and liquid phases-- 10:1 w/w surfactant/drug matrix**

| 10:1 Surfactant to Rapa 6x dilution | | | Cal. Rapa | Cal. Rapa | ratio | total |
|---|---|---|---|---|---|---|
| | Orginal rapa (mg) | Sediment wt (mg) | (Sediment) | (Supernate) | super/sed | (% of original) |
| DMAB | 2.1 | 3.73 | 1.23 | 1.02 | 0.83 | 107.1% |
| PEI Linear | 1.98 | 2.53 | 1.06 | 0.32 | 0.30 | 69.7% |
| PEI Low MW | 1.49 | 2.56 | 0.97 | 0.32 | 0.33 | 86.6% |
| PEI High MW | 1.71 | 2.95 | 1.04 | 0.38 | 0.37 | 83.0% |
| Poly Arginine 70,000 | 1.93 | 2.81 | 1.18 | 0.21 | 0.18 | 72.0% |
| Poly Arginine 5,000-15,000 | 1.91 | 5.11 | 1.1 | 0.51 | 0.46 | 84.3% |
| Poly Lysin 28,200 | 2.11 | 4.3 | 1.2 | 0.13 | 0.11 | 63.0% |
| Poly Histidine | 1.86 | 2.85 | 0.94 | 0.28 | 0.30 | 65.6% |
| Ethylhexadecyldimeth ylammonium Bromide | 2.18 | 3.21 | 0.04^{∗} | 1.26 | 31.50 | 59.6% |
| Dodecyltrimethylam monium Bromide | 2.26 | 2.56 | 1.2 | 0.78 | 0.65 | 87.6% |
| Poly Lisine 66,700 | 2.39 | 2.9 | 1.2 | 0.12 | 0.10 | 55.2% |
| Tetradodecylammoni umbromid | 2.23 | 3.6 | 1.24 | 0.39 | 0.31 | 73.1% |
| Dimethylditetradecyla | 1.87 | 1.9 | 0.85 | 1.21 | 1.42 | 110.2% |
| mmonium bromide | | | | | | |
| Tetrabutylammonium iodide | 1.86 | 2.05 | 1.07 | 0.42 | 0.39 | 80.1% |
| Dextran | 2.17 | 2.86 | 1.21 | 0.32 | 0.26 | 70.5% |
| Hexadimethrine Bromide | 1.93 | 2.54 | 1.08 | 0.28 | 0.26 | 70.5% |
| ^{∗}No sediment, very clear solution after sonication | | | | | | |

Preliminary zeta potential measurements were taken on series of DMAB/Rapa suspensions where the surfactant/drug is ≤ 1 (w/w). Table 8 shows these zeta potential measurements for various DMAB: Rapamycin solutions. In general, an increase in the concentration of cationic DMAB to the DMAB/Rapa suspension results in an increase in zeta potential. In Table 8, the w/w ratio of DMAB to Rapamycin is presented as is the mole ratio. In general, an increase in the concentration of cationic DMAB to the DMAB/Rapa suspension results in an increase in zeta potential. This suggests that more rapamycin is being coated with the DMAB thereby increasing its surface charge as manifested in the measurement of the zeta potential.

**TABLE 8**

| Mass ratio | DMAB (mg) | DMAB (mmol) | Rapa (mg) | Rapa (mmol) | DMAB/Rapa (mol/mol) | Zeta Potential (mV) | Std Error |
|---|---|---|---|---|---|---|---|
| 1:10 | 0.4 | 0.0009 | 4.0 | 0.0044 | 0.1974 | +55.38 | 0.57 |
| 1:8 | 0.5 | 0.0011 | 4.0 | 0.0044 | 0.2468 | +61.57 | 1.5 |
| 1:8 | 0.5 | 0.0011 | 4.0 | 0.0044 | 0.2468 | +72.36 | 1.22 |
| 1:6 | 0.7 | 0.0014 | 4.0 | 0.0044 | 0.3307 | +58.82 | 2.66 |
| 1:4 | 1.0 | 0.0022 | 4.0 | 0.0044 | 0.4935 | +71.02 | 2.21 |
| 1:2 | 2.0 | 0.0043 | 4.0 | 0.0044 | 0.9870 | +71.81 | 3.16 |
| 1:1 | 4.0 | 0.0086 | 4.0 | 0.0044 | 1.9741 | +82.99 | 1.02 |

Preliminary zeta potential measurements were taken on a Sodiumdodecylsulfate: Rapa suspension. In Table 9, the w/w ratio of SDS to Rapamycin is presented as is the mole ratio. This table shows that addition of anionic surfactant, SDS (Sodiumdodecylsulfate), induces negatively charged particles in contrast to the effect of cationic surfactant (e.g. DMAB and/or others noted in previous tables and/or referenced herein) Thus, SDS (Sodiumdodecylsulfate) in a solution with rapamycin was prepared as a negative control to validate results achieved with the DMAB, for example, which prepared positively charged particles as shown in Table 8. Table 9 shows the zeta potential measurement results of this negative control test.

**TABLE 9: Zeta Potential Measurement for Sodiumdodecylsulfate:Rapa Solution**

| Mass ratio | SDS (mg) | SDS (mmol) | Rapa (mg) | Rapa (mmol) | SDS/Rapa (mol/mol) | Zeta Potential (mV) | Std Error |
|---|---|---|---|---|---|---|---|
| 1:1 | 2.1 | 0.0073 | 2.1 | 0.0023 | 3.1736 | -83.57 | 4.5 |

Thus, generally increasing trend in zeta potential with increasing DMAB content suggests that increasing DMAB content below 1:1 by wt increases surface charge on Rapa particles.

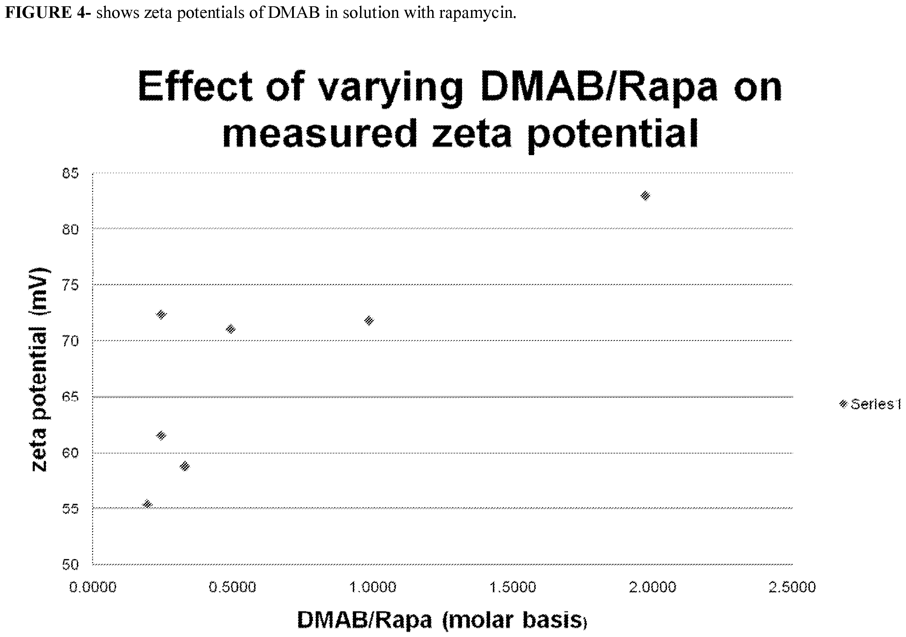

Figure 4 shows zeta potentials of DMAB in solution with rapamycin. The figure shows that high concentrations of DMAB in solution likely lead to high mV values. Measured potential is likely an average of particles and DMAB in solution. Common approach to this phenomenon is to centrifuge out the suspended particles and re-suspend in fresh water to minimize effect of dissolved DMAB not related to the particles.

As an alternative, in some embodiments, other molecules (surfactants, cations) may be used to positively charge crystalline sirolimus particles and/or nanoparticles. Zeta potential measurement may be used to determine particle size and surface charge.

In some embodiments of the devices provided herein the coating comprises a positive surface charge on a surface of the coating configured to contact the treatment site.

In some embodiments of the devices provided herein the coating comprises a surfactant. Some embodiments comprise mixing of sirolimus with surfactant. In some embodiments the surfactant is cationic. Surfactants may comprise at least one of a primary amine having pH < 10, and a secondary amine having pH < 4. In some embodiments surfactant comprises octenidine dihydrochloride. In some embodiments the surfactant comprises a permanently charged quaternary ammonium cation. In some embodiments the permanently charged quaternary ammonium cation comprises at least one of: an Alkyltrimethylammonium salt such as cetyl trimethylammonium bromide (CTAB), hexadecyl trimethyl ammonium bromide, cetyl trimethylammonium chloride (CTAC); Cetylpyridinium chloride (CPC); Polyethoxylated tallow amine (POEA); Benzalkonium chloride (BAC); Benzethonium chloride (BZT); 5-Bromo-5-nitro-1,3-dioxane; Dimethyldioctadecylammonium chloride; and Dioctadecyldimethylammonium bromide (DODAB). In some embodiments the surfactant comprises at least one of: didodecyldimethylammonium bromide (DMAB), linear isoform Polyethylenimine (linear PEI), Branched Low MW Polyethylenimine (PEI) (of about <25KDa), Branched Low MW Polyethylenimine (PEI) (of about <15KDa), Branched Low MW Polyethylenimine (PEI) (of about <10KDa), Branched High MW Polyethylenimine (of about >/=25 KDa), Poly-L-Arginine (average or nominal MW of about 70,000 Da), Poly-L-Arginine (average or nominal MW > about 50,000 Da), Poly-L-Arginine (average or nominal MW of about 5,000 to about 15,000 Da), Poly-L-Lysine (average or nominal MW of about 28,200 Da), Poly-L-Lysine (average or nominal MW of about 67,000 Da), Poly Histidine, Ethylhexadecyldimethylammonium Bromide, Dodecyltrimethyl Ammonium Bromide, Tetradodecylammonium bromide, Dimethylditetradecyl Ammonium bromide, Tetrabutylammonium iodide, DEAE-Dextran hydrochloride, and Hexadimethrine Bromide. The term "about" when used in the context of MW or Mn of various surfactants can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment.

In some embodiments of the devices provided herein the surfactant and the nanoparticles are mixed, lyophilized, and deposited together on the device.

In some embodiments of the devices provided herein the surfactant is deposited on the medical device after the nanoparticles are deposited thereon.

The positive surface charge may be about 20 mV to about 40mV. The positive surface charge may be at least one of: at least about 1 mV, over about 1 mV, at least about 5 mV, at least about 10 mV, about 10 mV to about 50 mV, about 20 mV to about 50 mV, about 10 mV to about 40 mV, about 30 mV to about 40 mV, about 20 mV to about 30 mV, and about 25 mV to about 35 mV. The term "about" when used in the context of charge can mean, for example, variations of 1 mV, 2 mV, 5 mV, 10mV, 1 mV to 5mV, 0 mV to 10mV, 5%, 10%, 25%, 30%, 50%, 75%, 5% to 50%, and/or 5% to 25%, depending on the embodiment.

A method may comprise forming a coating on a medical device comprising depositing a polymer on the medical device using an RESS process mixing a surfactant and nanoparticles of an active agent to prepare a agent-surfactant mixture, lyophilizing the agent-surfactant mixture and depositing the agent-surfactant mixture on the medical device using an eSTAT process.

The coating may release the nanoparticles into a treatment site over at least one of: about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

The devices provided herein may comprise depositing a second polymer on the medical device following depositing the agent-surfactant mixture on the device. The second polymer may comprise PLGA. The PLGA may have at least one of: a MW (weight average molecular weight) of about 30KDa and a Mn (number average molecular weight) of about 15KDa, a Mn of about 10KDa to about 25 KDa, and a MW of about 15 KDa to about 40KDa. The term "about" when used in the context of polymer MW or Mn can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment. Depositing the second polymer on the medical device may use at least one of a RESS coating process, an eSTAT coating process, a dip coating process, and a spray coating process.

One example coating method includes: PLGA (RESS), sirolimus/surfactant particles lyophilized (eSTAT). Another example coating method includes PLGA (RESS), sirolimus (eSTAT), surfactant (Dip Coated).

There are strong correlations between amount of positive charge and cell internalization of nanoparticles. Positive charged nanoparticles have increased ionic interactions with the negatively charged glycosaminoglycan enriched arterial wall. See, e.g. Small 2010, 6, No. 1, 12-2.

Zeta potential is a physical property which is exhibited by any particle in suspension. It can be used to optimize the formulations of suspensions and emulsions. Knowledge of the zeta potential can reduce the time needed to produce trial formulations. It is also an aid in predicting long-term stability.

Three of the fundamental states of matter are solids, liquids and gases. If one of these states is finely dispersed in another then we have a 'colloidal system'. These materials have special properties that are of great practical importance. There are various examples of colloidal systems that include aerosols, emulsions, colloidal suspensions and association colloids. In certain circumstances, the particles in a dispersion may adhere to one another and form aggregates of successively increasing size, which may settle out under the influence of gravity. An initially formed aggregate is called a floc and the process of its formation flocculation. The floc may or may not sediment or phase separate. If the aggregate changes to a much denser form, it is said to undergo coagulation. An aggregate usually separates out either by sedimentation (if it is more dense than the medium) or by creaming (if it less dense than the medium). The terms flocculation and coagulation have often been used interchangeably. Usually coagulation is irreversible whereas flocculation can be reversed by the process of deflocculation.

Zeta potential is a scientific term for electrokinetic potential in colloidal systems. In the colloidal chemistry literature, it is usually denoted using the Greek letter zeta, hence ζ-potential. From a theoretical viewpoint, zeta potential is electric potential in the interfacial double layer (DL) at the location of the slipping plane versus a point in the bulk fluid away from the interface. In other words, zeta potential is the potential difference between the dispersion medium and the stationary layer of fluid attached to the dispersed particle.

A value of 25 mV (positive or negative) can be taken as the arbitrary value that separates low-charged surfaces from highly-charged surfaces.

The significance of zeta potential is that its value can be related to the stability of colloidal dispersions (e.g., a multivitamin syrup). The zeta potential indicates the degree of repulsion between adjacent, similarly charged particles (the vitamins) in a dispersion. For molecules and particles that are small enough, a high zeta potential will confer stability, i.e., the solution or dispersion will resist aggregation. When the potential is low, attraction exceeds repulsion and the dispersion will break and flocculate. So, colloids with high zeta potential (negative or positive) are electrically stabilized while colloids with low zeta potentials tend to coagulate or flocculate as outlined in Table 10.

**TABLE 10**

| Zeta potential [mV] | Stability behavior of the colloid |
|---|---|
| from 0 to ±5, | Rapid coagulation or flocculation |
| from ±10 to ±30 | Incipient instability |
| from ±30 to ±40 | Moderate stability |
| from ±40 to ±60 | Good stability |
| more than ±61 | Excellent stability |

Zeta potential is widely used for quantification of the magnitude of the electrical charge at the double layer. However, zeta potential is not equal to the Stern potential or electric surface potential in the double layer. Such assumptions of equality should be applied with caution. Nevertheless, zeta potential is often the only available path for characterization of double-layer properties. Zeta potential should not be confused with electrode potential or electrochemical potential (because electrochemical reactions are generally not involved in the development of zeta potential).

In some embodiments, the surfactant is anionic. Certain anionic surfactants that may be used, for non-limiting example, include: surfactants based on permanent anions (such as sulfates, sulfonates, and phosphates), surfactants based on pH-dependent anions (such as carboxylates). Sulfates can include, for non-limiting example: Alkyl sulfates such as ammonium lauryl sulfate and sodium lauryl sulfate (SDS); Alkyl ether sulfates such as sodium laureth sulfate (also known as sodium lauryl ether sulfate (SLES)), and sodium myreth sulfate. Sulfonates can include, for non-limiting example: Docusates such as dioctyl sodium sulfosuccinate; Sulfonate fluorosurfactants such as perfluorooctanesulfonate (PFOS), perfluorobutanesulfonate; and Alkyl benzene sulfonates. Phosphates can include, for non-limiting example: Akyl aryl ether phosphate and Alkyl ether phosphate. Carboxylates can include, for non-limiting example, Alkyl carboxylates such as Fatty acid salts (soaps) and sodium stearate; Sodium lauroyl sarcosinate; and Carboxylate fluorosurfactants such as perfluorononanoate, and/or perfluorooctanoate (PFOA or PFO).

### Solubilized sirolimus: nanoemulsion

Nanoemulsions of an active agent (e.g. sirolimus) and a surfactant may be prepared and coated on a medical device such as a balloon as noted elsewhere herein. The medical device may or may not have a polymer coated thereon prior to deposition of the nanoemulsion on the medical device (not according to the invention). The surfactant, in some embodiments, is cationic. Nanoemulsions of an active agent (e.g. sirolimus), a polymer, and a surfactant may be prepared and coated on a medical device (such as a balloon of a balloon catheter) according to processes noted elsewhere herein. The medical device has a polymer coated thereon prior to deposition of the nanoemulsion on the medical device. The surfactant, in some embodiments, is cationic. Depending on the embodiment, there may be a polymer deposited on the medical device following the deposition of the nanoemulsion on the medical device.

A microfluidizer may be used to prepare a nanoemulsion, for example, the microfluidizer processor M-110EH-30 Basic BioPharma Microfluidizer processor by Particle Sciences, Inc. for preparing nanoemulsions and/or nanosuspensions. The process may comprise combination of polymer and active agent (e.g. sirolimus), via emulsion of a polymer (e.g. PLGA) and the active agent (e.g. sirolimus) with a positively charged surfactant mixed in to stabilize the emulsion. This emulsion may then be dip coated or sprayed onto the medical device.

An emulsion is a mixture of two or more immiscible (unblendable) liquids. Emulsions are part of a more general class of two-phase systems of matter called colloids. Although the terms colloid and emulsion are sometimes used interchangeably, emulsion tends to imply that both the dispersed and the continuous phase are liquid. In an emulsion, one liquid (the dispersed phase) is dispersed in the other (the continuous phase). Emulsions are made up of a dispersed and a continuous phase; the boundary between these phases is called the interface. Emulsions are unstable and thus do not form spontaneously. Microemulsions and nanoemulsions tend to appear clear due to the small size of the dispersed phase.

Energy input through shaking, stirring, homogenizing, or spray processes are needed to initially form an emulsion. Over time, emulsions tend to revert to the stable state of the phases comprising the emulsion. Some unstable emulsions quickly separate unless shaken continuously.

There are three types of emulsion instability: flocculation, creaming, and coalescence. Flocculation describes the process by which the dispersed phase comes out of suspension in flakes. Coalescence is another form of instability, which describes when small droplets combine to form progressively larger ones. Emulsions can also undergo creaming, the migration of one of the substances to the top (or the bottom, depending on the relative densities of the two phases) of the emulsion under the influence of buoyancy or centripetal force when a centrifuge is used.

Surface active substances (surfactants) can increase the kinetic stability of emulsions greatly so that, once formed, the emulsion does not change significantly lengths of storage.

An emulsifier (also known as an emulgent) is a substance which stabilizes an emulsion by increasing its kinetic stability. One class of emulsifiers is known as surface active substances, or surfactants. Sometimes the inner phase itself can act as an emulsifier, and the result is nanoemulsion - the inner state disperses into nano-size droplets within the outer phase.

A surfactant can be classified by the presence of formally charged groups in its head. A nonionic surfactant has no charge groups in its head. The head of an ionic surfactant carries a net charge. If the charge is negative, the surfactant is more specifically called anionic; if the charge is positive, it is called cationic. Cationic surfactants can be based on: pH-dependent primary, secondary or tertiary amines: primary amines become positively charged at pH < 10, secondary amines become charged at pH < 4, an example of this is Octenidine dihydrochloride. Cationic surfactants can alternatively be based on permanently charged quaternary ammonium cations. Examples of this include: Alkyltrimethylammonium salts: cetyl trimethylammonium bromide (CTAB) a.k.a. hexadecyl trimethyl ammonium bromide, cetyl trimethylammonium chloride (CTAC); Cetylpyridinium chloride (CPC); Polyethoxylated tallow amine (POEA); Benzalkonium chloride (BAC); Benzethonium chloride (BZT); 5-Bromo-5-nitro-1,3-dioxane; Dimethyldioctadecylammonium chloride; and Dioctadecyldimethylammonium bromide (DODAB).

Provided herein is a coated medical device comprising a medical device for delivering nanoparticles of an active agent to a treatment site; and a coating on the medical device comprising a polymer, a surfactant, and the nanoparticles, wherein the coating is prepared by forming a nanoemulsion comprising the nanoparticles, PLGA, and a surfactant, and wherein the coated medical device delivers a coating portion to the treatment site, which portion releases the nanoparticles into the treatment site over at least about 1 day. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

Provided herein is a coating for a medical device comprising a polymer, a surfactant, and nanoparticles of an active agent, wherein the coating is prepared by forming a nanoemulsion comprising the nanoparticles, polymer, and a surfactant, wherein the device delivers at least a portion of the coating to a treatment site which releases the nanoparticles into the treatment site over at least about 1 day. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

A method may comprise forming a coating on a medical device comprising providing an emulsion of a polymer, nanoparticles of an active agent, and a surfactant, depositing the emulsion on the medical device, wherein the coating delivers the nanoparticles to a treatment site over at least about 1 day. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

When referring to a device or coating that delivers nanoparticles or releases nanoparticles or delivers a portion of the coating or releases a portion of the coating to a treatment site over a certain period of time, the following is meant: First, the device deposits some amount of coating at the treatment site. The device itself (minus the coating that was delivered) may or may not be removed from the treatment site thereafter; however, some amount of coating is left behind at the site. This first process may take a minute, less than a minute, five minutes, a half hour, or another amount of time depending on the embodiment. For non-limiting example, delivery of the coating to the treatment site may last as long as the time it takes to inflate a balloon, hold inflation for a short period, and then deflate and withdraw the balloon.

The portion of the coating that is left behind at the treatment site has an amount of active agent in it, for example, nanoparticles of active agent, and the tissue of the treatment site uptakes the active agent nanoparticles over a period of time-i.e. a second process and second timing. This may be done in various ways, as noted herein and/or known to one of skill in the art, according to various cellular uptake processes and/or according to release of the active agent nanoparticles from a carrier such as a polymer by various degradation processes. The time of this uptake may be 1 day or longer, depending on the embodiment. This second process may be described as the device delivering the active agent (or nanoparticles thereof) to the treatment site over a period of time, or the coating delivering the active agent (or nanoparticles thereof) to the treatment site over a period of time, or the device releasing the active agent (or nanoparticles thereof) to the treatment site over a period of time, or the coating releasing the active agent (or nanoparticles thereof) to the treatment site over a period of time, depending on the embodiment. The second timing, may occur, over at least one of: about 1 day, about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

In some embodiments of the devices provided herein the coating portion delivered to the treatment site releases nanoparticles to the treatment site over at least one of: about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

The active agent in the embodiments of the devices provided herein comprises a macrolide immunosuppressive drug. The active agent may be selected from sirolimus, a prodrug, a derivative, an analog, a hydrate, an ester, and a salt thereof. A portion of the nanoparticles may be in crystalline form. The nanoparticles may be, on average, at least one of: at most 1 micrometer, about 1 micrometer, below about 1 micrometer, below about 750 nanometers (nm), below about 500 nanometers, about 100 nm to about 1 micrometer, about 300 nm to about 1 micrometer, about 100 nm to about 300 nm, about 300 nm to about 500 nm, below about 300nm, below about 100nm, and between about 50nm and about 300 nm. The term "about" when used in the context of nanoparticle size can mean variations of, for example, 5%, 10%, 25%, 50%, 75%, 0 % to 25%, 0% to 50%, 10%-50%, 50nm, 100nm, 10 nm to 100nm, 0 nm to 100 nm, 250nm, 300nm, and/or 0nm to 250nm, depending on the embodiment.

In the embodiments of the devices provided herein the polymer comprises PLGA. The PLGA may have at least one of: a MW of about 30KDa and a Mn of about 15KDa, a Mn of about 10KDa to about 25 KDa, and a MW of about 15 KDa to about 40KDa. The term "about" when used in the context of polymer MW or Mn can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment.

In some embodiments of the devices provided herein the treatment site is a vessel wall. The treatment site may be in or on the body of a subject. The treatment site may be a vascular wall. The treatment site may be a non-vascular lumen wall. The treatment site may be a vascular cavity wall. The treatment site may be a wall of a body cavity. In some embodiments, the body cavity is the result of a lumpectomy. In some embodiments, the treatment site is a cannulized site within a subject. In some embodiments, the treatment site is a sinus wall. In some embodiments, the treatmetn site is a sinus cavity wall. In some embodiments, the active agent comprises a corticosteroid. "Treatment site" or "intervention site" as used herein refers to the location in the body where the coating is intended to be delivered. The treatment site can be any substance in the medium surrounding the device, e.g., tissue, cartilage, a body fluid, etc. The treatment site can be tissue that requires treatment.

In some embodiments of the devices provided herein the medical device comprises a balloon. In some embodiments the medical device comprises a balloon of a balloon catheter. In some embodiments the medical device comprises at least one of a catheter, a balloon, a cutting balloon, a wire guide, a cannula, tooling, an orthopedic device, a structural implant, stent, stent-graft, graft, vena cava filter, a heart valve, cerebrospinal fluid shunts, pacemaker electrodes, axius coronary shunts, endocardial leads, an artificial heart, any implant for insertion into the body of a human or animal subject, including but not limited to stents (e.g., coronary stents, vascular stents including peripheral stents and graft stents, urinary tract stents, urethral/prostatic stents, rectal stent, oesophageal stent, biliary stent, pancreatic stent), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, vascular supports, and a medical device that is not permanantly implanted.

A method may comprise depositing a second polymer on the medical device following depositing the nanoparticles. In some embodiments, a second polymer is deposited on the medical device following the deposition of the nanoparticles. In some embodiments, the coating comprises a second polymer. The second polymer may comprise PLGA. The PLGA has at least one of: a MW of about 30KDa and a Mn of about 15KDa, a Mn of about 10KDa to about 25 KDa, and a MW of about 15 KDa to about 40KDa. The term "about" when used in the context of polymer MW or Mn can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment.In some embodiments depositing the second polymer on the device uses at least one of a RESS coating process, an eSTAT coating process, a dip coating process, and a spray coating process.

In some embodiments of the devices provided herein the coating comprises a positive surface charge on a surface of the coating configured to contact the treatment site.

In some embodiments of the devices provided herein the coating comprises a surfactant. In some embodiments the surfactant is cationic. In some embodiments the surfactant comprises at least one of a primary amine having pH < 10, and a secondary amine having pH < 4. In some embodiments surfactant comprises octenidine dihydrochloride. In some embodiments the surfactant comprises a permanently charged quaternary ammonium cation. In some embodiments the permanently charged quaternary ammonium cation comprises at least one of: an Alkyltrimethylammonium salt such as cetyl trimethylammonium bromide (CTAB), hexadecyl trimethyl ammonium bromide, cetyl trimethylammonium chloride (CTAC); Cetylpyridinium chloride (CPC); Polyethoxylated tallow amine (POEA); Benzalkonium chloride (BAC); Benzethonium chloride (BZT); 5-Bromo-5-nitro-1,3-dioxane; Dimethyldioctadecylammonium chloride; and Dioctadecyldimethylammonium bromide (DODAB). In some embodiments the surfactant comprises at least one of: didodecyldimethylammonium bromide (DMAB), linear isoform Polyethylenimine (linear PEI), Branched Low MW Polyethylenimine (PEI) (of about <25KDa), Branched Low MW Polyethylenimine (PEI) (of about <15KDa), Branched Low MW Polyethylenimine (PEI) (of about <10KDa), Branched High MW Polyethylenimine (of about >/=25 KDa), Poly-L-Arginine (average or nominal MW of about 70,000 Da), Poly-L-Arginine (average or nominal MW > about 50,000 Da), Poly-L-Arginine (average or nominal MW of about 5,000 to about 15,000 Da), Poly-L-Lysine (average or nominal MW of about 28,200 Da), Poly-L-Lysine (average or nominal MW of about 67,000 Da), Poly Histidine, Ethylhexadecyldimethylammonium Bromide, Dodecyltrimethyl Ammonium Bromide, Tetradodecylammonium bromide, Dimethylditetradecyl Ammonium bromide, Tetrabutylammonium iodide, DEAE-Dextran hydrochloride, and Hexadimethrine Bromide. The term "about" when used in the context of MW or Mn of various surfactants can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment.

A method may comprise depositing the emulsion on the medical device uses at least one of a RESS coating process, an eSTAT coating process, a dip coating process, and a spray coating process.

### Solubilized active agent: encapsulated into positively charged polymer (PLGA) nanoparticles (polymer encapsulation)

Solubilized active agent (e.g. sirolimus) in some embodiments is encapsulated into positively charged polymer (PLGA) nanoparticles (polymer encapsulation). These nanocapsules are then coated on the balloon.

Nanoencapsulation of the active agent (e.g. sirolimus) in polymer (e.g. PLGA) with a positively charged surface coating is done using, for example, Phosphorex processes and equipment. A Microfluidics processor (Microfluidizer) may be used additionally or alternatively for nanoencapsulization, e.g. an M-110EH-30 Basic BioPharma Microfluidizer (Particle Sciences, Inc.). Particle Sciences has a M-110EH-30 Basic BioPharma Microfluidizer processor for nanoemulsions, nanosuspensions, cell disruption, and nanoencapsulation in pilot and production volumes. Microfluidics reaction technology (MRT) which is a bottom-up approach may be used for nanoencapsulation. Microfluidics Reaction TechnologyTM (MRT) combines an impinging jet processor with application and process development to prepare nanoparticles bottom-up. Using continuous crystallization, chemical reactions and process intensification, MRT typically enables pharmaceutical, energy and chemical companies to achieve particle sizes impossible with any other method.

The resulting particles of active agent would have sizes only in the range of 1000 nanometers or below, 300 nanometers or below, or below 100 nanometers. The resulting nanoparticles of encapsulated active agent would have sizes only in the range of 1000 nanometers or below, 300 nanometers or below, or below 100 nanometers. In certain embodiments, there is no coating the medical devices (e.g. balloons of a balloon catheter) with a polymer (e.g. PLGA) prior to deposition of the polymer nanoencapsulated active agent particles on the devices, although there may be. In some embodiments, the process would involve an eSTAT processing of the polymer nanoencapsulated active agent particles onto the balloons.

The result, in one example, is the preparion of custom in the 50-150 nm range PLGA (30 kDa, 15 Mn) positively charged (20-40 mV) nanospheres having sirolimus encapsulated therein. 30 kDa is the MW of PLGA. (weight average molecular weight). 15 kDa Mn is the number average molecular weight of the PLGA (i.e. total weight of the sample divided by the number of molecules in the sample). Thus the polydispersity index (PDI) would be 30/15= 2.0 in this embodiment. This process efficiency is ~5% sirolimus encapsulated in Nanoparticles (w/w). That is, in one example embodiment, 5% of the total weight of the nanospheres is Sirolimus. (95% of the weight is the PLGA in the nanosphere). In one example embodiment, 10-25% of the total weight of the nanospheres is Sirolimus. (95% of the weight is the PLGA in the nanosphere).

To coat the balloon, in some embodiments, the encapsulated active agent (e.g. sirolimus) is coated on the medical device (e.g. a balloon of a balloon catheter) using the eSTAT process noted previously. In other embodiments, the encapsulated active agent is dip coated on the medical device. The result, in some embodiments where this process is used and the active agent is sirolimus and the polymer is PLGA, is that the artery wall uptake is about 5 micrograms -10 micrograms (total content per lesion for an average length lesion e.g. 17 mm to 23 mm length) of sirolimus upon delivery of the coating to the artery lesion over a therapeutic period of time, for example, over at least 1 day and in some embodiments over a longer period as noted elsewhere herein. This content of arterial uptake may be scaled according to lesion length variations (e.g. more for longer lesions, less for shorter lesions). It also varies according to the polymer used and active agent used.

Example of Customized PLGA-Sirolimus nanospheres: Sirolimus is enttrapped in PLGA nanoparticles made by a Phosphorex process; the nanoparticles having a positive (+) charge, and the nanospheres being about 150 nm on average. The process results in ~5% encapsulated w/w Sirolimus. (that is, by weight, 5% of the wt is Sirolimus, 95% of the wt is PLGA).

Coating efficiency using these active agent encapsulated in polymer nanospheres and eSTAT process for coating these nanospheres on a medical device (e.g. a balloon of a balloon catheter) as noted herein may be, for example, if there is 15.789 mg of the nanospheres, .789mg (or 5%) is active agent (e.g. Sirolimus as noted above). Since the estimated coating efficiency of the eSTAT process is 6%, then where 12 balloons are coated with this process having 15.789 mg of PLGA-Sirolimus nanospheres ejected into the chamber, 47 ug of sirolimus will be deposited on the balloons, or 3.95ug of Sirolmus deposited per balloon.

When referring to a device that delivers at least a portion of the coating to the treatment site, and the portion (or the device) delivers (or releases) the active agent nanoparticles into (or to) the treatment site over a certain period of time, the following is meant: First, the device deposits some amount of coating at the treatment site. The device itself (minus the coating that was delivered) may or may not be removed from the treatment site thereafter; however, some amount of coating is left behind at the site. This first process may take a minute, less than a minute, five minutes, a half hour, or another amount of time depending on the embodiment. For non-limiting example delivery of the coating to the treatment site may last as long as the time it takes to inflate a balloon, hold inflation for a short period, and then deflate and withdraw the balloon.

The portion of the coating that is left behind at the treatment site has an amount of active agent in it, for example, nanoparticles of active agent, and the tissue of the treatment site uptakes the active agent nanoparticles over a period of time-i.e. a second process and second timing. This may be done in various ways, as noted herein and/or known to one of skill in the art, according to various cellular uptake processes and/or according to release of the active agent nanoparticles from a carrier such as a polymer by various degradation processes. The time of this uptake may be 1 day or longer, depending on the embodiment. This second process may be described as the device delivering the active agent (or nanoparticles thereof) to the treatment site over a period of time, or the coating delivering the active agent (or nanoparticles thereof) to the treatment site over a period of time, or the device releasing the active agent (or nanoparticles thereof) to the treatment site over a period of time, or the coating releasing the active agent (or nanoparticles thereof) to the treatment site over a period of time, depending on the embodiment. The second timing, may occur, over at least one of: about 1 day, about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

In some embodiments of the devices provided herein the coating delivers the active agent nanoparticles to the treatment site over at least about 1 day. In some embodiments of the devices provided herein the coating delivers the active agent nanoparticles to the treatment site over at least one of: about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days. The term "about" when used in the context of release timing of active agent nanoparticles to the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

In some embodiments of the devices provided herein the polymer comprises PLGA. The PLGA may have at least one of: a MW of about 30KDa and a Mn of about 15KDa, a Mn of about 10KDa to about 25 KDa, and a MW of about 15 KDa to about 40KDa. The term "about" when used in the context of polymer MW or Mn can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment.

In some embodiments of the devices provided herein the coating comprises a positive surface charge. The positive surface charge may be about 20 mV to about 40mV. The positive surface charge may be at least one of: at least about 1 mV, over about 1 mV, at least about 5 mV, at least about 10 mV, about 10 mV to about 50 mV, about 20 mV to about 50 mV, about 10 mV to about 40 mV, about 30 mV to about 40 mV, about 20 mV to about 30 mV, and about 25 mV to about 35 mV. The term "about" when used in the context of charge can mean, for example, variations of 1 mV, 2 mV, 5 mV, 10mV, 1 mV to 5mV, 0 mV to 10mV, 5%, 10%, 25%, 30%, 50%, 75%, 5% to 50%, and/or 5% to 25%, depending on the embodiment.

In some embodiments of the devices provided herein, the w/w percent of active agent in the encapsulated active agent nanoparticles is about 5%. In some embodiments of the devices provided herein, the w/w percent of active agent in the encapsulated active agent nanoparticles is about 10-25%. In some embodiments of the devices provided herein, the w/w percent of active agent in the encapsulated active agent nanoparticles is at least one of: about 10%, about 25%, about 50%, about 5% to about 25%, about 5% to about 30 %, and about 10% to about 25%. The term "about" when used in the context of w/w percent of active agent nanoparticles in the encapsulated active agent nanoparticles can mean variations of (on an absolute percentage- and not a percent of a percent), for example, 1%, 5%, 10%, 0.5%, 0.75%, 1 % to 5%, 1% to 10%, 0% to 10%, 0% to 5%, 0.5% to 5%, 0.25%, 0.1%, and 0.25% to 1%, depending on the embodiment. For example a w/w percent of 7.5% of active agent nanoparticles in the encapsulated active agent nanoparticles can fall within a specification of about 10% where the variability is 5%, as this would mean the range is 5% to 15%-thus w/w percentages ranging 5%-15% would be equivalent to about 10%.

In some embodiments of the devices provided herein, at least a portion of the encapsulated active agent nanoparticles are nanospheres. At least a portion of the encapsulated active agent nanoparticles may be at least one of: a discoidal shape, a hemispherical shape, a cylindrical shape, a conical shape, a nanoreef shape, a nanobox shape, a cluster shape, a nanotube shape, a whisker shape, a rod shape, a fiber shape, a cup shape, a jack shape, a hexagonal shape, an ellipsoid shape, an oblate ellipsoid shape, a prolate ellipsoid shape, a torus shape, a spheroid shape, a taco-like shape, a bullet shape, a barrel shape, a lens shape, a capsule shape, a pulley wheel shape, a circular disc shape, a rectangular disc shape, a hexagonal disc shape, a flying saucer-like shape, a worm shape, a ribbon-like shape, and a ravioli-like shape.

The active agent in some embodiments of the devices provided herein comprises a macrolide immunosuppressive drug. The active agent may be selected from sirolimus, a prodrug, a derivative, an analog, a hydrate, an ester, and a salt thereof. A portion of the nanoparticles may be in crystalline form. The active agent nanoparticles may be, on average, at least one of: at most 1 micrometer, about 1 micrometer, below about 1 micrometer, below about 750 nanometers (nm), below about 500 nanometers, about 100 nm to about 1 micrometer, about 300 nm to about 1 micrometer, about 100 nm to about 300 nm, about 300 nm to about 500 nm, below about 300nm, below about 100nm, and between about 50nm and about 300 nm. The encapsulated active agent nanoparticles may be, on average, at least one of: at most 1 micrometer, about 1 micrometer, below about 1 micrometer, below about 750 nanometers (nm), below about 500 nanometers, about 100 nm to about 1 micrometer, about 300 nm to about 1 micrometer, about 100 nm to about 300 nm, about 300 nm to about 500 nm, below about 300nm, below about 100nm, and between about 50nm and about 300 nm. The term "about" when used in the context of nanoparticle size can mean variations of, for example, 5%, 10%, 25%, 50%, 75%, 0 % to 25%, 0% to 50%, 10%-50%, 50nm, 100nm, 10 nm to 100nm, 0 nm to 100 nm, 250nm, 300nm, and/or 0nm to 250nm, depending on the embodiment.

In some embodiments of the devices provided herein the treatment site is a vessel wall. The treatment site may be in or on the body of a subject. The treatment site may be a vascular wall. The treatment site may be a non-vascular lumen wall. The treatment site may be a vascular cavity wall. The treatment site may be a wall of a body cavity. In some embodiments, the body cavity is the result of a lumpectomy. In some embodiments, the treatment site is a cannulized site within a subject. In some embodiments, the treatment site is a sinus wall. In some embodiments, the treatmetn site is a sinus cavity wall. In some embodiments, the active agent comprises a corticosteroid. "Treatment site" or "intervention site" as used herein refers to the location in the body where the coating is intended to be delivered. The treatment site can be any substance in the medium surrounding the device, e.g., tissue, cartilage, a body fluid, etc. The treatment site can be tissue that requires treatment.

In some embodiments of the devices provided herein the coating comprises a positive surface charge on a surface of the coating configured to contact the treatment site.

In some embodiments of the devices provided herein the encapsulated active agent nanoparticles are micelles.

In some embodiments of the devices provided herein the medical device comprises a balloon. In some embodiments the medical device comprises a balloon of a balloon catheter. In some embodiments the medical device comprises at least one of a catheter, a balloon, a cutting balloon, a wire guide, a cannula, tooling, an orthopedic device, a structural implant, stent, stent-graft, graft, vena cava filter, a heart valve, cerebrospinal fluid shunts, pacemaker electrodes, axius coronary shunts, endocardial leads, an artificial heart, any implant for insertion into the body of a human or animal subject, including but not limited to stents (e.g., coronary stents, vascular stents including peripheral stents and graft stents, urinary tract stents, urethral/prostatic stents, rectal stent, oesophageal stent, biliary stent, pancreatic stent), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, vascular supports, and a medical device that is not permanantly implanted.

In some embodiments of the devices provided herein depositing the encapsulated active agent nanoparticles comprises using an eSTAT process. In some embodiments of the devices provided herein depositing a second polymer on the medical device following depositing the encapsulated active agent nanoparticles on the medical device.

In some embodiments of the devices provided herein the second polymer comprises PLGA. The PLGA may have at least one of: a MW of about 30KDa and a Mn of about 15KDa, a Mn of about 10KDa to about 25 KDa, and a MW of about 15 KDa to about 40KDa. The term "about" when used in the context of polymer MW or Mn can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment. Depositing the second polymer on the medical device may use at least one of a RESS coating process, an eSTAT coating process, a dip coating process, and a spray coating process.

Dip coating processes to coat a medical device with a polymer encapsulated active agent may be adjusted according to known methods and variations thereof known to one of ordinary skill in the art to achieve the desired tissue uptake and coating content based on the active agent and polymer used to encapsule the active agent. In a dip coating process, however, potential therapeutic and stability benefits of crystalline drug are reduced as compared to a process which keeps the active agent (drug) in its crystalline form (at least in part). Nevertheless, this may be a viable coating for a balloon to deliver a therapeutic effect of sirolimus (or any active agent noted herein) to the treatment site (e.g. vessel) over time.

In some embodiments, micelles for drug delivery are prepared and applied to the balloon as a coating. Micelles are self-assembling nanosized colloidal particles with a hydrophobic core and hydrophilic shell. Polymeric micelles possess high stability both in vitro and in vivo. They have good biocompatibility, and solubilize a broad variety of poorly soluble pharmaceuticals. Micelles as target drug delivery systems can have an enhanced permeability/retention effect into compromised vasculature. They can have specific targeting ligand molecules on the micelle surface. They can formed of stimuli-responsive amphiphilic block-copolymers . See, e.g. Reference Pharmaceutical Research, Vol. 24, No. 1, January 2007, incorporated herein by reference in its entirety.

Other Block Copolymers Used to Prepare Micelles Loaded with Various Pharmaceuticals may include, for non-limiting example: pluronics, Pluronic/polyethyleneimine, Polycaprolactone-b-PEG, Poly(delta-valerolactone)-b-methoxy-PEG, Polycaprolactone-b-methoxy-PEG, Poly(caprolacton/trimethylene carbonate)-PEG, Poly(aspartic acid)-b-PEG, Poly(glutamic acid)-b-PEG, Poly(benzyl-L-glutamate)-b-PEG, Poly(D,L-lactide)-b-methoxy-PEG, Poly(benzyl-L-aspartate)-b, Poly(hydroxy-ethylene oxide), Poly(2-ethyl-2-oxazoline)-b-poly((-caprolactone), Poly(2-ethyl-2-oxazoline)-b-poly(L-lactide), PEG-lipid, Various polymer-lipid conjugates, Poly(L-histidine)-b-PEG (folate-targeted), and Chitosan grafted with palmitoyl.
Mixed micelles of Pluronic F-127 and PPS-PEO block copolymer modified to display sulfate groups on the terminus of the PEO block act as a heparin mimics and bind to collagen in the extracellular matrix. Mixed micelles with sulfate functionality demonstrated enhanced collagen I binding, suggestive of the potential for binding to the extracellular milieu. See, e.g. Journal of Controlled Release 137 (2009) 146-151) Nanoparticles without Polymer (devices, coatings, methods of coating)

Provided herein is a coated medical device comprising: a medical device for delivering nanoparticles of an active agent to a treatment site; and a coating on the device comprising the active agent nanoparticles, wherein the coated medical device delivers at least a portion of the coating to the treatment site which portion releases active agent nanoparticles into the treatment site over at least about 1 day. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

Provided herein is a coating for a medical device comprising nanoparticles of an active agent, wherein the coating delivers the nanoparticles into a treatment site over at least about 1 day. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

A method may comprise forming coating on a medical device with nanoparticles of an active agent comprising depositing the nanoparticles on the medical device using an eSTAT process.

The active agent in the embodiments of the devices provided herein comprises a macrolide immunosuppressive drug. The active agent may be selected from sirolimus, a prodrug, a derivative, an analog, a hydrate, an ester, and a salt thereof. A portion of the nanoparticles may be in crystalline form. The nanoparticles may be, on average, at least one of: at most 1 micrometer, about 1 micrometer, below about 1 micrometer, below about 750 nanometers (nm), below about 500 nanometers, about 100 nm to about 1 micrometer, about 300 nm to about 1 micrometer, about 100 nm to about 300 nm, about 300 nm to about 500 nm, below about 300nm, below about 100nm, and between about 50nm and about 300 nm. The term "about" when used in the context of nanoparticle size can mean variations of, for example, 5%, 10%, 25%, 50%, 75%, 0 % to 25%, 0% to 50%, 10%-50%, 50nm, 100nm, 10 nm to 100nm, 0 nm to 100 nm, 250nm, 300nm, and/or 0nm to 250nm, depending on the embodiment.

When referring to a device that delivers at least a portion of the coating to the treatment site, and the portion (or the device) delivers (or releases) the active agent nanoparticles into (or to) the treatment site over a certain period of time, the following is meant: First, the device deposits some amount of coating at the treatment site. The device itself (minus the coating that was delivered) may or may not be removed from the treatment site thereafter; however, some amount of coating is left behind at the site. This first process may take a minute, less than a minute, five minutes, a half hour, or another amount of time depending on the embodiment. For non-limiting example delivery of the coating to the treatment site may last as long as the time it takes to inflate a balloon, hold inflation for a short period, and then deflate and withdraw the balloon.

The portion of the coating that is left behind at the treatment site has an amount of active agent in it, for example, nanoparticles of active agent, and the tissue of the treatment site uptakes the active agent nanoparticles over a period of time-i.e. a second process and second timing. This may be done in various ways, as noted herein and/or known to one of skill in the art, according to various cellular uptake processes. The time of this uptake may be 1 day or longer, depending on the embodiment. This second process may be described as the device delivering the active agent (or nanoparticles thereof) to the treatment site over a period of time, or the coating delivering the active agent (or nanoparticles thereof) to the treatment site over a period of time, or the device releasing the active agent (or nanoparticles thereof) to the treatment site over a period of time, or the coating releasing the active agent (or nanoparticles thereof) to the treatment site over a period of time, depending on the embodiment. The second timing, may occur, over at least one of: about 1 day, about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

In some embodiments of the devices provided herein the coating portion delivered to the treatment site releases nanoparticles into the treatment site over at least one of: about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days. The term "about" when used in the context of release timing of active agent nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

In some embodiments of the devices provided herein the treatment site is a vessel wall. The treatment site may be in or on the body of a subject. The treatment site may be a vascular wall. The treatment site may be a non-vascular lumen wall. The treatment site may be a vascular cavity wall. The treatment site may be a wall of a body cavity. In some embodiments, the body cavity is the result of a lumpectomy. In some embodiments, the treatment site is a cannulized site within a subject. In some embodiments, the treatment site is a sinus wall. In some embodiments, the treatmetn site is a sinus cavity wall. In some embodiments, the active agent comprises a corticosteroid. "Treatment site" or "intervention site" as used herein refers to the location in the body where the coating is intended to be delivered. The treatment site can be any substance in the medium surrounding the device, e.g., tissue, cartilage, a body fluid, etc. The treatment site can be tissue that requires treatment.

In some embodiments of the devices provided herein the medical device comprises a balloon. In some embodiments the medical device comprises a balloon of a balloon catheter. In some embodiments the medical device comprises at least one of a catheter, a balloon, a cutting balloon, a wire guide, a cannula, tooling, an orthopedic device, a structural implant, stent, stent-graft, graft, vena cava filter, a heart valve, cerebrospinal fluid shunts, pacemaker electrodes, axius coronary shunts, endocardial leads, an artificial heart, any implant for insertion into the body of a human or animal subject, including but not limited to stents (e.g., coronary stents, vascular stents including peripheral stents and graft stents, urinary tract stents, urethral/prostatic stents, rectal stent, oesophageal stent, biliary stent, pancreatic stent), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, vascular supports, and a medical device that is not permanantly implanted.

Some embodiments of the devices provided comprise depositing a polymer on the medical device following depositing the nanoparticles. The polymer may comprise PLGA. The PLGA has at least one of: a MW of about 30KDa and a Mn of about 15KDa, a Mn of about 10KDa to about 25 KDa, and a MW of about 15 KDa to about 40KDa. The term "about" when used in the context of polymer MW or Mn can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment. In some embodiments depositing the polymer on the device uses at least one of a RESS coating process, an eSTAT coating process, a dip coating process, and a spray coating process.

In some embodiments of the devices provided herein the coating comprises a positive surface charge on a surface of the coating configured to contact the treatment site.

In some embodiments of the devices provided herein the coating comprises a surfactant. In some embodiments the surfactant is cationic. In some embodiments the surfactant comprises at least one of a primary amine having pH < 10, and a secondary amine having pH < 4. In some embodiments surfactant comprises octenidine dihydrochloride. In some embodiments the surfactant comprises a permanently charged quaternary ammonium cation. In some embodiments the permanently charged quaternary ammonium cation comprises at least one of: an Alkyltrimethylammonium salt such as cetyl trimethylammonium bromide (CTAB), hexadecyl trimethyl ammonium bromide, cetyl trimethylammonium chloride (CTAC); Cetylpyridinium chloride (CPC); Polyethoxylated tallow amine (POEA); Benzalkonium chloride (BAC); Benzethonium chloride (BZT); 5-Bromo-5-nitro-1,3-dioxane; Dimethyldioctadecylammonium chloride; and Dioctadecyldimethylammonium bromide (DODAB). In some embodiments the surfactant comprises at least one of: didodecyldimethylammonium bromide (DMAB), linear isoform Polyethylenimine (linear PEI), Branched Low MW Polyethylenimine (PEI) (of about <25KDa), Branched Low MW Polyethylenimine (PEI) (of about <15KDa), Branched Low MW Polyethylenimine (PEI) (of about <10KDa), Branched High MW Polyethylenimine (of about >/=25 KDa), Poly-L-Arginine (average or nominal MW of about 70,000 Da), Poly-L-Arginine (average or nominal MW > about 50,000 Da), Poly-L-Arginine (average or nominal MW of about 5,000 to about 15,000 Da), Poly-L-Lysine (average or nominal MW of about 28,200 Da), Poly-L-Lysine (average or nominal MW of about 67,000 Da), Poly Histidine, Ethylhexadecyldimethylammonium Bromide, Dodecyltrimethyl Ammonium Bromide, Tetradodecylammonium bromide, Dimethylditetradecyl Ammonium bromide, Tetrabutylammonium iodide, DEAE-Dextran hydrochloride, and Hexadimethrine Bromide. The term "about" when used in the context of MW or Mn of various surfactants can mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment.

In some embodiments of the devices provided herein the surfactant and the nanoparticles are mixed, lyophilized, and deposited together on the device. In some embodiments of the devices provided herein the surfactant is deposited on the medical device after the nanoparticles are deposited thereon.

A method may comprise forming a coating on a medical device comprising mixing a surfactant and nanoparticles of an active agent to prepare a agent-surfactant mixture, lyophilizing the agent-surfactant mixture, and depositing the agent-surfactant mixture on the device using an eSTAT process.

The coating may release the nanoparticles into a treatment site over at least one of: about 1 day, about 3 days, about 5 days, about 1 week, about 1.5 weeks, about 2 weeks, about 14 days, about 3 weeks, about 21 days, about 4 weeks, about 28 days, about 1 month, about 1.5 months, about 2 months, at least about 1 day, at least about 3 days, at least about 5 days, at least about 1 week, at least about 1.5 weeks, at least about 2 weeks, at least about 14 days, at least about 3 weeks, at least about 21 days, at least about 4 weeks, at least about 28 days, at least about 1 month, at least about 1.5 months, at least about 2 months, about 7 to about 14 days, about 14 to about 21 days, about 14 to about 28 days, about 21 to about 28 days, and about 7 to about 28 days. The term "about" when used in the context of release timing of nanoparticles into the treatment site can mean variations of, for example, up to 10%, up to 25%, up to 50%, up to 12 hrs, up to 6 hrs, up to 3 hrs, up to 2 hrs, 2-6 hrs, 1-12 hrs, depending on the embodiment.

In the embodiments of the devices provided herein the coating on the medical device comprises a positive surface charge. In some embodiments, the surfactant of the agent-surfactant mixture is cationic. In some embodiments, the surfactant comprises a primary amines having pH < 10, and a secondary amines having pH < 4.

In some embodiments, the surfactant comprises octenidine dihydrochloride. In some embodiments, the surfactant comprises a permanently charged quaternary ammonium cation. In some embodiments, the permanently charged quaternary ammonium cation comprises at least one of: an Alkyltrimethylammonium salt such as cetyl trimethylammonium bromide (CTAB), hexadecyl trimethyl ammonium bromide, cetyl trimethylammonium chloride (CTAC); Cetylpyridinium chloride (CPC); Polyethoxylated tallow amine (POEA); Benzalkonium chloride (BAC); Benzethonium chloride (BZT); 5-Bromo-5-nitro-1,3-dioxane; Dimethyldioctadecylammonium chloride; and Dioctadecyldimethylammonium bromide (DODAB). In some embodiments, the surfactant comprises at least one of: didodecyldimethylammonium bromide (DMAB), linear isoform Polyethylenimine (linear PEI), Branched Low MW Polyethylenimine (PEI) (of about <25KDa), Branched Low MW Polyethylenimine (PEI) (of about <15KDa), Branched Low MW Polyethylenimine (PEI) (of about <10KDa), Branched High MW Polyethylenimine (of about >/=25 KDa), Poly-L-Arginine (average or nominal MW of about 70,000 Da), Poly-L-Arginine (average or nominal MW > about 50,000 Da), Poly-L-Arginine (average or nominal MW of about 5,000 to about 15,000 Da), Poly-L-Lysine (average or nominal MW of about 28,200 Da), Poly-L-Lysine (average or nominal MW of about 67,000 Da), Poly Histidine, Ethylhexadecyldimethylammonium Bromide, Dodecyltrimethyl Ammonium Bromide, Tetradodecylammonium bromide, Dimethylditetradecyl Ammonium bromide, Tetrabutylammonium iodide, DEAE-Dextran hydrochloride, and Hexadimethrine Bromide. The term "about" when used in the context of MW or Mn can of various surfactants mean variations of, for example, 5%, 10%, 25%, 50%, 75%, 0 % to 25%, 0% to 50%, 10%-50%, 1kDa, 5kDa, 10kDa, 500Da, 200Da, 100 Da to 500Da, 0 Da to 1Kda, 0 Da to 5 kDa, 0 Da to 10 kDa, and/or 100Da to 1kDa, depending on the embodiment.

A method may comprise comprises depositing a polymer on the medical device following depositing the agent-surfactant mixture on the device. The polymer may comprise PLGA. In some embodiments, depositing the polymer on the medical device uses at least one of a RESS coating process, an eSTAT coating process, a dip coating process, and a spray coating process.

In some embodiments of the devices provided herein the medical device comprises a balloon. In some embodiments the medical device comprises a balloon of a balloon catheter. In some embodiments the medical device comprises at least one of a catheter, a balloon, a cutting balloon, a wire guide, a cannula, tooling, an orthopedic device, a structural implant, stent, stent-graft, graft, vena cava filter, a heart valve, cerebrospinal fluid shunts, pacemaker electrodes, axius coronary shunts, endocardial leads, an artificial heart, any implant for insertion into the body of a human or animal subject, including but not limited to stents (e.g., coronary stents, vascular stents including peripheral stents and graft stents, urinary tract stents, urethral/prostatic stents, rectal stent, oesophageal stent, biliary stent, pancreatic stent), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, vascular supports, and a medical device that is not permanantly implanted.

"Active agent" as used herein refers to any pharmaceutical agent or active biological agent as described herein.

"Activity" as used herein refers to the ability of a pharmaceutical or active biological agent to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). Thus the activity of a pharmaceutical or active biological agent should be of therapeutic or prophylactic value.

"Therapeutically desirable morphology" as used herein refers to the gross form and structure of the pharmaceutical agent, once deposited on the substrate, so as to provide for optimal conditions of ex vivo storage, in vivo preservation and/or in vivo release. Such optimal conditions may include, but are not limited to increased shelf life, increased in vivo stability, good biocompatibility, good bioavailability or modified release rates. Typically, for the present invention, the desired morphology of a pharmaceutical agent would be crystalline or semi-crystalline or amorphous, although this may vary widely depending on many factors including, but not limited to, the nature of the pharmaceutical agent, the disease to be treated/prevented, the intended storage conditions for the substrate prior to use or the location within the body of any biomedical implant. Preferably 100% of the pharmaceutical agent is in crystalline form.

"Secondary, tertiary and quaternary structure " as used herein are defined as follows. The active biological agents of the present invention will typically possess some degree of secondary, tertiary and/or quaternary structure, upon which the activity of the agent depends. As an illustrative, non-limiting example, proteins possess secondary, tertiary and quaternary structure. Secondary structure refers to the spatial arrangement of amino acid residues that are near one another in the linear sequence. The α-helix and the β-strand are elements of secondary structure. Tertiary structure refers to the spatial arrangement of amino acid residues that are far apart in the linear sequence and to the pattern of disulfide bonds. Proteins containing more than one polypeptide chain exhibit an additional level of structural organization. Each polypeptide chain in such a protein is called a subunit. Quaternary structure refers to the spatial arrangement of subunits and the nature of their contacts. For example hemoglobin consists of two α and two β chains. It is well known that protein function arises from its conformation or three dimensional arrangement of atoms (a stretched out polypeptide chain is devoid of activity). Thus one aspect of the present invention is to manipulate active biological agents, while being careful to maintain their conformation, so as not to lose their therapeutic activity.

"Pharmaceutical agent" as used herein refers to any of a variety of drugs or pharmaceutical compounds that can be used as active agents to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). It is possible that the pharmaceutical agents of the invention may also comprise two or more drugs or pharmaceutical compounds.

In some embodiments, the active agent is selected from rapamycin, a prodrug, a derivative, an analog, a hydrate, an ester, and a salt thereof. In some embodiments, the active agent is selected from sirolimus, a prodrug, a derivative, an analog, a hydrate, an ester, and a salt thereof. As used herein, rapamycin and sirolimus are interchangable terms. In some embodiments, the active agent is selected from one or more of sirolimus, everolimus, zotarolimus and biolimus. In some embodiments, the active agent comprises a macrolide immunosuppressive (limus) drug. In some embodiments, the macrolide immunosuppressive drug comprises one or more of rapamycin, biolimus (biolimus A9), 40-O-(2-Hydroxyethyl)rapamycin (everolimus), 40-O-Benzyl-rapamycin, 40-O-(4'-Hydroxymethyl)benzyl-rapamycin, 40-O-[4'-(1,2-Dihydroxyethyl)]benzyl-rapamycin, 40-O-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 4O-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 4O-O-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 4O-O-(2-Aminoethyl)-rapamycin, 4O-O-(2-Acetaminoethyl)-rapamycin 4O-O-(2-Nicotinamidoethyl)-rapamycin, 4O-O-(2-(N-Methyl-imidazo-2'-ylcarbethoxamido)ethyl)-rapamycin, 4O-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), 42-[3-hydroxy-2-(hydroxymethyl)-2-methylpropanoate]rapamycin (temsirolimus), (42S)-42-Deoxy-42-(1H-tetrazol-1-yl)-rapamycin (zotarolimus), picrolimus, novolimus, and salts, derivatives, isomers, racemates, diastereoisomers, prodrugs, hydrate, ester, or analogs thereof.

The pharmaceutical agents may, if desired, also be used in the form of their pharmaceutically acceptable salts or derivatives (meaning salts which retain the biological effectiveness and properties of the compounds of this invention and which are not biologically or otherwise undesirable), and in the case of chiral active ingredients it is possible to employ both optically active isomers and racemates or mixtures of diastereoisomers. As well, the pharmaceutical agent may include a prodrug, a hydrate, an ester, a polymorph, a derivative or analogs of a compound or molecule.

The pharmaceutical agent may be an antibiotic agent.

Pharmaceutical agents, include but are not limited to paclitaxel. Pharmaceutical agents, include but are not limited to NO donors. Pharmaceutical agents, include but are not limited to phosphorylcholine. Pharmaceutical agents, include but are not limited to tretinoin. Pharmaceutical agents, include but are not limited to dexamethasone. Pharmaceutical agents, include but are not limited to melatonin. Pharmaceutical agents, include but are not limited to antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals, chemotherapeutic agents and amino acids. Examples of suitable active ingredients are acarbose, antigens, beta-receptor blockers, non-steroidal antiinflammatory drugs [NSAIDs], cardiac glycosides, acetylsalicylic acid, virustatics, aclarubicin, acyclovir, cisplatin, actinomycin, alpha- and beta-sympatomimetics, (dmeprazole, allopurinol, alprostadil, prostaglandins, amantadine, ambroxol, amlodipine, methotrexate, S-aminosalicylic acid, amitriptyline, amoxicillin, anastrozole, atenolol, azathioprine, balsalazide, beclomethasone, betahistine, bezafibrate, bicalutamide, diazepam and diazepam derivatives, budesonide, bufexamac, buprenorphine, methadone, calcium salts, potassium salts, magnesium salts, candesartan, carbamazepine, captopril, cefalosporins, cetirizine, chenodeoxycholic acid, ursodeoxycholic acid, theophylline and theophylline derivatives, trypsins, cimetidine, clarithromycin, clavulanic acid, clindamycin, clobutinol, clonidine, cotrimoxazole, codeine, caffeine, vitamin D and derivatives of vitamin D, colestyramine, cromoglicic acid, coumarin and coumarin derivatives, cysteine, cytarabine, cyclophosphamide, ciclosporin, cyproterone, cytabarine, dapiprazole, desogestrel, desonide, dihydralazine, diltiazem, ergot alkaloids, dimenhydrinate, dimethyl sulphoxide, dimeticone, domperidone and domperidan derivatives, dopamine, doxazosin, doxorubizin, doxylamine, dapiprazole, benzodiazepines, diclofenac, glycoside antibiotics, desipramine, econazole, ACE inhibitors, enalapril, ephedrine, epinephrine, epoetin and epoetin derivatives, morphinans, calcium antagonists, irinotecan, modafinil, orlistat, peptide antibiotics, phenytoin, riluzoles, risedronate, sildenafil, topiramate, macrolide antibiotics, oestrogen and oestrogen derivatives, progestogen and progestogen derivatives, testosterone and testosterone derivatives, androgen and androgen derivatives, ethenzamide, etofenamate, etofibrate, fenofibrate, etofylline, etoposide, famciclovir, famotidine, felodipine, fenofibrate, fentanyl, fenticonazole, gyrase inhibitors, fluconazole, fludarabine, fluarizine, fluorouracil, fluoxetine, flurbiprofen, ibuprofen, flutamide, fluvastatin, follitropin, formoterol, fosfomicin, furosemide, fusidic acid, gallopamil, ganciclovir, gemfibrozil, gentamicin, ginkgo, Saint John's wort, glibenclamide, urea derivatives as oral antidiabetics, glucagon, glucosamine and glucosamine derivatives, glutathione, glycerol and glycerol derivatives, hypothalamus hormones, goserelin, gyrase inhibitors, guanethidine, halofantrine, haloperidol, heparin and heparin derivatives, hyaluronic acid, hydralazine, hydrochlorothiazide and hydrochlorothiazide derivatives, salicylates, hydroxyzine, idarubicin, ifosfamide, imipramine, indometacin, indoramine, insulin, interferons, iodine and iodine derivatives, isoconazole, isoprenaline, glucitol and glucitol derivatives, itraconazole, ketoconazole, ketoprofen, ketotifen, lacidipine, lansoprazole, levodopa, levomethadone, thyroid hormones, lipoic acid and lipoic acid derivatives, lisinopril, lisuride, lofepramine, lomustine, loperamide, loratadine, maprotiline, mebendazole, mebeverine, meclozine, mefenamic acid, mefloquine, meloxicam, mepindolol, meprobamate, meropenem, mesalazine, mesuximide, metamizole, metformin, methotrexate, methylphenidate, methylprednisolone, metixene, metoclopramide, metoprolol, metronidazole, mianserin, miconazole, minocycline, minoxidil, misoprostol, mitomycin, mizolastine, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, norfloxacin, novamine sulfone, noscapine, nystatin, ofloxacin, olanzapine, olsalazine, omeprazole, omoconazole, ondansetron, oxaceprol, oxacillin, oxiconazole, oxymetazoline, pantoprazole, paracetamol, paroxetine, penciclovir, oral penicillins, pentazocine, pentifylline, pentoxifylline, perphenazine, pethidine, plant extracts, phenazone, pheniramine, barbituric acid derivatives, phenylbutazone, phenytoin, pimozide, pindolol, piperazine, piracetam, pirenzepine, piribedil, piroxicam, pramipexole, pravastatin, prazosin, procaine, promazine, propiverine, propranolol, propyphenazone, prostaglandins, protionamide, proxyphylline, quetiapine, quinapril, quinaprilat, ramipril, ranitidine, reproterol, reserpine, ribavirin, rifampicin, risperidone, ritonavir, ropinirole, roxatidine, roxithromycin, ruscogenin, rutoside and rutoside derivatives, sabadilla, salbutamol, salmeterol, scopolamine, selegiline, sertaconazole, sertindole, sertralion, silicates, sildenafil, simvastatin, sitosterol, sotalol, spaglumic acid, sparfloxacin, spectinomycin, spiramycin, spirapril, spironolactone, stavudine, streptomycin, sucralfate, sufentanil, sulbactam, sulphonamides, sulfasalazine, sulpiride, sultamicillin, sultiam, sumatriptan, suxamethonium chloride, tacrine, tacrolimus, taliolol, tamoxifen, taurolidine, tazarotene, temazepam, teniposide, tenoxicam, terazosin, terbinafine, terbutaline, terfenadine, terlipressin, tertatolol, tetracyclins, teryzoline, theobromine, theophylline, butizine, thiamazole, phenothiazines, thiotepa, tiagabine, tiapride, propionic acid derivatives, ticlopidine, timolol, tinidazole, tioconazole, tioguanine, tioxolone, tiropramide, tizanidine, tolazoline, tolbutamide, tolcapone, tolnaftate, tolperisone, topotecan, torasemide, antioestrogens, tramadol, tramazoline, trandolapril, tranylcypromine, trapidil, trazodone, triamcinolone and triamcinolone derivatives, triamterene, trifluperidol, trifluridine, trimethoprim, trimipramine, tripelennamine, triprolidine, trifosfamide, tromantadine, trometamol, tropalpin, troxerutine, tulobuterol, tyramine, tyrothricin, urapidil, ursodeoxycholic acid, chenodeoxycholic acid, valaciclovir, valproic acid, vancomycin, vecuronium chloride, Viagra, venlafaxine, verapamil, vidarabine, vigabatrin, viloazine, vinblastine, vincamine, vincristine, vindesine, vinorelbine, vinpocetine, viquidil, warfarin, xantinol nicotinate, xipamide, zafirlukast, zalcitabine, zidovudine, zolmitriptan, zolpidem, zoplicone, zotipine and the like. See, e.g., US Patent No. 6,897,205; see also US Patent No. 6,838,528; US Patent No. 6,497,729.

The pharmaceutical agents may, if desired, also be used in the form of their pharmaceutically acceptable salts or derivatives (meaning salts which retain the biological effectiveness and properties of the compounds of this invention and which are not biologically or otherwise undesirable), and in the case of chiral active ingredients it is possible to employ both optically active isomers and racemates or mixtures of diastereoisomers. As well, the pharmaceutical agent may include a prodrug, a hydrate, an ester, a derivative or analogs of a compound or molecule.

A "pharmaceutically acceptable salt" may be prepared for any pharmaceutical agent having a functionality capable of forming a salt, for example an acid or base functionality. Pharmaceutically acceptable salts may be derived from organic or inorganic acids and bases. The term "pharmaceutically-acceptable salts" in these instances refers to the relatively non-toxic, inorganic and organic base addition salts of the pharmaceutical agents.

"Prodrugs" are derivative compounds derivatized by the addition of a group that endows greater solubility to the compound desired to be delivered. Once in the body, the prodrug is typically acted upon by an enzyme, e.g., an esterase, amidase, or phosphatase, to generate the active compound.

An "anti-cancer agent", "anti-tumor agent" or "chemotherapeutic agent" refers to any agent useful in the treatment of a neoplastic condition. There are many chemotherapeutic agents available in commercial use, in clinical evaluation and in pre-clinical development that are useful in the devices of the present invention for treatment of cancers.

In some embodiments provided herein the macrolide immunosuppressive drug is at least 97% crystalline. In some embodiments of the devices provided herein macrolide immunosuppressive drug is at least 98% crystalline. In some embodiments of the devices provided herein the macrolide immunosuppressive drug is at least 99% crystalline.

In some embodiments provided herein the pharmaceutical agent is at least 97% crystalline. In some embodiments of the devices provided herein pharmaceutical agent is at least 98% crystalline. In some embodiments of the devices provided herein the pharmaceutical agent is at least 99% crystalline.

In some embodiments, the coating exhibits an X-ray spectrum showing the presence of said pharmaceutical agent in crystalline form. In some embodiments, the coating exhibits a Raman spectrum showing the presence of said pharmaceutical agent in crystalline form. In some embodiments, the coating exhibits a Differential Scanning Calorimetry (DSC) curve showing the presence of said pharmaceutical agent in crystalline form. The device of Claims 36-38, wherein said coating exhibits Wide Angle X-ray Scattering (WAXS) spectrum showing the presence of said pharmaceutical agent in crystalline form. In some embodiments, the coating exhibits a wide angle radiation scattering spectrum showing the presence of said pharmaceutical agent in crystalline form. In some embodiments, the coating exhibits an Infra Red (IR) spectrum showing the presence of said pharmaceutical agent in crystalline form.

"Stability" as used herein in refers to the stability of the drug in a coating deposited on a substrate in its final product form (e.g., stability of the drug in a coated balloon). The term "stability" and/or "stable" in some embodiments is defined by 5% or less degradation of the drug in the final product form. The term stability in some embodiments is defined by 3% or less degradation of the drug in the final product form. The term stability in some embodiments is defined by 2% or less degradation of the drug in the final product form. The term stability in some embodiments is defined by 1% or less degradation of the drug in the final product form.

In some embodiments, the pharmaceutical agent is at least one of: 95% crystalline, 97% crystalline, and 99% crystalline following sterilization of the device. In some embodiments, the pharmaceutical agent crystallinity is stable wherein the crystallinity of the pharmaceutical agent following sterilization is compared to the crystallinity of the pharmaceutical agent at least one of: 1 week after sterilization, 2 weeks after sterilization, 4 weeks after sterilization, 1 month after sterilization, 2 months after sterilization, 45 days after sterilization, 60 days after sterilization, 90 days after sterilization, 3 months after sterilization, 4 months after sterilization, 6 months after sterilization, 9 months after sterilization, 12 months after sterilization, 18 months after sterilization, and 2 years after sterilization. In some embodiments, the pharmaceutical agent crystallinity is stable wherein the crystallinity of the pharmaceutical agent prior to sterilization is compared to the crystallinity of the pharmaceutical agent at least one of: 1 week after sterilization, 2 weeks after sterilization, 4 weeks after sterilization, 1 month after sterilization, 2 months after sterilization, 45 days after sterilization, 60 days after sterilization, 90 days after sterilization, 3 months after sterilization, 4 months after sterilization, 6 months after sterilization, 9 months after sterilization, 12 months after sterilization, 18 months after sterilization, and 2 years after sterilization. In such embodiments, different devices may be tested from the same manufacturing lot to determine stability of the pharmaceutical agent at the desired time points.

In some embodiments, the pharmaceutical agent crystallinity is stable at at least one of: 1 week after sterilization, 2 weeks after sterilization, 4 weeks after sterilization, 1 month after sterilization, 2 months after sterilization, 45 days after sterilization, 60 days after sterilization, 90 days after sterilization, 3 months after sterilization, 4 months after sterilization, 6 months after sterilization, 9 months after sterilization, 12 months after sterilization, 18 months after sterilization, and 2 years after sterilization.

In some embodiments, the pharmaceutical agent crystallinity on the device tested at a time point after sterilization does not differ more than 1%, 2%, 3%, 4%, and/or 5% from the crystallinity tested on a second device manufactured from the same lot of devices and the same lot of pharmaceutical agent at testing time point before sterilization (i.e. the crystallinity drops no more than from 99 to 95% crystalline, which is a 4 % difference in crystallinity; the crystallinity drops no more than from 99 to 96% crystalline, for example, which is a 3 % difference in crystallinity; the crystallinity drops no more than from 99 to 97% crystalline, for example, which is a 2 % difference in crystallinity; the crystallinity drops no more than from 99 to 98% crystalline, for example, which is a 1 % difference in crystallinity; in other examples, the starting crystallinity percentage is one of 100%, 98%, 96%, 97%, 96%, 95%, and/or anything in between).

In some embodiments, crystallinity of the pharmaceutical agent on the device tested at a time point after sterilization does not differ more than 1%, 2%, 3%, 4%, and/or 5% from the crystallinity of pharmaceutical from the same lot of pharmaceutical agent tested at testing time point before sterilization of the pharmaceutical agent.

In some embodiments, crystallinity of the pharmaceutical agent does not drop more than 1%, 2%, 3%, 4%, and/or 5% between two testing time points after sterilization neither of which time point being greater than 2 years after sterilization. In some embodiments, crystallinity of the pharmaceutical agent does not drop more than 1%, 2%, 3%, 4%, and/or 5% between two testing time points after sterilization neither of which time point being greater than 5 years after sterilization. In some embodiments, two time points comprise two of: 1 week after sterilization, 2 weeks after sterilization, 4 weeks after sterilization, 1 month after sterilization, 2 months after sterilization, 45 days after sterilization, 60 days after sterilization, 90 days after sterilization, 3 months after sterilization, 4 months after sterilization, 6 months after sterilization, 9 months after sterilization, 12 months after sterilization, 18 months after sterilization, 2 years after sterilization, 3 years after sterilization, 4 years after sterilization, and 5 years after sterilization.

In some embodiments, the pharmaceutical agent is at least one of: 95% crystalline, 97% crystalline, and 99% crystalline following sterilization of the device. In some embodiments, the pharmaceutical agent crystallinity is stable wherein the crystallinity of the pharmaceutical agent following sterilization is compared to the crystallinity of the pharmaceutical agent at least one of: 1 week after sterilization, 2 weeks after sterilization, 4 weeks after sterilization, 1 month after sterilization, 2 months after sterilization, 45 days after sterilization, 60 days after sterilization, 90 days after sterilization, 3 months after sterilization, 4 months after sterilization, 6 months after sterilization, 9 months after sterilization, 12 months after sterilization, 18 months after sterilization, and 2 years after sterilization. In some embodiments, the pharmaceutical agent crystallinity is stable wherein the crystallinity of the pharmaceutical agent prior to sterilization is compared to the crystallinity of the pharmaceutical agent at least one of: 1 week after sterilization, 2 weeks after sterilization, 4 weeks after sterilization, 1 month after sterilization, 2 months after sterilization, 45 days after sterilization, 60 days after sterilization, 90 days after sterilization, 3 months after sterilization, 4 months after sterilization, 6 months after sterilization, 9 months after sterilization, 12 months after sterilization, 18 months after sterilization, and 2 years after sterilization. In such embodiments, different devices may be tested from the same manufacturing lot to determine stability of the pharmaceutical agent at the desired time points.

In some embodiments, the pharmaceutical agent crystallinity is stable at at least one of: 1 week after sterilization, 2 weeks after sterilization, 4 weeks after sterilization, 1 month after sterilization, 2 months after sterilization, 45 days after sterilization, 60 days after sterilization, 90 days after sterilization, 3 months after sterilization, 4 months after sterilization, 6 months after sterilization, 9 months after sterilization, 12 months after sterilization, 18 months after sterilization, and 2 years after sterilization.

In some embodiments, the pharmaceutical agent crystallinity on the device tested at a time point after sterilization does not differ more than 1%, 2%, 3%, 4%, and/or 5% from the crystallinity tested on a second device manufactured from the same lot of devices and the same lot of pharmaceutical agent at testing time point before sterilization (i.e. the crystallinity drops no more than from 99 to 95% crystalline, which is a 4 % difference in crystallinity; the crystallinity drops no more than from 99 to 96% crystalline, for example, which is a 3 % difference in crystallinity; the crystallinity drops no more than from 99 to 97% crystalline, for example, which is a 2 % difference in crystallinity; the crystallinity drops no more than from 99 to 98% crystalline, for example, which is a 1 % difference in crystallinity; in other examples, the starting crystallinity percentage is one of 100%, 98%, 96%, 97%, and/or anything in between).

In some embodiments, crystallinity of the pharmaceutical agent on the device tested at a time point after sterilization does not differ more than 1%, 2%, 3%, 4%, and/or 5% from the crystallinity of pharmaceutical from the same lot of pharmaceutical agent tested at testing time point before sterilization of the pharmaceutical agent.

In some embodiments, crystallinity of the pharmaceutical agent does not drop more than 1%, 2%, 3%, 4%, and/or 5% between two testing time points after sterilization neither of which time point being greater than 2 years after sterilization. In some embodiments, crystallinity of the pharmaceutical agent does not drop more than 1%, 2%, 3%, 4%, and/or 5% between two testing time points after sterilization neither of which time point being greater than 5 years after sterilization. In some embodiments, two time points comprise two of: 1 week after sterilization, 2 weeks after sterilization, 4 weeks after sterilization, 1 month after sterilization, 2 months after sterilization, 45 days after sterilization, 60 days after sterilization, 90 days after sterilization, 3 months after sterilization, 4 months after sterilization, 6 months after sterilization, 9 months after sterilization, 12 months after sterilization, 18 months after sterilization, 2 years after sterilization, 3 years after sterilization, 4 years after sterilization, and 5 years after sterilization.

In some embodiments provided herein the macrolide immunosuppressive drug is at least 97% crystalline. In some embodiments of the or devices provided herein macrolide immunosuppressive drug is at least 98% crystalline. In some embodiments of the m devices provided herein the macrolide immunosuppressive drug is at least 99% crystalline.

In some embodiments provided herein the pharmaceutical agent is at least 97% crystalline. In some embodiments of the methods and/or devices provided herein pharmaceutical agent is at least 98% crystalline. In some embodiments of the methods and/or devices provided herein the pharmaceutical agent is at least 99% crystalline.

In some embodiments, the device has a pharmaceutical agent content of from about 0.5 µg/mm to about 20 µg/mm. In some embodiments, the device has a pharmaceutical agent content of from about 8 µg/mm to about 12 µg/mm. In some embodiments, the device has a pharmaceutical agent content of from about 5 µg to about 500 µg. In some embodiments, the device has a pharmaceutical agent content of from about 100 µg to about 160 µg. In some embodiments, the device has a pharmaceutical agent content of from about 100 µg to about 160 µg. The term "about" when used in the context of pharmaceutical agent content can mean variations of, for example, up to 5%, 10%, up to 25%, up to 50%, up to 75%, up to 100%, 0.5 µg/mm, 1 µg/mm, 5 µg/mm, 0.1 µg/mm to 5 µg/mm, 1 µg/mm to 5 µg/mm, and/or 0.1 µg/mm to 0.5 µg/mm, depending on the embodiment.

Content is expressed herein in units of µg/mm, however, this may simply be converted to µg/mm2 or another amount per area (e.g., µg/cm2).

"Therapeutically desirable morphology" as used herein refers to the gross form and structure of the pharmaceutical agent, once deposited on the substrate, so as to provide for optimal conditions of ex vivo storage, in vivo preservation and/or in vivo release. Such optimal conditions may include, but are not limited to increased shelf life (i.e., shelf stability), increased in vivo stability, good biocompatibility, good bioavailability or modified release rates. Typically, for the present invention, the desired morphology of a pharmaceutical agent would be crystalline although this may vary widely depending on many factors including, but not limited to, the nature of the pharmaceutical agent, the disease to be treated/prevented, the intended storage conditions for the substrate prior to use or the location within the body of any biomedical implant. At least 95%, 97%, 98%, 99%, 99.5%, and/or 100% of the pharmaceutical agent is in crystalline form

"Biological agent" or "biologic agent" or "biologic" as used herein, refers to a wide range of medicinal products, such as vaccines, blood and blood components, allergenics, somatic cells, gene therapies, tissues, and recombinant therapeutic proteins prepared by biologic processes (as distinguished from thoses prepared by chemistry means). Biologics can be composed of sugars, proteins, or nucleic acids, or complex combinations of these substances, or may be living entities such as cells and tissues. Biologics are isolated from a variety of natural sources-human, animal, or microorganism-and may be produced by biotechnology methods and other technologies. Gene-based and cellular biologics, for example, may be used to treat a variety of medical conditions. Biologics made by biologic processes involving recombinant DNA technology may include substances that are nearly identical to the body's own key signalling proteins (e.g. erythropoetin, growth hormone, biosynthetic human insulin and its analogues), monoclonal antibodies (custom designed, e.g.), and/or receptor constructs (e.g. fusion protiens). In some instances, a biologic (or biologic agent or biological agent) is one of the active substances produced from or extracted from a biological (living) system, and may require, in addition to physico-chemical testing, biological testing for full characterization.

"Biocompatible" as used herein, refers to any material that does not cause injury or death to the animal or induce an adverse reaction in an animal when placed in intimate contact with the animal's tissues. Adverse reactions include for example inflammation, infection, fibrotic tissue formation, cell death, or thrombosis. The terms "biocompatible " and "biocompatibility" when used herein are art-recognized and mean that the referent is neither itself toxic to a host (e.g., an animal or human), nor degrades (if it degrades) at a rate that produces byproducts (e.g., monomeric or oligomeric subunits or other byproducts) at toxic concentrations, causes inflammation or irritation, or induces an immune reaction in the host. It is not necessary that any subject composition have a purity of 100% to be deemed biocompatible. Hence, a subject composition may comprise 99%, 98%, 97%, 96%, 95%, 90% 85%, 80%, 75% or even less of biocompatible agents, e.g., including polymers and other materials and excipients described herein, and still be biocompatible. "Non-biocompatible" as used herein, refers to any material that may cause injury or death to the animal or induce an adverse reaction in the animal when placed in intimate contact with the animal's tissues. Such adverse reactions are as noted above, for example.

"Polymer" as used herein, refers to a series of repeating monomeric units that have been cross-linked or polymerized. Any suitable polymer can be used to carry out the present invention. It is possible that the polymers of the invention may also comprise two, three, four or more different polymers. In some embodiments, of the invention only one polymer is used. In some preferred embodiments a combination of two polymers are used. Combinations of polymers can be in varying ratios, to provide coatings with differing properties. Those of skill in the art of polymer chemistry will be familiar with the different properties of polymeric compounds.

Polymers useful in the devices and methods of the present invention include, for example, stable polymers, biostable polymers, durable polymers, inert polymers, organic polymers, organic-inorganic copolymers, inorganic polymers, bioabsorbable, bioresorbable, resorbable, degradable, and biodegradable polymers. These categories of polymers may, in some cases, be synonymous, and is some cases may also and/or alternatively overlap. Those of skill in the art of polymer chemistry will be familiar with the different properties of polymeric compounds.

The coating comprises a polymer. In some embodiments, the active agent comprises a polymer. In some embodiments, the polymer comprises at least one polylactide-co-glycolide (PLGA).

Examples of polymers that may be used in the present invention include, but are not limited to polycarboxylic acids, cellulosic polymers, proteins, polypeptides, polyvinylpyrrolidone, maleic anhydride polymers, polyamides, polyvinyl alcohols, polyethylene oxides, glycosaminoglycans, polysaccharides, polyesters, aliphatic polyesters, polyurethanes, polystyrenes, copolymers, silicones, silicone containing polymers, polyalkyl siloxanes, polyorthoesters, polyanhydrides, copolymers of vinyl monomers, polycarbonates, polyethylenes, polypropytenes, polylactic acids, polylactides, polyglycolic acids, polyglycolides, polylactide-co-glycolides, polycaprolactones, poly(e-caprolactone)s, polyhydroxybutyrate valerates, polyacrylamides, polyethers, polyurethane dispersions, polyacrylates, acrylic latex dispersions, polyacrylic acid, polyalkyl methacrylates, polyalkylene-co-vinyl acetates, polyalkylenes, aliphatic polycarbonates polyhydroxyalkanoates, polytetrahalooalkylenes, poly(phosphasones), polytetrahalooalkylenes, poly(phosphasones), and mixtures, combinations, and copolymers thereof.

The polymers of the present invention may be natural or synthetic in origin, including gelatin, chitosan, dextrin, cyclodextrin, Poly(urethanes), Poly(siloxanes) or silicones, Poly(acrylates) such as [rho]oly(methyl methacrylate), poly(butyl methacrylate), and Poly(2-hydroxy ethyl methacrylate), Poly( vinyl alcohol) Poly(olefins) such as poly(ethylene), [rho]oly(isoprene), halogenated polymers such as Poly(tetrafluoroethylene) - and derivatives and copolymers such as those commonly sold as Teflon(R) products, Poly(vinylidine fluoride), Poly(vinyl acetate), Poly(vinyl pyrrolidone), Poly(acrylic acid), Polyacrylamide, Poly(ethylene- co-vinyl acetate), Poly(ethylene glycol), Poly(propylene glycol), Poly(methacrylic acid); etc.

Examples of polymers that may be used in the present invention include, but are not limited to polycarboxylic acids, cellulosic polymers, proteins, polypeptides, polyvinylpyrrolidone, maleic anhydride polymers, polyamides, polyvinyl alcohols, polyethylene oxides, glycosaminoglycans, polysaccharides, polyesters, aliphatic polyesters, polyurethanes, polystyrenes, copolymers, silicones, silicone containing polymers, polyalkyl siloxanes, polyorthoesters, polyanhydrides, copolymers of vinyl monomers, polycarbonates, polyethylenes, polypropytenes, polylactic acids, polylactides, polyglycolic acids, polyglycolides, polylactide-co-glycolides, polycaprolactones, poly(e-caprolactone)s, polyhydroxybutyrate valerates, polyacrylamides, polyethers, polyurethane dispersions, polyacrylates, acrylic latex dispersions, polyacrylic acid, polyalkyl methacrylates, polyalkylene-co-vinyl acetates, polyalkylenes, aliphatic polycarbonates polyhydroxyalkanoates, polytetrahalooalkylenes, poly(phosphasones), polytetrahalooalkylenes, poly(phosphasones), and mixtures, combinations, and copolymers thereof.

The polymers of the present invention may be natural or synthetic in origin, including gelatin, chitosan, dextrin, cyclodextrin, Poly(urethanes), Poly(siloxanes) or silicones, Poly(acrylates) such as [rho]oly(methyl methacrylate), poly(butyl methacrylate), and Poly(2-hydroxy ethyl methacrylate), Poly( vinyl alcohol) Poly(olefins) such as poly(ethylene), [rho]oly(isoprene), halogenated polymers such as Poly(tetrafluoroethylene) - and derivatives and copolymers such as those commonly sold as Teflon(R) products, Poly(vinylidine fluoride), Poly(vinyl acetate), Poly(vinyl pyrrolidone), Poly(acrylic acid), Polyacrylamide, Poly(ethylene-co-vinyl acetate), Poly(ethylene glycol), Poly(propylene glycol), Poly(methacrylic acid); etc.

Suitable polymers also include absorbable and/or resorbable polymers including the following, combinations, copolymers and derivatives of the following: Polylactides (PLA), Polyglycolides (PGA), PolyLactide-co-glycolides (PLGA), Polyanhydrides, Polyorthoesters, Poly(N-(2- hydroxypropyl) methacrylamide), Poly(l-aspartamide), including the derivatives DLPLA - poly(dl-lactide); LPLA - poly(l-lactide); PDO - poly(dioxanone); PGA-TMC - poly(glycolide-co-trimethylene carbonate); PGA-LPLA - poly(l-lactide-co-glycolide); PGA-DLPLA - poly(dl-lactide-co-glycolide); LPLA-DLPLA - poly(l-lactide-co-dl-lactide); and PDO-PGA-TMC - poly(glycolide-co-trimethylene carbonate-co-dioxanone), and combinations thereof.

"Copolymer" as used herein refers to a polymer being composed of two or more different monomers. A copolymer may also and/or alternatively refer to random, block, graft, copolymers known to those of skill in the art.

The terms "bioabsorbable," "biodegradable," "bioerodible," "bioresorbable," and "resorbable" are art-recognized synonyms. These terms are used herein interchangeably. Bioabsorbable polymers typically differ from non-bioabsorbable polymers in that the former may be absorbed (e.g.; degraded) during use. In certain embodiments, such use involves in vivo use, such as in vivo therapy, and in other certain embodiments, such use involves in vitro use. In general, degradation attributable to biodegradability involves the degradation of a bioabsorbable polymer into its component subunits, or digestion, e.g., by a biochemical process, of the polymer into smaller, non-polymeric subunits. In certain embodiments, biodegradation may occur by enzymatic mediation, degradation in the presence of water (hydrolysis) and/or other chemical species in the body, or both. The bioabsorbability of a polymer may be shown in-vitro as described herein or by methods known to one of skill in the art. An in-vitro test for bioabsorbability of a polymer does not require living cells or other biologic materials to show bioabsorption properties (e.g. degradation, digestion). Thus, resorbtion, resorption, absorption, absorbtion, erosion may also be used synonymously with the terms "bioabsorbable," "biodegradable," "bioerodible," and "bioresorbable." Mechanisms of degradation of a bioaborbable polymer may include, but are not limited to, bulk degradation, surface erosion, and combinations thereof.

As used herein, the term "biodegradation" encompasses both general types of biodegradation. The degradation rate of a biodegradable polymer often depends in part on a variety of factors, including the chemical identity of the linkage responsible for any degradation, the molecular weight, crystallinity, biostability, and degree of cross-linking of such polymer, the physical characteristics (e.g., shape and size) of the implant, and the mode and location of administration. For example, the greater the molecular weight, the higher the degree of crystallinity, and/or the greater the biostability, the biodegradation of any bioabsorbable polymer is usually slower.

"Degradation" as used herein refers to the conversion or reduction of a chemical compound to one less complex, e.g., by splitting off one or more groups of atoms. Degradation of the coating may reduce the coating's cohesive and adhesive binding to the device, thereby facilitating transfer of the coating to the intervention site.

As used herein, the term "durable polymer" refers to a polymer that is not bioabsorbable (and/or is not bioerodable, and/or is not biodegradable, and/or is not bioresorbable) and is, thus biostable. In some embodiments, the device comprises a durable polymer. The polymer may include a cross-linked durable polymer. Example biocompatible durable polymers include, but are not limited to: polyester, aliphatic polyester, polyanhydride, polyethylene, polyorthoester, polyphosphazene, polyurethane, polycarbonate urethane, aliphatic polycarbonate, silicone, a silicone containing polymer, polyolefin, polyamide, polycaprolactam, polyamide, polyvinyl alcohol, acrylic polymer, acrylate, polystyrene, epoxy, polyethers, celluiosics, expanded polytetrafluoroethylene, phosphorylcholine, polyethyleneyerphthalate, polymethylmethavrylate, poly(ethylmethacrylate/n-butylmethacrylate), parylene C, polyethylene-co-vinyl acetate, polyalkyl methacrylates, polyalkylene-co-vinyl acetate, polyalkylene, polyalkyl siloxanes, polyhydroxyalkanoate, polyfluoroalkoxyphasphazine, poly(styrene-b-isobutylene-b-styrene), poly-butyl methacrylate, polybyta-diene, and blends, combinations, homopolymers, condensation polymers, alternating, block, dendritic, crosslinked, and copolymers thereof. The polymer may include a thermoset material. The polymer may provide strength for the coated implantable medical device. The polymer may provide durability for the coated implantable medical device. The coatings provided herein provide substantial protection from these by establishing a multi-layer coating which can be bioabsorbable or durable or a combination thereof, and which can both deliver active agents and provide elasticity and radial strength for the vessel in which it is delivered.

In some embodiments, the polymer comprises is at least one of: a fluoropolymer, PVDF-HFP comprising vinylidene fluoride and hexafluoropropylene monomers, PC (phosphorylcholine), Polysulfone, polystyrene-b-isobutylene-b-styrene, PVP (polyvinylpyrrolidone), alkyl methacrylate, vinyl acetate, hydroxyalkyl methacrylate, and alkyl acrylate. In some embodiments, the alkyl methacrylate comprises at least one of methyl methacrylate, ethyl methacrylate, propyl methacrylate, butyl methacrylate, hexyl methacrylate, octyl methacrylate, dodecyl methacrylate, and lauryl methacrylate. In some embodiments, the alkyl acrylate comprises at least one of methyl acrylate, ethyl acrylate, propyl acrylate, butyl acrylate, hexyl acrylate, octyl acrylate, dodecyl acrylates, and lauryl acrylate.

In some embodiments, the coating comprises a plurality of polymers. In some embodiments, the polymers comprise hydrophilic, hydrophobic, and amphiphilic monomers and combinations thereof. In one embodiment, the polymer comprises at least one of a homopolymer, a copolymer and a terpolymer. The homopolymer may comprise a hydrophilic polymer constructed of a hydrophilic monomer selected from the group consisting of poly(vinylpyrrolidone) and poly(hydroxylalkyl methacrylate). The copolymer may comprise comprises a polymer constructed of hydrophilic monomers selected from the group consisting of vinyl acetate, vinylpyrrolidone and hydroxyalkyl methacrylate and hydrophobic monomers selected from the group consisting of alkyl methacrylates including methyl, ethyl, propyl, butyl, hexyl, octyl, dodecyl, and lauryl methacrylate and alkyl acrylates including methyl, ethyl, propyl, butyl, hexyl, octyl, dodecyl, and lauryl acrylate. The terpolymer may comprise a polymer constructed of hydrophilic monomers selected from the group consisting of vinyl acetate and poly(vinylpyrrolidone), and hydrophobic monomers selected from the group consisting of alkyl methacrylates including methyl, ethyl, propyl, butyl, hexyl, octyl, dodecyl, and lauryl methacrylate and alkyl acrylates including methyl, ethyl, propyl, butyl, hexyl, octyl, dodecyl, and lauryl acrylate.

In one embodiment, the polymer comprises three polymers: a terpolymer, a copolymer and a homopolymer. In one such embodiment the terpolymer has the lowest glass transition temperature (Tg), the copolymer has an intermediate Tg and the homopolymer has the highest Tg. In one embodiment the ratio of terpolymer to copolymer to homopolymer is about 40:40:20 to about 88:10:2. In another embodiment, the ratio is about 50:35:15 to about 75:20:5. In one embodiment the ratio is approximately 63:27:10. In such embodiment, the terpolymer has a Tg in the range of about 5° C. to about 25° C., a copolymer has a Tg in the range of about 25° C. to about 40° C. and a homopolymer has a Tg in the range of about 170° C. to about 180° C. In some embodiments, the polymer system comprises a terpolymer (C19) comprising the monomer subunits n-hexyl methacrylate, N-vinylpyrrolidone and vinyl acetate having a Tg of about 10° C. to about 20° C., a copolymer (C10) comprising the monomer subunits n-butyl methacrylacte and vinyl acetate having a Tg of about 30° C to about 35° C. and a homopolymer comprising polyvinylpyrrolidone having a Tg of about 174° C. As used herein, the term "about" when used in context of ratios of polymers can mean variability of 5%, 10%, 25%, 50%, 10%-50%, 1% to 50%, and/or 10% to 25%, depending on the embodiment. As used herein, the term "about" when used in context of Tg of polymers can mean variability of 1°C, 5°C, 10°C, and/or 25°C, depending on the embodiment.

Some embodiments comprise about 63% of C19, about 27% of C10 and about 10% of polyvinyl pyrrolidone (PVP). The C10 polymer is comprised of hydrophobic n-butyl methacrylate to provide adequate hydrophobicity to accommodate the active agent and a small amount of vinyl acetate. The C19 polymer is soft relative to the C10 polymer and is synthesized from a mixture of hydrophobic n-hexyl methacrylate and hydrophilic N-vinyl pyrrolidone and vinyl acetate monomers to provide enhanced biocompatibility. Polyvinyl pyrrolidone (PVP) is a medical grade hydrophilic polymer. As used herein, the term "about" when used in context of percentages of polymers can mean variability (in absolute percent, not as a percent of a percent) of 5%, 10%, 25%, 50%, 10%-50%, 1% to 50%, and/or 10% to 25%, depending on the embodiment.

In some embodiments, the polymer is not a polymer selected from: PBMA (poly n-butyl methacrylate), Parylene C, and polyethylene-co-vinyl acetate.

The coating comprises PLGA (poly(lactide-co-glycolide) as a bioabsorbable polymer. In some embodiments, the coating further comprises at least one of: Polylactides (PLA); Polyanhydrides; Polyorthoesters; Poly(N-(2- hydroxypropyl) methacrylamide); DLPLA - poly(dl-lactide); LPLA - poly(l-lactide); PGA - polyglycolide; PDO - poly(dioxanone); PGA-TMC - poly(glycolide-co-trimethylene carbonate); PGA-LPLA - poly(l-lactide-co-glycolide); PGA-DLPLA - poly(dl-lactide-co-glycolide); LPLA-DLPLA - poly(l-lactide-co-dl-lactide); DLPLA poly(dl-lactide), PCL poly(e-caprolactone) PDO, poly(dioxolane) PGA-TMC, 85/15 DLPLG p(dl-lactide-co-glycolide), 75/25 DLPL, 65/35 DLPLG, 50/50 DLPLG, TMC poly(trimethylcarbonate), p(CPP:SA) poly(1,3-bis-p-(carboxyphenoxy)propane-co-sebacic acid).and PDO-PGA-TMC - poly(glycolide-co-trimethylene carbonate-co-dioxanone), and combinations, copolymers, and derivatives thereof. In some embodiments, the bioabsorbable polymer comprises between 1% and 95% glycolic acid content PLGA-based polymer.

In some embodiments of the devices provided herein, the polymer comprises at least one of polycarboxylic acids, cellulosic polymers, proteins, polypeptides, polyvinylpyrrolidone, maleic anhydride polymers, polyamides, polyvinyl alcohols, polyethylene oxides, glycosaminoglycans, polysaccharides, polyesters, aliphatic polyesters, polyurethanes, polystyrenes, copolymers, silicones, silicone containing polymers, polyalkyl siloxanes, polyorthoesters, polyanhydrides, copolymers of vinyl monomers, polycarbonates, polyethylenes, polypropytenes, polylactic acids, polylactides, polyglycolic acids, polyglycolides, polylactide-co-glycolides, polycaprolactones, poly(e-caprolactone)s, polyhydroxybutyrate valerates, polyacrylamides, polyethers, polyurethane dispersions, polyacrylates, acrylic latex dispersions, polyacrylic acid, polyalkyl methacrylates, polyalkylene-co-vinyl acetates, polyalkylenes, aliphatic polycarbonates polyhydroxyalkanoates, polytetrahalooalkylenes, poly(phosphasones), polytetrahalooalkylenes, poly(phosphasones), and mixtures, combinations, and copolymers thereof. The polymers of the present invention used in addition to PLGA may be natural or synthetic in origin, including gelatin, chitosan, dextrin, cyclodextrin, Poly(urethanes), Poly(siloxanes) or silicones, Poly(acrylates) such as [rho]oly(methyl methacrylate), poly(butyl methacrylate), and Poly(2-hydroxy ethyl methacrylate), Poly( vinyl alcohol) Poly(olefins) such as poly(ethylene), [rho]oly(isoprene), halogenated polymers such as Poly(tetrafluoroethylene) - and derivatives and copolymers such as those commonly sold as Teflon(R) products, Poly(vinylidine fluoride), Poly(vinyl acetate), Poly(vinyl pyrrolidone), Poly(acrylic acid), Polyacrylamide, Poly(ethylene-co-vinyl acetate), Poly(ethylene glycol), Poly(propylene glycol), Poly(methacrylic acid); etc. Suitable polymers also include absorbable and/or resorbable polymers including the following, combinations, copolymers and derivatives of the following: Polylactides (PLA), Polyglycolides (PGA), Polyanhydrides, Polyorthoesters, Poly(N-(2- hydroxypropyl) methacrylamide), Poly(l-aspartamide), including the derivatives DLPLA - poly(dl-lactide); LPLA - poly(l-lactide); PDO - poly(dioxanone); PGA-TMC - poly(glycolide-co-trimethylene carbonate); PGA-LPLA - poly(l-lactide-co-glycolide); PGA-DLPLA - poly(dl-lactide-co-glycolide); LPLA-DLPLA - poly(l-lactide-co-dl-lactide); and PDO-PGA-TMC - poly(glycolide-co-trimethylene carbonate-co-dioxanone), and combinations thereof.

In some embodiments of the methods and/or devices provided herein, the polymer has a dry modulus between 3,000 and 12,000 KPa. In some embodiments, the polymer is capable of becoming soft after implantation. In some embodiments, the polymer is capable of becoming soft after implantation by hydration, degradation or by a combination of hydration and degradation. In some embodiments, the polymer is adapted to transfer, free, and/or dissociate from the substrate when at the intervention site due to hydrolysis of the polymer.

In some embodiments, in vitro elution is carried out in a 1:1 spectroscopic grade ethanol/phosphate buffer saline at pH 7.4 and 37°C; wherein the amount of active agent released is determined by measuring UV absorption.

In some embodiments of the devices provided herein, the bioabsorbable polymer is capable of resorbtion in at least one of: about 1 day, about 3 days, about 5 days, about 7 days, about 14 days, about 3 weeks, about 4 weeks, about 45 days, about 60 days, about 90 days, about 180 days, about 6 months, about 9 months, about 1 year, about 1 to about 2 days, about 1 to about 5 days, about 1 to about 2 weeks, about 2 to about 4 weeks, about 45 to about 60 days, about 45 to about 90 days, about 30 to about 90 days, about 60 to about 90 days, about 90 to about 180 days, about 60 to about 180 days, about 180 to about 365 days, about 6 months to about 9 months, about 9 months to about 12 months, about 9 months to about 15 months, and about 1 year to about 2 years. The term "about" when used in the context of resorbtion of polymer can mean variations of, for example, up to 10%, up to 25%, up to 50%, up to 12 hrs, up to 6 hrs, up to 3 hrs, up to 2 hrs, 2-6 hrs, 1-12 hrs, 1 day, 2 days, 5 days, 7 days, and/or 1-5 days, depending on the embodiment.

In some embodiments, the in vivo pharmaceutical agent tissue uptake in arteries is determined according to LC-MS methods as noted herein, and/or as known to one of ordinary skill in the art depending on the particular pharmaceutical agent used.

In some embodiments of the devices provided herein, wherein the coating is formed on the substrate by a process comprising at least one of: depositing a polymer by an e-RESS, an e-SEDS, or an e-DPC process. Generally speaking when an "e-" is placed in front of an acronym herein (e-RESS), it refers to an electrostatic capture process incorporated into, or in addition to the process described by the acronym. "Electrostatically charged" or "electrical potential" or "electrostatic capture" or "e-" as used herein refers to the collection of the spray-produced particles upon a substrate that has a different electrostatic potential than the sprayed particles. Thus, the substrate is at an attractive electronic potential with respect to the particles exiting, which results in the capture of the particles upon the substrate. i.e. the substrate and particles are oppositely charged, and the particles transport through the gaseous medium of the capture vessel onto the surface of the substrate is enhanced via electrostatic attraction. This may be achieved by charging the particles and grounding the substrate or conversely charging the substrate and grounding the particles, by charging the particles at one potential (e.g. negative charge) and charging the substrate at an opposited potential (e.g. positive charge), or by some other process, which would be easily envisaged by one of skill in the art of electrostatic capture.

"Electrostatic Rapid Expansion of Supercritical Solutions" or "e-RESS" or "eRESS" as used herein refers to Electrostatic Capture as described herein combined with Rapid Expansion of Supercritical Solutions as described herein. In some embodiments, Electrostatic Rapid Expansion of Supercritical Solutions refers to Electrostatic capture as described in the art, e.g., in U.S. Pat. No. 6,756,084, "Electrostatic deposition of particles generated from rapid expansion of supercritical fluid solutions,".

"Solution Enhanced Dispersion of Supercritical Solutions" or "SEDS" as used herein involves a spray process for the generation of polymer particles, which are formed when a compressed fluid (e.g. supercritical fluid, preferably supercritical CO2) is used as a diluent to a vehicle in which a polymer is dissolved (one that can dissolve both the polymer and the compressed fluid). The mixing of the compressed fluid diluent with the polymer-containing solution may be achieved by encounter of a first stream containing the polymer solution and a second stream containing the diluent compressed fluid, for example, within one spray nozzle or by the use of multiple spray nozzles. The solvent in the polymer solution may be one compound or a mixture of two or more ingredients and may be or comprise an alcohol (including diols, triols, etc.), ether, amine, ketone, carbonate, or alkanes, or hydrocarbon (aliphatic or aromatic) or may be a mixture of compounds, such as mixtures of alkanes, or mixtures of one or more alkanes in combination with additional compounds such as one or more alcohols, (e.g., from 0 or 0.1 to 5% of a Ci to Ci5 alcohol, including diols, triols, etc.). See for example U.S. Pat. No. 6,669,785. The solvent may optionally contain a surfactant, as also described in, e.g., U.S. Pat. No. 6,669,785.

In one embodiment of the SEDS process, a first stream of fluid comprising a polymer dissolved in a common solvent is co-sprayed with a second stream of compressed fluid. Polymer particles are produced as the second stream acts as a diluent that weakens the solvent in the polymer solution of the first stream. The now combined streams of fluid, along with the polymer particles, flow out of the nozzle assembly into a collection vessel. Control of particle size, particle size distribution, and morphology is achieved by tailoring the following process variables: temperature, pressure, solvent composition of the first stream, flow-rate of the first stream, flow-rate of the second stream, composition of the second stream (where soluble additives may be added to the compressed gas), and conditions of the capture vessel. Typically the capture vessel contains a fluid phase that is at least five to ten times (5-1Ox) atmospheric pressure.

"Electrostatic Dry Powder Coating" or "e-DPC" or "eDPC" as used herein refers to Electrostatic Capture as described herein combined with Dry Powder Coating. e-DPC deposits material (including, for example, polymer or impermeable dispersed solid) on the device or other substrate as dry powder, using electrostatic capture to attract the powder particles to the substrate. Dry powder spraying ("Dry Powder Coating" or "DPC") is well known in the art, and dry powder spraying coupled with electrostatic capture has been described, for example in U.S. Pat. Nos: 5,470,603, 6,319,541, and 6,372,246. Methods for depositing coatings are described, e.g., in WO 2008/148013, "Polymer Films for Medical Device Coating,".

"Compressed fluid" as used herein refers to a fluid of appreciable density (e.g., >0.2 g/cc) that is a gas at standard temperature and pressure. "Supercritical fluid," "near-critical fluid," "near-supercritical fluid," "critical fluid," "densified fluid," or "densified gas," as used herein refers to a compressed fluid under conditions wherein the temperature is at least 80% of the critical temperature of the fluid and the pressure is at least 50% of the critical pressure of the fluid, and/or a density of +50% of the critical density of the fluid.

Examples of substances that demonstrate supercritical or near critical behavior suitable for the present invention include, but are not limited to carbon dioxide, isobutylene, ammonia, water, methanol, ethanol, ethane, propane, butane, pentane, dimethyl ether, xenon, sulfur hexafluoride, halogenated and partially halogenated materials such as chlorofluorocarbons, hydrochlorofluorocarbons, hydrofluorocarbons, perfluorocarbons (such as perfluoromethane and perfuoropropane, chloroform, trichloro-fluoromethane, dichloro-difluoromethane, dichlorotetrafluoroethane) and mixtures thereof. Preferably, the supercritical fluid is hexafluoropropane (FC-236EA), or 1,1,1,2,3,3-hexafluoropropane. Preferably, the supercritical fluid is hexafluoropropane (FC-236EA), or 1,1,1,2,3,3-hexafluoropropane for use in PLGA polymer coatings.

"Sintering" as used herein refers to the process by which parts of the polymer or the entire polymer becomes continuous (e.g., formation of a continuous polymer film). As discussed herein, the sintering process is controlled to produce a fully conformal continuous polymer (complete sintering) or to produce regions or domains of continuous coating while producing voids (discontinuities) in the polymer. As well, the sintering process is controlled such that some phase separation is obtained or maintained between polymer different polymers (e.g., polymers A and B) and/or to produce phase separation between discrete polymer particles. Through the sintering process, the adhesions properties of the coating are improved to reduce flaking of detachment of the coating from the substrate during manipulation in use. As described herein, in some embodiments, the sintering process is controlled to provide incomplete sintering of the polymer. In embodiments involving incomplete sintering, a polymer is formed with continuous domains, and voids, gaps, cavities, pores, channels or, interstices that provide space for sequestering a therapeutic agent which is released under controlled conditions. Depending on the nature of the polymer, the size of polymer particles and/or other polymer properties, a compressed gas, a densified gas, a near critical fluid or a super-critical fluid may be employed. In one example, carbon dioxide is used to treat a substrate that has been coated with a polymer and a drug, using dry powder and RESS electrostatic coating processes. In another example, isobutylene is employed in the sintering process. In other examples a mixture of carbon dioxide and isobutylene is employed. In another example, 1,1,2,3,3-hexafluoropropane is employed in the sintering process.

When an amorphous material is heated to a temperature above its glass transition temperature, or when a crystalline material is heated to a temperature above a phase transition temperature, the molecules comprising the material are more mobile, which in turn means that they are more active and thus more prone to reactions such as oxidation. However, when an amorphous material is maintained at a temperature below its glass transition temperature, its molecules are substantially immobilized and thus less prone to reactions. Likewise, when a crystalline material is maintained at a temperature below its phase transition temperature, its molecules are substantially immobilized and thus less prone to reactions. Accordingly, processing drug components at mild conditions, such as the deposition and sintering conditions described herein, minimizes cross-reactions and degradation of the drug component. One type of reaction that is minimized by the processes of the invention relates to the ability to avoid conventional solvents which in turn minimizes -oxidation of drug, whether in amorphous, semi-crystalline, or crystalline form, by reducing exposure thereof to free radicals, residual solvents, protic materials, polar-protic materials, oxidation initiators, and autoxidation initiators.

"Dipping Process" and "Spraying Process" as used herein refer to methods of coating substrates that have been described at length in the art. These processes can be used for coating medical devices with pharmaceutical agents. Spray coating, described in, e.g., U.S. Pat. No. 7,419,696, "Medical devices for delivering a therapeutic agent and method of preparation" and elsewhere herein, can involve spraying or airbrushing a thin layer of solubilized coating or dry powder coating onto a substrate. Dip coating involves, e.g., dipping a substrate in a liquid, and then removing and drying it. Dip coating is described in, e.g., U.S. Pat. No. 5,837,313 "Drug release stent coating process,".

"Intervention site" as used herein refers to the location in the body where the coating is intended to be delivered (by transfer from, freeing from, and/or dissociating from the substrate). The intervention site can be any substance in the medium surrounding the device, e.g., tissue, cartilage, a body fluid, etc. The intervention site can be the same as the treatment site, i.e., the substance to which the coating is delivered is the same tissue that requires treatment. Alternatively, the intervention site can be separate from the treatment site, requiring subsequent diffusion or transport of the pharmaceutical or other agent away from the intervention site.

In some embodiments of the devices provided herein, the coating is delivered to an intervention site. In some embodiments of the devices provided herein, the nanoparticle is delivered to an intervention site. The intervention site may be in or on the body of a subject. In some embodiments, the intervention site is a vascular wall. In some embodiments, the intervention site is a non-vascular lumen wall. In some embodiments, the intervention site is a vascular cavity wall.

In some embodiments of the devices provided herein, the intervention site is a wall of a body cavity. In some embodiments, the body cavity is the result of a lumpectomy. In some embodiments, the intervention site is a cannulized site within a subject.

In some embodiments of the devices provided herein, the intervention site is a sinus wall. In some embodiments, the intervention site is a sinus cavity wall. In some embodiments, the active agent comprises a corticosteroid.

"Substrate" as used herein, refers to any surface upon which it is desirable to deposit a coating. Biomedical implants are of particular interest for the present invention; however the present invention is not intended to be restricted to this class of substrates. Those of skill in the art will appreciate alternate substrates that could benefit from the coating process described herein, such as pharmaceutical tablet cores, as part of an assay apparatus or as components in a diagnostic kit (e.g. a test strip). Examples of substrates that can be coated using the methods of the invention include surgery devices or medical devices, e.g., a catheter, a balloon, a cutting balloon, a wire guide, a cannula, tooling, an orthopedic device, a structural implant, stent, stent-graft, graft, vena cava filter, a heart valve, cerebrospinal fluid shunts, pacemaker electrodes, axius coronary shunts, endocardial leads, an artificial heart, and the like.

"Biomedical implant" or "biological implant" or "medical device" as used herein refers to any implant for insertion into the body of a human or animal subject, including but not limited to stents (e.g., coronary stents, vascular stents including peripheral stents and graft stents, urinary tract stents, urethral/prostatic stents, rectal stent, oesophageal stent, biliary stent, pancreatic stent), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, vascular supports, etc.

The implants may be formed from any suitable material, including but not limited to polymers (including stable or inert polymers, organic polymers, organic-inorganic copolymers, inorganic polymers, and biodegradable polymers), metals, metal alloys, inorganic materials such as silicon, and composites thereof, including layered structures with a core of one material and one or more coatings of a different material. Substrates made of a conducting material facilitate electrostatic capture. However, the invention contemplates the use of electrostatic capture, as described herein, in conjunction with substrate having low conductivity or which are non-conductive. To enhance electrostatic capture when a non-conductive substrate is employed, the substrate is processed for example while maintaining a strong electrical field in the vicinity of the substrate. In some embodiments, however, no electrostatic capture is employed in applying a coating to the substrate. In some embodiments of the devices provided herein, the substrate is not charged in the coating process. In some embodiments of the devices provided herein, an electrical potential is not prepared between the substrate and the coating apparatus.

Subjects into which biomedical implants of the invention may be applied or inserted include both human subjects (including male and female subjects and infant, juvenile, adolescent, adult and geriatric subjects) as well as animal subjects (including but not limited to pig, rabbit, mouse, dog, cat, horse, monkey, etc.) for veterinary purposes and/or medical research.

As used herein, a biological implant may include a medical device that is not permanantly implanted. A biological implant in some embodiments may comprise a device which is used in a subject on a transient basis. For non-limiting example, the biomedical implant may be a balloon, which is used transiently to dilate a lumen and thereafter may be deflated and/or removed from the subject during the medical procedure or thereafter. In some embodiments, the biological implant may be temporarily implanted for a limited time, such as during a portion of a medical procedure, or for only a limited time (some time less than permanantly implanted), or may be transiently implanted and/or momentarily placed in the subject. In some embodiments, the biological implant is not implanted at all, rather it is merely inserted into a subject during a medical procedure, and subsequently removed from the subject prior to or at the time the medical procedure is completed. In some embodiments, the biological implant is not permenantly implanted since it completely resorbs into the subject (i.e. is completely resorbed by the subject). In a preferred embodiment the biomedical implant is an expandable balloon that can be expanded within a lumen (naturally occuring or non-naturally occurring) having a coating thereon that is freed (at least in part) from the balloon and left behind in the lumen when the balloon is removed from the lumen.

"Balloon" as used herein refers to a flexible sac that can be inflated within a natural or non-natural body lumen or cavity, or used to prepare a cavity, or used to enlarge an existing cavity. The balloon can be used transiently to dilate a lumen or cavity and thereafter may be deflated and/or removed from the subject during the medical procedure or thereafter. In embodiments, the balloon can be expanded within the body and has a coating thereon that is freed (at least in part) from the balloon and left behind in the lumen or cavity when the balloon is removed. A coating can be applied to a balloon either after the balloon has been compacted for insertion, resulting in a coating that partially covers the surface of the balloon, or it can be applied prior to or during compaction. In embodiments, a coating is applied to the balloon both prior to and after compaction of the balloon. In embodiments, the balloon is compacted by, e.g., crimping or folding. Methods of compacting balloons have been described, e.g., in U.S. Pat. No. 7,308,748, "Method for compressing an intraluminal device," and U.S. Pat. No. 7,152,452, "Assembly for crimping an intraluminal device and method of use," relating to uniformly crimping a balloon onto a catheter or other intraluminal device, and 5,350,361 "Tri-fold balloon for dilatation catheter and related method," relating to balloon folding methods and devices. In some embodiments the balloon is delivered to the intervention site by a delivery device. In some embodiments, the delivery device comprises catheter. In some embodiments, the balloon is an angioplasty balloon. Balloons can be delivered, removed, and visualized during delivery and removal by methods known in the art, e.g., for inserting angioplasty balloons, stents, and other medical devices. Methods for visualizing a treatment area and planning instrument insertion are described, e.g., in U.S. Pat. No. 7,171,255, "Virtual reality 3D visualization for surgical procedures" and U.S. Pat. No. 6,610,013, "3D ultrasound-guided intraoperative prostate brachytherapy,".

"Compliant balloon" as used herein refers to a balloon which conforms to the intervention site relatively more than a semi-compliant balloon and still more so than a non-compliant balloon. Compliant balloons expand and stretch with increasing pressure within the balloon, and are made from such materials as polyethylene or polyolefin copolymers. There is in the art a general classification of balloons based on their expandability or "compliance" relative to each other, as described e.g., in U.S. Pat. No. 5,556,383, "Block copolymer elastomer catheter balloons." Generally, "non-compliant" balloons are the least elastic, increasing in diameter about 2-7%, typically about 5%, as the balloon is pressurized from an inflation pressure of about 6 atm to a pressure of about 12 atm, that is, they have a "distension" over that pressure range of about 5%. "Semi-compliant" balloons have somewhat greater distensions, generally 7-16% and typically 10-12% over the same pressurization range. "Compliant" balloons are still more distensible, having distensions generally in the range of 16-40% and typically about 21% over the same pressure range. Maximum distensions, i.e. distension from nominal diameter to burst, of various balloon materials may be significantly higher than the distension percentages discussed above because wall strengths, and thus burst pressures, vary widely between balloon materials. These distension ranges are intended to provide general guidance, as one of skill in the art will be aware that the compliance of a balloon is dependent on the dimensions and/or characteristics of the cavity and/or lumen walls, not only the expandability of the balloon.

A compliant balloon may be used in the vasculature of a subject. A compliant balloon might also be used in any tube or hole outside the vasculature (whether naturally occurring or or man-made, or prepared during an injury). For a non-limiting example, a compliant balloon might be used in a lumpectomy to put a coating at the site where a tumor was removed, to: treat an abscess, treat an infection, prevent an infection, aid healing, promote healing, or for a combination of any of these purposes. The coating in this embodiment may comprise a growth factor.

"Non-Compliant balloon" as used herein refers to a balloon that does not conform to the intervention site, but rather, tends to cause the intervention site to conform to the balloon shape. Non-compliant balloons, commonly made from such materials as polyethylene terephthalate (PET) or polyamides, remain at a preselected diameter as the internal balloon pressure increases beyond that required to fully inflate the balloon. Non-compliant balloons are often used to dilate spaces, e.g., vascular lumens. As noted with respect to a compliant balloon, one of skill in the art will be aware that the compliance of a balloon is dependent on the dimensions and/or characteristics of the cavity and/or lumen walls, not only the expandability of the balloon.

"Cutting balloon" as used herein refers to a balloon commonly used in angioplasty having a special balloon tip with cutting elements, e.g., small blades, wires, etc. The cutting elements can be activated when the balloon is inflated. In angioplasty procedures, small blades can be used score the plaque and the balloon used to compress the fatty matter against the vessel wall. A cutting balloon might have tacks or other wire elements which in some embodiments aid in freeing the coating from the balloon, and in some embodiments, may promote adherence or partial adherence of the coating to the target tissue area, or some combination thereof. In some embodiments, the cutting balloon cutting elements also score the target tissue to promote the coating's introduction into the target tissue. In some embodiments, the cutting elements do not cut tissue at the intervention site. In some embodiments, the cutting balloon comprises tacking elements as the cutting elements.

"Inflation pressure" as used herein refers to the pressure at which a balloon is inflated. As used herein the nominal inflation pressure refers to the pressure at which a balloon is inflated in order to achieve a particular balloon dimension, usually a diameter of the balloon as designed. The "rated burst pressure" or "RBP" as used herein refers to the maximum statistically guaranteed pressure to which a balloon can be inflated without failing. For PTCA and PTA catheters, the rated burst pressure is based on the results of in vitro testing ot the PTCA and/or PTA catheters, and normally means that at least 99.9% of the balloons tested (with 95% confidence ) will not burst at or below this pressure.

The examples described herein are provided to illustrate selected embodiments. They should not be considered as limiting the scope of the invention, but merely as being illustrative and representative thereof. For each example listed herein, multiple analytical techniques may be provided. Any single technique of the multiple techniques listed may be sufficient to show the parameter and/or characteristic being tested, or any combination of techniques may be used to show such parameter and/or characteristic. Those skilled in the art will be familiar with a wide range of analytical techniques for the characterization of drug/polymer coatings. Techniques presented here, but not limited to, may be used to additionally and/or alternatively characterize specific properties of the coatings with variations and adjustments employed which would be obvious to those skilled in the art.

Sample Preparation Generally speaking, coatings on stents, on balloons, on coupons, on other substrates, or on samples prepared for in-vivo models are prepared as herein. Nevertheless, modifications for a given analytical method are presented within the examples shown, and/or would be obvious to one having skill in the art. Thus, numerous variations, changes, and substitutions will now occur to those skilled in the art without departing from the invention. It should be understood that various alternatives to the embodiments of the invention described herein and examples provided may be employed in practicing the invention and showing the parameters and/or characteristics described.

In some examples, the balloons are made of a compliant polymer. In some examples, the balloons are made of a non-compliant polymer. The balloons may be, in some examples, 5 to 50 mm in length, preferably 10-20 mm in length.

Balloons can be coated while inflated, and later compacted, or they can be coated while uninflated. If a balloon is coated while inflated and later folded or otherwise compacted, then a portion of the coating can be protected during insertion by virtue of being disposed within the portion of the balloon that is not exposed until inflation. The coating can also be protected by using a sheath or other covering, as described in the art for facilitating insertion of an angioplasty balloon.

The coating released from a balloon may be analyzed (for example, for analysis of a coating band and/or coating a portion of the balloon). Alternatively, in some examples, the coating is analyzed directly on the balloon. This coating, and/or coating and balloon, may be sliced into sections which may be turned 90 degrees and visualized using the surface composition techniques presented herein or other techniques known in the art for surface composition analysis (or other characteristics, such as crystallinity, for example). In this way, what could be an analysis of coating composition through a depth when the coating is on the balloon or as removed from the balloon (i.e. a depth from the abluminal surface of the coating to the surface of the removed coating that once contacted the balloon or a portion thereof), becomes a surface analysis of the coating which can, for example, show the layers in the slice of coating, at much higher resolution. Residual coating on an extracted balloon also can be analyzed and compared to the amount of coating on an unused balloon, using, e.g., HPLC, as noted herein. Coating removed from the balloon, or analyzed without removal and/or release from the balloon, may be treated the same way, and assayed, visualized, and/or characterized as presented herein using the techniques described and/or other techniques known to a person of skill in the art.

Sample Preparation for In-Vivo Models: Devices comprising balloons having coatings disclosed herein are deployed in the porcine coronary arteries of pigs (domestic swine, juvenile farm pigs, or Yucatan miniature swine). Porcine coronary angioplasty is exploited herein since such model yields results that are comparable to other investigations assaying neointimal hyperplasia in human subjects. The balloons are expanded to a 1:1.1 balloon:artery ratio. At multiple time points, animals are euthanized (e.g. t = 1 day, 7 days, 14 days, 21 days, and 28 days), the tissue surrounding the intervention site is extracted, and assayed.

Devices comprising balloons having coatings disclosed herein alternatively are implanted in the common iliac arteries of New Zealand white rabbits. The balloons are expanded to a 1:1.1 balloon:artery ratio. At multiple time points, animals are euthanized (e.g., t = 1 day, 7 days, 14 days, 21 days, and 28 days), the tissue surrounding the intervention site is extracted, and assayed.

In-vivo testing: A group of 27 New Zealand white rabbits is prepared for a Seldinger procedure using a balloon coated with PLGA according to an RESS proces followed by a nanoparticles of crystalline sirolimus with total loading of sirolimus -20 µg. The device is placed at a coronary artery intervention site with the assistance of fluoroscopy to aid in positioning the device at the same location in each subject. Six animals are subjected to the procedure using a coated balloon that does not have sirolimus in the coating. After deployment and removal of the device, 3 control animals are sacrificed at 1 hour post deployment and serum and tissue samples are collected. The 3 remaining control animals are sacrificed at 56 days post deployment. During the course of the study, serum samples are collected from control and drug-treated animals every five days. The drug treated animals, 3 each, are sacrificed at 1 hour, 24 hours, 7 days, 14 days, 28 days, 42 days and 56 days post deployment. A serum sample as well as a tissue sample from the deployment site is collected.

The tissue and serum samples may be subjected to analysis for sirolimus concentration. In order to determine the amount of coating freed from the device and/or delivered to the intervention site as a percent of the total amount of coating on the substrate, the tissue concentration of sirolimus at the one hour time point (or any time point within the first day following of the procedure) may be used used along with the total content expected for the coating (based on the total content for the manufacturing lot) or along with the content of coating remaining on the device once removed and the percentage calculated. This percentage is correlative of the percent of coating freed, dissociated, and/or transferred from the device and delivered to the intervention site. Alternatively, the tissue may be analyzed by various means (noted herein, including but not limited to SEM, TEM, and, where image enhanced polymers are used, various imaging means capable of detecting these enhanced polymers) to detect the percent of the coating freed, dissociated and/or transferred from the substrate and delivered to the intervention site. Again, the amount of coating known to be on the substrate based on manufacturing lot characteristics, and/or an assessment of the coating remaining on the device following removal of the device from the subject (for example, wherein the device is an angioplasty catheter and the substrate is the balloon of the catheter) may be used to determine the percent of coating freed, dissociated, and/or transferred from the device. In some instances, an assessment of the device following the procedure alone is sufficient to assess the amount freed or dissociated from the substrate, without determination of the amount delivered to the intervention site. Additionally, where a determination of improvement and/or disease treatment is desired, levels of proinflammatory markers could be tested to show improvement and/or treatment of a disease and/or ailment, for example, by testing high sensitive C-reactive protein (hsCRP), interleukin-6 (IL-6), interleukin-1β (IL-1β), and/or monocyte chemoattractant protein-1 (MCP-1). The release kinetics of the drug may be shown by plotting the sirolimus concentrations at the timepoints noted above.

For embodiments using different drugs other than sirolimus, the biomarkers are selected based on the disease to be treated and the drugs administered during the course of therapy as determined by one of skill in the art. These biomarkers may be used to show the treatment results for each subject.

Other in-vivo tests described herein may be used instead of this test and/or in addition to this test, adjusted for the particularities of this device, as would be known to one of ordinary skill in the art.

In-vitro testing: One sample of the coated balloon prepared as noted herein is secured to a balloon catheter. A segment of optically clear TYGON^{®} B-44-3 tubing with O.D. = 0.125", I.D. = 0.0625" (Available from McMaster-Carr Part Number: 5114K11 (www.mcmaster.com)) is filled with phosphate-buffered saline solution and immersed in a water bath at 37 °C to mimic physiological conditions of deployment into a subject. The coated balloon is inserted into the tubing and the balloon is inflated to at least 25% below the balloon's nominal pressure to mechanically transfer the coating from the balloon to the tubing wall. The balloon is deflated and removed from the tubing. Optical microscopy is performed on the tubing and/or the balloon (which is inflated to at least 25% below the balloon's nominal pressure, at least) to determine the presence and amount of coating transferred to the tubing and/or the amount of coating freed, dissociated, and/or transferred from the balloon. Other in-vitro tests described herein may be used instead of this test and/or in addition to this test, adjusted for the particularities of this device, as would be known to one of ordinary skill in the art.

In-vitro Coating test: One sample of the coated compliant balloon is secured to a balloon catheter. A segment of optically clear TYGON^{®} B-44-3 tubing with O.D. = 0.125", I.D. = 0.0625" (Available from McMaster-Carr Part Number: 5114K11 (www.mcmaster.com)) is filled with phosphate-buffered saline solution and immersed in a water bath at 37 °C to mimic physiological conditions of deployment into a subject. The coated balloon is inserted into the tubing and the balloon is inflated to at least 25% below the balloon's nominal pressure to mechanically transfer the coating from the balloon to the tubing wall. The balloon is deflated and removed from the tubing. The section of tubing exposed to the deployed balloon is cut away from the remainder of the tubing and the interior of the excised tubing rinsed with a small amount of ethanol and an amount of methylene chloride to make up 25 mL total volume of rinsings which are collected in a flask for analysis. Analysis by HPLC as described herein is performed to determine the amount of material freed, dissociated, and/or transferred from the balloon. This analysis may instead and/or alternatively include testing of the substrate itself to determine the amount of coating freed, dissociated, and/or transferred from the device during this in-vitro test.

For embodiments related to non-vascular or non-lumenal applications, e.g. a tumor site or other cavity or a cannulized site, the same technique is employed with the modification that the tissue to be assayed is resected from the tissue adjoining cavity receiving drug treatment.

Method for the determination of sirolimus levels: Media may be assayed for sirolimus content using HPLC. Calibration standards containing known amounts of drug are to determine the amount of drug eluted. The multiple peaks present for the sirolimus (also present in the calibration standards) are added to give the amount of drug eluted at that time period (in absolute amount and as a cumulative amount eluted). HPLC analysis is performed using Waters HPLC system, set up and run on each sample as provided in the Table 11 below using an injection volume of 100 uL (microliters).

**TABLE 11**

| Time point (minutes) | % Acetonitrile | % Ammonium Acetate (0.5%), pH 7.4 | Flow Rate (mL/min) |
|---|---|---|---|
| 0.00 | 10 | 90 | 1.2 |
| 1.00 | 10 | 90 | 1.2 |
| 12.5 | 95 | 5 | 1.2 |
| 13.5 | 100 | 0 | 1.2 |
| 14.0 | 100 | 0 | 3 |
| 16.0 | 100 | 0 | 3 |
| 17.0 | 10 | 90 | 2 |
| 20.0 | 10 | 90 | 0 |

In-vitro testing of release kinetics: One sample of the coated balloon with total loading of sirolimus -20 µg prepared as noted herein is secured to a balloon catheter. A flask containing exactly 25 mL of pH 7.4 aqueous phosphate buffer equilibrated to 37 °C equipped for magnetic stirring is prepared. Into this flask is placed the coated balloon and the catheter portion of the apparatus is secured such that the balloon does not touch the sides of the flask. The balloon is inflated to 120 psi with sterile water. Aliquots of 100 microliters are removed prior to addition of the balloon, after placement of the balloon but prior to inflation of the balloon, and at regular time intervals of 2, 4, 6, 8, 10, 12, and 14 minutes. Upon removal of each aliquot an equivalent volume of aqueous buffer is added to maintain the volume at 25 mL. The aliquots are analyzed by HPLC as described herein for the concentration of sirolimus.

Crystallinity The presence and or quantification of the Active agent crystallinity can be determined from a number of characterization methods known in the art, but not limited to, XRPD, vibrational spectroscopy (FTIR, NIR, Raman), polarized optical microscopy, calorimetry, thermal analysis and solid-state NMR.

X-Ray Diffraction to Determine the Presence and/or Quantification of Active Agent Crystallinity: Active agent and polymer coated proxy substrates are prepared using 316L stainless steel coupons for X-ray powder diffraction (XRPD) measurements to determine the presence of crystallinity of the active agent. The coating on the coupons is equivalent to the coating on the balloons or other substrates described herein. Coupons of other materials described herein, such as cobalt-chromium alloys, may be similarly prepared and tested. Likewise, substrates such as balloons, or other medical devices described herein may be prepared and tested. Where a coated balloon is tested, the balloon may be cut lengthwise and opened to lay flat in a sample holder.

For example XRPD analyses are performed using an X-ray powder diffractometer (for example, a Bruker D8 Advance X-ray diffractometer) using Cu Kα radiation. Diffractograms are typically collected between 2 and 40 degrees 2 theta. Where required low background XRPD sample holders are employed to minimize background noise.

The diffractograms of the deposited active agent are compared with diffractograms of known crystallized active agents, for example micronized crystalline sirolimus in powder form. XRPD patterns of crystalline forms show strong diffraction peaks whereas amorphous show diffuse and non-distinct patterns. Crystallinity is shown in arbitrary Intensity units.

A related analytical technique which may also be used to provide crystallinity detection is wide angle scattering of radiation (e.g.; Wide Anle X-ray Scattering or WAXS), for example, as described in F. Unger, et al., "Poly(ethylene carbonate): A thermoelastic and biodegradable biomaterial for drug eluting stent coatings?" Journal of Controlled Release, Volume 117, Issue 3, 312-321 (2007) for which the technique and variations of the technique specific to a particular sample would be obvious to one of skill in the art.

Raman Spectroscopy: Raman spectroscopy, a vibrational spectroscopy technique, can be useful, for example, in chemical identification, characterization of molecular structures, effects of bonding, identification of solid state form, environment and stress on a sample. Raman spectra can be collected from a very small volume (< 1 µm3 ); these spectra allow the identification of species present in that volume. Spatially resolved chemical information, by mapping or imaging, terms often used interchangeably, can be achieved by Raman microscopy.

Raman spectroscopy and other analytical techniques such as described in Balss, et al., "Quantitative spatial distribution of sirolimus and polymers in drug-eluting stents using confocal Raman microscopy" J. of Biomedical Materials Research Part A, 258-270 (2007), and/or described in Belu et al., "Three-Dimensional Compositional Analysis of Drug Eluting Stent Coatings Using Cluster Secondary Ion Mass Spectroscopy" Anal. Chem. 80: 624-632 (2008) may be used

For example, to test a sample using Raman microscopy and in particular confocal Raman microscopy, it is understood that to get appropriate Raman high resolution spectra sufficient acquisition time, laser power, laser wavelength, sample step size and microscope objective need to be optimized. For example a sample (a coated balloon) is prepared as described herein. Alternatively, a coated coupon could be tested in this method. Maps are taken on the coating using Raman microscopy. A WITec CRM 200 scanning confocal Raman microscope using a Nd:YAG laser at 532 nm is applied in the Raman imaging mode. The laser light is focused upon the sample using a 100× dry objective (numerical aperture 0.90), and the finely focused laser spot is scanned into the sample. As the laser scans the sample, over each 0.33 micron interval a Raman spectrum with high signal to noise is collected using 0.3 seconds of integration time. Each confocal cross-sectional image of the coatings displays a region 70 µm wide by 10 µm deep, and results from the gathering of 6300 spectra with a total imaging time of 32 min.

Multivariate analysis using reference spectra from samples of rapamycin (amorphous and crystalline) and polymer are used to deconvolve the spectral data sets, to provide chemical maps of the distribution.

Raman Spectroscopy may also and/or alternatively be used as described in Belu, et al., "Chemical imaging of drug eluting coatings: Combining surface analysis and confocal Rama microscopy" J. Controlled Release 126: 111-121 (2008) (referred to as Belu- Chemical Imaging). Coated balloons and/or coated coupons may be prepared according to the methods described herein, and tested according to the testing methods of Belu- Chemical Imaging.

A WITec CRM 200 scanning confocal Raman microscope (Ulm, Germany) using a NiYAG laser at 532 nm may be applied in Raman imaging mode. The balloon sample may be placed upon a piezoelectrically driven table, the laser light focused on the balloon coating using a 100x dry objective (Nikon, numerical aperture 0.90), and the finely focused laser spot scanned into the coating. As the laser scans the sample, over each 0.33 micron interval, for example, a Raman spectrum with high signal to noise may be collected using 0.3 s of integration time. Each confocal cross-sectional image of the coatings may display a region 70 micron wide by 10 microns deep, and results from the gathering of 6300 spectra with total imaging time of 32 min. To deconvolute the spectra and obtain separate images of drug (phramaceutical agent) and polymer, all the specrral data (6300 spectra over the entire spectral region 500-3500 cm-1) may be processed using an augmented classical least squares algorithm (Eigenvector Research, Wenatchee WA) using basis spectra obtained from samples of the drug (e.g. rapamycin amorphous and/or crystalline) and the polymer (e.g. PLGA or other polymer).

For each balloon, several areas may be measured by Raman to ensure that the trends are reproducible. Images may be taken on the coatings before elution, and/or at time points following elution. For images taken following elution, balloons may be removed from the elution media and dried in a nitrogen stream. A warming step (e.g. 70C for 10 minutes) may be necessary to reduce cloudiness resulting from soaking the coating in the elution media (to reduce and/or avoid light scattering effects when testing by Raman).

Infrared (IR) Spectroscopy: Infrared (IR) Spectroscopy such as FTIR and ATR-IR are well utilized techniques that can be applied to show, for example, the quantitative drug content, the distribution of the drug in the sample coating, the quantitative polymer content in the coating, and the distribution of polymer in the coating. Infrared (IR) Spectroscopy such as FTIR and ATR-IR can similarly be used to show, for example, drug crystallinity and/or identity. The following table (Table 12) lists the typical IR materials for various applications. These IR materials are used for IR windows, diluents or ATR crystals.

**TABLE 12**

| MATERIAL | NACL | KBR | CSI | AGCL | GE | ZNSE | DIAMOND |
|---|---|---|---|---|---|---|---|
| Transmission range (cm-1) | 40,000 ∼625 | 40,000 ∼400 | 40,000 ∼200 | 25,000 ∼360 | 5,500 ∼625 | 20,000 ∼454 | 40,000 ∼2,500 & 1667-33 |
| Water sol (g/100g, 25C) | 35.7 | 53.5 | 44.4 | Insol. | Insol. | Insol. | Insol. |
| Attacking materials | Wet Solvents | Wet Solvents | Wet Solvents | Ammonium Salts | H2SO4, aqua regin | Acids, strong alkalies, chlorinated solvents | K2Cr2Os, conc. H2SO4 |

In one test, a coupon of crystalline ZnSe is coated by the processes described herein, preparing a PDPDP (Polymer, Drug, Polymer, Drug, Polymer) layered coating that is about 10 microns thick. The coated coupon is analyzed using FTIR. The resulting spectrum shows crystalline drug as determined by comparison to the spectrum obtained for the crystalline form of a drug standard (i.e. a reference spectrum).

Differential Scanning Calorimetry (DSC) to test Crystallinity DSC can provide qualitative evidence of the crystallinity of the drug (e.g. rapamycin) using standard DSC techniques obvious to one skilled in the art. Crystalline melt can be shown using this analytical method (e.g. rapamycin crystalline melting - at about 185 decrees C to 200 degrees C, and having a heat of fusion at or about 46.8 J/g). The heat of fusion decreases with the percent crystallinity. Thus, the degree of crystallinity could be determined relative to a pure sample, or versus a calibration curve prepared from a sample of amorphous drug spiked and tested by DSC with known amounts of crystalline drug. Presence (at least) of crystalline drug on a balloon could be measured by removing (scraping or stripping) some drug from the balloon and testing the coating using the DSC equipment for determining the melting temperature and the heat of fusion of the sample as compared to a known standard and/or standard curve.

Atomic Force Microscopy (AFM) to test Coating Microstructure: AFM is a high resolution surface characterization technique. AFM is used in the art to provide topographical imaging, in addition when employed in Tapping Mode^{™} can image material and or chemical properties of the surface. Additionally cross-sectioned samples can be analyzed. The technique can be used under ambient, solution, humidified or temperature controlled conditions. Other modes of operation are well known and can be readily employed here by those skilled in the art.

A balloon as described herein is obtained. AFM is used to determine the microstructure of the coating. A balloon as described herein is obtained. AFM may be employed as described in Ranade et al., "Physical characterization of controlled release of paclitaxel from the TAXUS Express2 drug-eluting stent" J. Biomed. Mater. Res. 71(4):625-634 (2004).

For example, polymer and drug morphologies, coating composition, and physical structure may be determined using atomic force microscopy (AFM) analysis. A multi-mode AFM (Digital Instruments/Veeco Metrology, Santa Barbara, CA) controlled with Nanoscope IIIa and NanoScope Extender electronics is used. Samples are examined in the dry state using AFM before elution of the drug (e.g. rapamycin). Samples are also examined at select time points through a elution period (e.g. 48 hours) by using an AFM probe-tip and flow-through stage built to permit analysis of wet samples. The wet samples are examined in the presence of the same elution medium used for in-vitro kinetic drug release analysis (e.g. PBS-Tween20, or 10 mM Tris, 0.4 wt.% SDS, pH 7.4). Saturation of the solution is prevented by frequent exchanges of the release medium with severl volumes of fresh medium. TappingMode^{™} AFM imaging may be used to show topography (a real-space projection of the coating surface microstructure) and phase-angle changes of the AFM over the sample area to contrast differences in the materials properties. The AFM topography images can be three-dimensionally rendered to show the surface of a coated balloon, which can show holes or voids of the coating which may occur as the polymer is absorbed and the drug is released from the polymer over time, for example.

Nano X-Ray Computer Tomography to test Coating Microstructure: Another technique that may be used to view the physical structure of a device in 3-D is Nano X-Ray Computer Tomography (e.g. such as made by SkyScan), which could be used in an elution test and/or bioabsorbability test, as described herein to show the physical structure of the coating remaining on balloons at each time point, as compared to a scan prior to elution/ bioabsorbtion.

Determination of the Total Content of the Active Agent: Determination of the total content of the active agent in a coated balloon may be tested using techniques described herein as well as other techniques obvious to one of skill in the art, for example using GPC and HPLC techniques to extract the drug from the coated balloon and determine the total content of drug in the sample.

UV-VIS can be used to quantitatively determine the mass of rapamycin coated onto the balloons. A UV-Vis spectrum of Rapamycin can be shown and a Rapamycin calibration curve can be obtained, (e.g. λ @ 277nm in ethanol). Rapamycin is then dissolved from the coated balloon in ethanol, and the drug concentration and mass calculated.

In one test, the total amount of rapamycin present in units of micrograms per balloon is determined by reverse phase high performance liquid chromatography with UV detection (RP-HPLC-UV). The analysis is performed with modifications of literature-based HPLC methods for rapamycin that would be obvious to a person of skill in the art. The average drug content of samples (n=10) from devices comprising balloons and coatings as described herein, and/or methods described herein are tested.

### EXAMPLE 1: COATINGS PREPARED WITH AND WITHOUT SHEAR MIXING (the examples demonstrate the principle of the invention but are not according to the invention as claimed).

A formulation of coating called F15 (Formulation 15) herein was produced in multiple lots and having various rapamycin: polyarginine ratios. F15 (Formulation 15) comprised PLGA i.e. about 50:50 Lactic acid: Glycolic acid, Sirolimus having an average size of 1.5 µm, and Polyarginine 5-15 kDa.

Coated balloons were prepared using the F15 coating lots, however, this coating could be applied to any medical device. Thus, although this example is, in certain descriptions, stated with respect to balloons, any device could be coated with this coating and delivered to tissue of a treatment site. In some embodiments, the treatment site for the coating is actually the tissue en route to a site that is the focus of a surgery or other diagnostic test or intervention. Likewise, other active agents, binding agents (surfactants, etc) and/or polymers could be used adapting methods and description herein to form the coating and/or coated device, even though this example references sirolimus, PLGA, and polyarginine.

The method for producing the devices coated in this example comprised using an RESS process for coating the PLGA on the balloon, and using an eSTAT process for coating the Sirolimus and the positive charged molecule to the balloon. The general process for coating was 1) Polymer coat by RESS processes, 2) Sirolimus and binding agent coat by eSTAT processes, 3) sinter the coated balloon. The binding agent (i.e. charged particle, surfactant, and/or cationic particle) was part of the Sirolimus coating step wherein the balloon was coated with both the Sirolimus and the binding agent using an eSTAT process.

The sirolimus was mixed with the binding agent (e.g. the surfactant, the cationic particle, the charged molecule, for non-limiting example) in the following manner. Again, the process may be adapted to different binding agents and different active agents, however, it is described herein as used with respect to sirolimus and the binding agents which were surfactants in the formulations noted in this example. Lyophilisation or "freeze drying" processed produced a dry powder of associated drug and binding agent (e.g. surfactant) suitable for depositing onto balloons via the eSTAT method. Other processes familiar to one of skill in the art may be used as an alternative to lyophilisation in order to associate the drug and binding agent in a form suitable for deposition on the balloons via a method described herein. In this example, rapamycin (sirolimus) was suspended in water with a binding agent to coat the sirolimus with binding agent. The well-suspended sirolimus and binding agent solution was frozen, retaining the sirolimus and binding agent assembly, and the water was removed by sublimation to produce the dry sirolimus and binding agent material.

A pre-lyophilisation set of steps may be used in the process of preparing the dried sirolimus and binding agent solution for use in the eSTAT coating process. The solution prepared thereby may be used in a freeze dryer. The desired quantity of drug (e.g. sirolimus) and binding agent were weighed out into a 100mL Schott bottle. Then 50mL of water is added, in increments of 10mL, to the Schott bottle. During each increment the solution is mixed with a stir rod to insure the sirolimus is being wetted. After the 50mL is added the solution is sonicated in a bath sonicator (Branson 1510) for 1 hr. In the final pre-lyophilisation step, the well-suspended solution is carefully transferred to a 50mL conical centrifuge tube using a plastic pipette; unsuspended sirolimus and/or binding agent particles (typically found floating on the surface of the suspension, are not transferred. Note: the efficiency of the sirolimus suspension by the binding agent affects the actual sirolimus to surfactant ratio of the transferred solution and the final recovered powder, often changing it from the initial sirolimus and binding agent ratio weighed out.

The Lyophilisation steps may be as follows: The recovered suspension in the 50mL centrifuge tube is immersed in liquid nitrogen until the solution is completely frozen. Parafilm is used to cover the opening of the tube containing the frozen suspension, while perforations are made in the film to allow escape of the vapor phase water. The tube containing the frozen sample is loaded into a freeze dryer containment vessel and the vessel is attached to one of the freeze dryer stations. The switch above the nozzle for the loaded station is activated to begin the process. The lyophilisation step is complete when all of the frozen moisture is visibly absent from the tube. The sample, which may exist as a xerogel following lyophilisation, is easily converted to a free-flowing dry powder by shaking or stirring when the process is complete. It usually takes 1-2 days for a sample prepared as described above to complete the lyophilisation step. Note: the freeze-drier may need to be periodically be defrosted to remove the accumulated moisture from the samples in order to work effectively.

The following steps were taken to make the sirolimus and binding agent dry solution in the eSTAT coating process of the balloons (which had been pre-coated using an RESS process with PLGA as noted elsewhere herein). Measure out required quantities of sirolimus and binding agent into a 100mL Schott bottle. Add 50mL of water, in increments of 10mL, to the Schott bottle. During each increment use a stir rod to mix the sirolimus and binding agent solution. After 50mL of water is added, sonicate the solution for 1 hr. After sonication use a plastic pipette to transfer the suspended solution to a 50mL centrifuge tube. Avoid transfer of any unsuspended sirolimus and binding agent particles. Place 50mL conical tube (without lid) in liquid nitrogen until solution is completely frozen. Cover the top of the conical tube with parafilm and make holes in film for water to travel through. Seal the 50mL conical bottle in the lyophilisation vessel and connect the vessel to a freeze dryer nozzle station. Turn switch above the nozzle to evacuate the air from the vessel. Keep sample on the freeze-drier until all water has been removed (typically 1-2 days)

The F15 coated balloons (Lot 1) had 106.53 ± 22.55 µg average amount of sirolimus coated on each of the balloons tested in this example. These were average amounts of drug found on sample balloons coated according to the same procedures noted herein. The amount of sirolimus coated on the balloon is the average sirolimus concentration based on UV-Vis analysis before pleating, folding, and sterilization of the balloons.

Additional lots of F15 were produced with multiple sirolimus: polyarginine ratios. Regardless of the rapamycin: polyarginine ratio, however, indicative of F15 is that it comprises PLGA i.e. about 50:50 Lactic acid: Glycolic acid, Sirolimus having an average size of 1.5 µm, and Polyarginine 5-15 kDa. The sirolimus was in crystalline form. The following Rapamycin:Polyarginine Ratios were produced for this Example: 1:1, 5:1, 10:1, and 50:1.

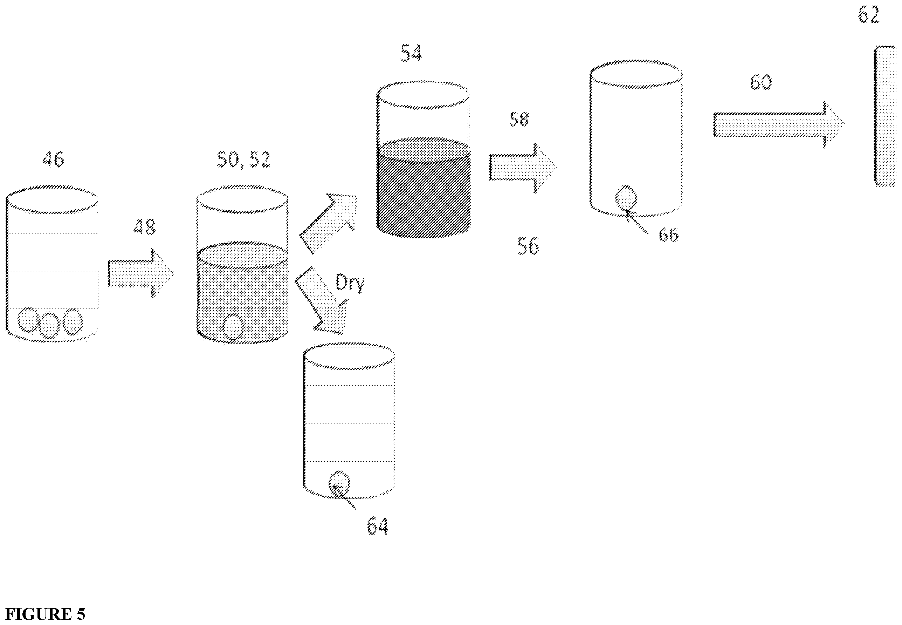

In some lots (generally called F15 Lot 3 1:1, 5:1, 10:1, or 50:1), the production method for the formulation was as depicted in Figure 5. The method for these lots was as follows: Dissolve 25mg Poly-L-arginine hydrochloride (Aldrich P4663) (also called polyarginine herein) (cas 26982-20-7, 5-15kDa) in 50ml deionized water in 100ml bottle and add 250mg Sirolimus (1.5 micron particle size, crystalline form) (step 46). Sonicate (Branson 1510 bench top ultrasonic cleaner) for 2h (step 48). Manually separate well-suspended liquid portion from unsuspended solids using pipette (step 50). Centrifuge ∼50ml suspension for 30min at 10,000 rpm (ThermoElectronCorp. IEC Multi RF Centrifuge) (step 52). Decant supernatant without allowing sediment to come to dryness (step 54). There will be an amount of unsupended fraction 64 following centrifuge step 52. Add aqueous solution of poly-L-arginine hydrochloride concentration to produce desired Sirolimus/poly-L-arginine hydrochloride ratio (step 56). Re-suspend sediment by shaking and 10 minute sonication. Lyophilize suspension to produce un-agglomerated Sirolimus/polyarginine powder solid lyophilisate 66 (Flexi-Dry MP) (step 58). This step took two to three days to achieve completion. This lyophilized solid 66 was eSTAT coated onto PLGA coated balloons as a powder (dry coating as described herein) (step 60) to produce a coated balloon 62 comprising PLGA, rapamycin, and polyarginine, wherein the rapamycin is crystalline in form.

For the ratio-forming step, the ratios were produced as follows: 95ml Masterbatch (combination of "well-suspended" portions of 4 sonicated solutions), estimated to be ∼5mg/ml solids, was divided into 5 portions. For the 50:1 ratio of sirolimus to polyarginine, 20ml water was added to the first 18ml portion, it was sonicated to re-suspend and lyophilized to produce 50.3 mg solid lyophilisate. For the 10:1 ratio of sirolimus to polyarginine, 9 mg polyarginine was dissolved in 20ml water and was added to the second 18ml portion whis was sonicated to re-suspend and lyophilized to produce 116.6 mg solid lyophilisate. For the 5:1 ratio of sirolimus to polyarginine, 18mg polyarginine was dissolved in 20ml water and was added to the third 18ml portion, it was sonicated to re-suspend and lyophilized to produce 127.4 mg solid lyophilisate. For the 1:1 ratio of sirolimus to polyarginine, 90mg polyarginine was dissolved in 20ml water and was added to the fourth 18ml portion, it was sonicated to re-suspend and lyophilized to produce 142.4 mg solid lyophilisate.

In some lots (generally called F15 Lot 4 5:1 or 10:1), the production methods for a F15 formulation having a 5:1 ratio of rapamycin to polyarginine lot and a F15 formulation having a 10:1 ratio of rapamycin to polyarginine lot included a high shear mixer to improve the suspension and reduce sediment as compared to that found in Lot 3 (improving yield and actual ratio of the formulation as compared to the target ratio). Steps were as follows. Dissolve 25mg (10:1) or 50 mg (5:1) Poly-L-arginine hydrochloride (Aldrich P4663) (cas 26982-20-7) (5-15kDa) (also called polyarginine) in 25ml deionized water in 20ml vial. Add 250mg Sirolimus (1.5micron particle size, crystalline in form). Mix for 10min at 10,000 rpm in Laboratory Mixer (Silverson L4RT) using micro mixer head attachment to form a suspension (this mixing leaves little or no sediment). The Lab Mixer is a High Shear Mixer having an impeller for mechanical mixing. Run mixer with 25ml pure water to recover residual material (rinse water). Combine suspension with rinse water. Lyophilize suspension to produce un-agglomerated Sirolimus/polyarginine powder (Flexi-Dry MP), which took two to three days to achieve completion. This lyophilized solid 66 was eSTAT coated onto PLGA coated balloons as a powder (dry coating as described herein) to produce a coated balloon comprising PLGA, rapamycin, and polyarginine, wherein the rapamycin is crystalline in form.

The amount of rapamycin that was found in the actual coated balloon was also determined and could be used to determine an actual rapamycin:polyarginine ratio (as opposed to the target ratio provided as noted elsewhere herein). To measure the amount of sirolimus on individual balloons ultraviolet-visible spectroscopy (UV-Vis) was employed. After sintering, coated balloons are cut (the stylus is removed before cutting) from the catheter wires leaving only ∼1/4" of the wires remaining connected to the balloons. Balloons were placed in individual 5 ml scintillation vials containing 4 ml of ethanol or methanol (sirolimus is soluble in ethanol up to 50 mg/ml). Sonication for 3 h removes sirolimus from the balloons. Following sonication UV-Vis is performed. Due to sirolimus being a triene (containing three double bonds) it produces UV absorbance at 3 wavelengths: 1 major peak at 277 nm and two smaller peaks at 267 nm and 288 nm. Uncoated GHOST rapid exchange nylon balloons and PLGA have also been individually sonicated for 3 h in ethanol and showed no interfering extractives for sirolimus measurements. The absorbance of sirolimus subtracted from the absorbance of an uncoated balloon at 277 nm is used in conjunction with a standard curve to calculate the amount of sirolimus per coated balloon. A standard of 3 from a batch of 12 coated balloons underwent UV-Vis analysis to obtain a batch average of sirolimus per balloon (measured in µg). The UV-Vis analysis could also be used to determine the presence and/or quantitate the amount of polymer (in this Example, PLGA) in the coating. UV-Vis testing of both lots 3 and 4 revealed presence of both polyarginine and rapamycin on the coated balloons. For Lot 3, the following actual ratios were determined: for Lot 3 1:1 (ideal) actual was about 1.3:1, for Lot 3 5:1 (ideal) actual was about 7.1:1, Lot 3 10:1(ideal) actual was about 14.3:1, for Lot 3 50:1(ideal) actual was about 43.1:1. Likewise, for Lot 4 5:1(ideal) actual was about 6.1:1.

F15 Lot 3 coated balloons were delivered to arteries of animals of a rabbit study to assess if and how much of the rapamycin was retained in rabbit iliac arteries for 72 hours. The following coated balloon formulations were as follows in Table 13

**Table 13:**

| Sirolimus: Polyarginine Ratio | Sirolimus/Balloon (µg) (n=6)^{∗} | Coating Appearance |
|---|---|---|
| 1:1 | 65.37 ± 3.84 | Transparent, Speckled |
| 5:1 | 79.02 ± 9.83 | Translucent, Very Thick |
| 10:1 | 89.71 ± 5.27 | Translucent, Very Thick |
| 50:1 | 81.28 ± 4.61 | Translucent, Very Thick |

| | | |
|---|---|---|
| ^{∗} Average Sirolimus concentrations based on UV-Vis analysis before pleating/folding and sterilization of balloons. | | |

Study design for this study was as depcited in Table 14.

**Table 14:**

| Test Article | Animals | Number of Vessels Per Animal | Blood PK Per Animal | Necropsy Time Points |
|---|---|---|---|---|
| F15 Sirolimus: Polyarginine Ratios: 1:1, 5:1, 10:1, 50:1 | n=3 per ratio | n=2 denuded rabbit iliac arteries | n=2 time points (baseline/before necropsy) 3 days (± 5%) | |
| Totals | 12 | 24 | 24 | 3 days (± 5%) |

Deployed balloons, blood samples and denuded iliac arteries analyzed for Sirolimus levels.

The following results were determined as shown in Tables 15, 16, 17. Most retention from 10:1 ratio of F15 Lot 3 (1.8 ± 1.2 ng/mg). Sirolimus blood levels below 1 ng/ml by 3 days.

**Table 15:**

| F15 Ratio | Arterial Sirolimus Concentration (ng/mg) | SD | Total Sirolimus per Artery (µg) | SD |
|---|---|---|---|---|
| F15 1:1 Lot 3, n=6 | 1.52 | 2.63 | 0.022 | 0.035 |
| F15 5:1 Lot 3, n=6 | 0.67 | 0.53 | 0.013 | 0.011 |
| F15 10:1 Lot 3, n=6 | 1.77 | 1.18 | 0.041 | 0.030 |
| F15 50:1 Lot 3, n=6 | 0.63 | 0.18 | 0.010 | 0.003 |

**Table 16:**

| F15 Ratio | Sirolimus Concentration on Balloon After Deployment (ug) | % Sirolimus Released/Lost^{∗} |
|---|---|---|
| 1:1 Lot 3 | 20.6 ± 9.8 (n=6) | 68.9 ± 14.0% (n=6) |
| 5:1 Lot 3 | 37.9 ± 6.3 (n=6) | 52.0 ± 7.8% (n=6) |
| 10:1 Lot 3 | 41.3 ± 7.2 (n=6) | 53.7 ± 9.1% (n=6) |
| 50:1 Lot 3 | 45.4 ± 6.9 (n=6) | 44.2 ± 8.6% (n=6) |

| | | |
|---|---|---|
| ^{∗} Based on Balloon Batch Averages of Sirolimus | | |

The amount of Sirolimus coated on balloons was as follows: F15 (1:1, Lot 3) Sirolimus coated on balloons = 65.37 ± 3.84; F15 (5:1, Lot 3) Sirolimus coated on balloons = 79.02 ± 9.83; F15 (10:1, Lot 3) Sirolimus coated on balloons = 89.71 ± 5.27; F15 (50:1, Lot 3) Sirolimus coated on balloons = 81.28 ± 4.61.

**Table 17:**

| F15 Ratio | Sirolimus Concentration in Whole Blood (ng/mL) | Est. Total Sirolimus in Blood (µg) ^{∗} |
|---|---|---|
| 1:1 Lot 3 | 0.29 ± 0.03 (n=3) | 0.054 ± 0.01 (n=3) |
| 5:1 Lot 3 | 0.50 ± 0.15 (n=3) | 0.096 ± 0.03 (n=3) |
| 10:1 Lot 3 | 0.43 ± 0.12 (n=3) | 0.081 ± 0.02 (n=3) |
| 50:1 Lot 3 | 0.38 ± 0.08 (n=3) | 0.064 ± 0.01 (n=3) |

| | | |
|---|---|---|
| ^{∗} Based on 56 mL Blood per kg; BQL = below quantitation limit (0.1 ng/ml) | | |

F15 Lot 4 coated balloons were delivered to arteries of animals of a rabbit study to assess if and how much of the rapamycin was retained in rabbit iliac arteries for 72 hours.

A method may comprise coating at least a portion of a medical device thereby forming on the medical device a coating comprising an active agent and a binding agent, wherein the method comprises: dissolving the binding agent to form a binding agent solution, combining the binding agent solution and the active agent, mixing the combined binding agent and active agent using a high shear mixer, forming a suspension comprising the combined mixed active agent and binding agent, lyophilising the suspension to form a lyophilisate of the active agent and the binding agent, and coating the medical device with the lyophilisate in powder form using an eSTAT process, wherein the active agent coated on the medical device comprises active agent in crystalline form.

In some embodiments, the high shear mixer is a mechanical mixer. In some embodiments, the mechanical mixer comprises an impeller, propeller, and/or a high speed saw tooth disperser. In some embodiments, the mechanical mixer comprise a high pressure pump. In some embodiments, the high shear mixer comprises a sonic mixer. In some embodiments, the sonic mixer comprises a sonicator. In some embodiments, the sonic mixer comprises a benchtop bath based sonicator. In some embodiments, the sonic mixer comprises an ultrasonic mixer. In some embodiments, the sonic mixer comprises an megasonic mixer.

In some embodiments, the mechanical mixer comprise a high pressure pump (up to 40,000 psi (2578 bar)) that forces particles into an interaction chamber at speeds up to 400 m/s. The interaction chamber may comprise engineered microchannels. Inside the chamber, the product may be exposed to consistent impact and shear forces and then cooled.

A high shear mixer disperses, or transports, one phase or ingredient (liquid, solid, gas) into a main continuous phase (liquid), with which it would normally be immiscible. In some embodiments of a mechanical mixer that is a high shear mixer, a rotor or impellor, together with a stationary component known as a stator, or an array of rotors and stators, is used either in a tank containing the solution to be mixed, or in a pipe through which the solution passes, to create shear. A high shear mixer can be used to create emulsions, suspensions, lyosols (gas dispersed in liquid), and granular products.

Fluid undergoes shear when one area of fluid travels with a different velocity relative to an adjacent area. In some embodiments of a mechanical mixer that is a high shear mixer, the high shear mixer uses a rotating impeller or high-speed rotor, or a series of such impellers or inline rotors, usually powered by an electric motor, to "work" the fluid, creating flow and shear. The tip velocity, or speed of the fluid at the outside diameter of the rotor, will be higher than the velocity at the centre of the rotor, and it is this velocity difference that creates shear.

A stationary component may be used in combination with the rotor, and is referred to as the stator. The stator creates a close-clearance gap between the rotor and itself and forms an extremely high shear zone for the material as it exits the rotor. The rotor and stator combined together are often referred to as the mixing head, or generator. A large high shear rotor-stator mixer may contain a number of generators.

In some embodiments the mechanical mixer comprises a batch high shear mixer. In a batch high shear mixer, the components to be mixed (whether immiscible liquids or powder in liquid) are fed from the top into a mixing tank containing the mixer on a rotating shaft at the bottom of the tank. A batch high shear mixer can process a given volume of material approximately twice as fast as an inline rotor-stator mixer of the same power rating; such mixers continue to be used where faster processing by volume is the major requirement, and space is not limited. When mixing sticky solutions, some of the product may be left in the tank, necessitating cleaning. However, there are designs of batch high shear mixers that clean the tank as part of the operating run. Some high shear mixers are designed to run dry, limiting the amount of cleaning needed in the tank.

In some embodiments the mechanical mixer comprises an inline high shear rotor-stator mixer. Generally speaking this version takse the same rotor and stator from tbe batch high shear mixer and installs it in a housing with inlet and outlet connections. Then the rotor is driven through a shaft seal thus resuling in a rotor-stator mixer that behaves like a centrifugal pumping device. That is, in an inline high shear rotor-stator mixer, the rotor-stator array is contained in a housing with an inlet at one end and an outlet at the other, and the rotor driven through a seal. The components to be mixed are drawn through the generator array in a continuous stream, with the whole acting as a centrifugal pumping device. Inline high shear mixers offer a more controlled mixing environment, take up less space, and can be used as part of a continuous process. Equilibrium mixing can be achieved by passing the product through the inline high shear mixer more than once. Since the inline mixer may be positioned in a flowing stream, the mixing may be more controlled than in a batch configuration, so the number of passes through the high shear zone can be monitored.

An inline rotor-stator mixer equipped for powder induction offers flexibility, capability, and portability to serve multiple mix vessels of virtually any size. Its straightforward operation and convenience further maximize equipment utility while simplifying material handling.

When used with a vacuum pump and hopper, an inline shear mixer can be a very effective way to incorporate powders into liquid streams. Otherwise known as high shear powder inductors, these systems have the advantage of keeping the process on the floor level instead of working with heavy bags on mezzanines. High shear powder induction systems also offer easy interchangeability with multiple tanks.

A high shear granulator is a process array consisting of an inline or batch high shear mixer and a fluid-bed dryer. In a granulation process, only the solid component of the mixture is required. Fluid is used only as an aid to processing. The high shear mixer processes the solid material down to the desired particle size, and the mixture is then pumped to the drying bed where the fluid is removed, leaving behind the granular product.

In an ultra-high shear inline mixer, the high shear mixing takes place in a single or multiple passes through a rotor-stator array. The mixer is designed to subject the product to higher shear and a larger number of shearing events than a standard inline rotor-stator mixer, producing an exceptionally narrow particle-size distribution. Sub-micrometre particle sizes are possible using the ultra-high shear technology. To achieve this, the machine is equipped with stators with precision-machined holes or slots through which the product is forced by the rotors. The rotor-stator array can also include a mechanism whereby the momentum of the flow is changed (for example by forcing it sideways through the stator), allowing for more processing in a single pass.

High shear mixers may be used to produce standard mixtures of ingredients that do not naturally mix. When the total fluid is composed of two or more liquids, the final result is an emulsion; when composed of a solid and a liquid, it is termed a suspension and when a gas is dispersed throughout a liquid, the result is a lyosol. Each class may or may not be homogenized, depending on the amount of input energy.

To achieve a standard mix, the technique of equilibrium mixing may be used. A target characteristic is identified, such that once the mixed product has acquired that characteristic, it will not change significantly thereafter, no matter how long the product is processed. For dispersions, this is the equilibrium particle size. For emulsions, it is the equilibrium droplet size. The amount of mixing required to achieve equilibrium mixing is measured in tank turnover - the number of times the volume of material must pass through the high shear zone.

In some embodiments, the sonic mixer comprises a sonicator. In some embodiments, the sonic mixer comprises a benchtop bath based sonicator. In some embodiments, the sonic mixer comprises an ultrasonic mixer. The ultrasonic mixer may employ ultrasonic frequencies of any one or more of: about 18kHz at least, about 20kHz at least, less than 400 kHz, less than 500 kHz, about 18kHz to about 400kHz, about 20kHz to about 500 kHz, at most about 400kHz, and at most about 500 kHz. In some embodiments, the sonic mixer comprises an megasonic mixer. The megasonic mixer may employ megasonic frequencies of any one or more of: about 500kHz at least, about 700kHz at least, about 800kHz at least, less than about 5MHz, less than about 4MHz, about 500kHz to about 5MHz, about 700kHz to about 4MHz, at most about 5MHz, at most about 4MHz, at least about 1MHz, and any frequency in the MHz range.

In some embodiments, a ratio of the active agent to the binding agent is 1:1, 2:1, 3:1, 4:1, 5:1, 10:1, 3:2, 52 or 5:3 as a target ratio. In some embodiments, the actual ratio of the active agent to the binding agent is +/-30% of the target ratio. In some embodiments, the actual ratio is calculated based on UV-Vis testing of the medical device.

In some embodiments, when the device is delivered to a treatment site in vivo, at least 3%, at least 5%, or at least 10% of the active agent is transferred to tissue of the treatment site.

In some embodiments, the binding agents comprises at least one of: Polyarginine, Polyarginine 9-L-pArg, DEAE-Dextran (Diethylaminoethyl cellulose- Dextran), DMAB (Didodecyldimethylammonium bromide), PEI (Polyethyleneimine), TAB (Tetradodecylammonium bromide), and DMTAB (Dimethylditetradecylammonium bromide).

In some embodiments, an average molecular weight of the binding agent is controlled.

In some embodiments, a size of the active agent in the coating is controlled.

In some embodiments, the active agent is sirolimus and wherein the sirolimus has have an average size of at least one of: about 1.5 µm, about 2.5 µm, about 645nm, about 100-200 nm, another controlled size, or a combination thereof (wherein 1.5 and 2.5 micrometers are not according to the invention).

In some embodiments, the active agent is sirolimus and wherein sirolimus at least 50%, at least 75% and/or at least 90% of the sirolimus as is 1.5 µm, 2.5 µm, 645nm, 100-200 nm, or another controlled size (wherein 1.5 and 2.5 micrometers are not according to the invention).

The coating comprises nanoparticles, and the nanoparticles may comprise an active agent and a polymer.

In some embodiments, the coating comprises PLGA comprising about 50:50 Lactic acid: Glycolic acid.

In some embodiments, the coating comprised and about a 10:1 ratio of the active agent to the binding agent, wherein the active agent comprises sirolimus wherein the binding agent comprises Polyarginine.

In some embodiments, which are not according to the invention, the sirolimus has an average size of 1.5 µm or 2.5 µm.

In some embodiments, the Polyarginine average molecular weight is 70kDa. In some embodiments, the Polyarginine average molecular weight is 5-15kDa.

In some embodiments, the active agent and the binding agent are lyophilized prior to deposition on the medical device.

In some embodiments, at least about 2 ng/mg of active agent, at least about 3 ng/mg of active agent, at least about 5 ng/mg of active agent, at least about 10 ng/mg of active agent, at least about 20 ng/mg of active agent, at least about 30 ng/mg of active agent, and/or at least about 40 ng/mg of active agent are found in tissue 72 hours after delivery of the medical device to the treatment site.

In some embodiments, the device releases at least one of: at least 5% of the active agent to tissue upon delivery of the medical device to the treatment site, at least 7% of the active agent to tissue upon delivery of the medical device to the treatment site, at least 10 % of the active agent to tissue upon delivery of the medical device to the treatment site, at least 15 % of the active agent to tissue upon delivery of the medical device to the treatment site, at least 20 % of the active agent to tissue upon delivery of the medical device to the treatment site, at least 25 % of the active agent to tissue upon delivery of the medical device to the treatment site, at least 25 % of the active agent to tissue upon delivery of the medical device to the treatment site, at least 30 % of the active agent to tissue upon delivery of the medical device to the treatment site, at least 40 % of the active agent to tissue upon delivery of the medical device to the treatment site, at least 50 % of the active agent to tissue upon delivery of the medical device to the treatment site, between 2% and 50% of the active agent to tissue upon delivery of the medical device to the treatment site, between 3% and 50% of the active agent to tissue upon delivery of the medical device to the treatment site, between 5% and 50% of the active agent to tissue upon delivery of the medical device to the treatment site, between 3% and 30% of the active agent to tissue upon delivery of the medical device to the treatment site, between 3% and 25% of the active agent to tissue upon delivery of the medical device to the treatment site, between 3% and 20 of the active agent to tissue upon delivery of the medical device to the treatment site, between 3% and 15% of the active agent to tissue upon delivery of the medical device to the treatment site, between 1% and 15% of the active agent to tissue upon delivery of the medical device to the treatment site, between 1% and 10% of the active agent to tissue upon delivery of the medical device to the treatment site, between 3% and 10% of the active agent to tissue upon delivery of the medical device to the treatment site, and between 1% and 5% of the active agent to tissue upon delivery of the medical device to the treatment site.

Much of the description herein is provided with reference to a balloon and a treatment site that is a artery for ease of description and brevity. Nevertheless, the methods, descriptions, devices, and coatings described herein apply to alternative devices and treatment locations.

Unless otherwise stated, use of the term "about" in this description can mean variations of 0.1%, 0.5%, 1%, 2%, 3%, 5%, 10%, 15%, 20%, 25%, 30%, 40%, and/or 50%, depending on the particular embodiment. Where the element being described is itself expressed as a percent, the variations are not meant to be percents of percents, rather they are variations as an absolute percent-i.e. an element that is expressed as "about 5%" may be actually 5%+/- 1%, or from 4% to 6%, depending on the embodiment. Only the variations that would be rational to one of ordinary skill in the art are contemplated herein. For example, where the element itself is expressed as a small percent, and a person of ordinary skill would know that the element is not rational to go below 0, the variations contemplated would not go below zero (i.e. about 5% could mean 5% +/-5% or 0-10%, but not 5% +/-10% or -5% to 15%, where this is not reasonable to one of skill in the art for the element being described).

## Claims

1. A device comprising:
a medical device for delivering nanoparticles of an active agent to a treatment site; and
a removable coating on the medical device comprising the active agent nanoparticles, wherein the active agent is a macrolide immunosuppressive drug, at least 95% of which is in crystalline form, and which active agent nanoparticles are, on average, at most 1 micrometer, at least a portion of the coating being deliverable to the treatment site which portion releases active agent nanoparticles into the treatment site over at least about 1 day, and wherein preparing the coating comprises depositing poly(lactide-co-glycolide) (PLGA), followed by depositing the active agent nanoparticles, wherein the coating further comprises a cationic binding agent in a target ratio of active agent:cationic binding agent of 1:1 to 10:1, wherein the actual ratio on the device is ±30% of the target ratio.

2. The device of claim 1, wherein the coating comprises a positive surface charge on a surface of the coating configured to contact the treatment site.

3. The device of claim 2, wherein the positive surface charge is at least one of: at least 1 mV, over 1 mV, at least 5 mV, at least 10 mV, 10 mV to 50 mV, 20 mV to 50 mV, 10 mV to 40 mV, 30 mV to 40 mV, 20 mV to 30 mV, 20 mV to 40 mV, and 25 mV to 35 mV.

4. The device of claim 1, wherein the cationic binding agent is selected from Polyarginine, Polyarginine 9-L-pArg, DEAE-Dextran (Diethylaminoethyl cellulose-Dextran), DMAB (Didodecyldimethylammonium bromide), PEI (Polyethyleneimine), TAB (Tetradodecylammonium bromide), or DMTAB (Dimethylditetradecylammonium bromide).

## Patentansprüche

1. Vorrichtung, umfassend:
eine medizinische Vorrichtung zur Zuführung von Nanopartikeln eines Wirkstoffs an eine Behandlungsstelle; und
eine entfernbare Beschichtung auf der medizinischen Vorrichtung, welche die Wirkstoff-Nanopartikel umfasst, wobei der Wirkstoff ein Makrolid-immunsuppressiver Arzneistoff ist, von dem mindestens 95% in kristalliner Form vorliegen, und wobei die Wirkstoff-Nanopartikel im Durchschnitt höchstens 1 Mikrometer sind, wobei mindestens ein Teil der Beschichtung an die Behandlungsstelle abgegeben werden kann, wobei dieser Teil Wirkstoff-Nanopartikel über mindestens etwa 1 Tag in die Behandlungsstelle freisetzt, und wobei die Herstellung der Beschichtung das Aufbringen von Poly(lactid-co-glycolid) (PLGA) und anschließend das Abscheiden der Wirkstoff-Nanopartikel umfasst, wobei die Beschichtung weiterhin ein kationisches Bindemittel in einem Zielverhältnis von Wirkstoff: kationischem Bindemittel von 1:1 bis 10:1 umfasst, wobei das tatsächliche Verhältnis auf der Vorrichtung ±30% des Zielverhältnisses beträgt.

2. Vorrichtung nach Anspruch 1, wobei die Beschichtung eine positive Oberflächenladung auf einer Oberfläche der Beschichtung umfasst, die eingerichtet ist um die Behandlungsstelle zu berühren.

3. Vorrichtung nach Anspruch 2, wobei die positive Oberflächenladung mindestens eine der Folgenden ist: mindestens 1 mV, über 1 mV, mindestens 5 mV, mindestens 10 mV, 10 mV bis 50 mV, 20 mV bis 50 mV, 10 mV bis 40 mV, 30 mV bis 40 mV, 20 mV bis 30 mV, 20 mV bis 40 mV und 25 mV bis 35 mV.

4. Vorrichtung nach Anspruch 1, wobei das kationische Bindemittel ausgewählt ist aus Polyarginin, Polyarginin 9-L-pArg, DEAE-Dextran (Diethylaminoethylcellulose-Dextran), DMAB (Didodecyldimethylammoniumbromid), PEI (Polyethylenimin), TAB (Tetradodecylammoniumbromid) oder DMTAB (Dimethylditetradecylammoniumbromid).

## Revendications

1. Dispositif comprenant :
un dispositif médical pour distribuer des nanoparticules d'un principe actif à un site de traitement ; et un revêtement amovible sur le dispositif médical comprenant les nanoparticules de principe actif, dans lequel le principe actif est un médicament immunosuppresseur macrolide, dont au moins 95 % est sous forme cristalline, et lesquelles nanoparticules de principe actif mesurent, en moyenne, au plus 1 micromètre, au moins une partie du revêtement pouvant être administrée au site de traitement dont une partie libère des nanoparticules de principe actif dans le site de traitement sur au moins environ 1 jour, et dans lequel la préparation du revêtement comprend le dépôt de poly(lactide-co-glycolide) (PLGA), suivi du dépôt des nanoparticules de principe actif, dans lequel le revêtement comprend en outre un agent de liaison selon un rapport cible de principe actif:agent de liaison cationique de 1:1 à 10:1, dans lequel le rapport réel sur le dispositif est de ± 30 % du rapport cible.

2. Dispositif selon la revendication 1, dans lequel le revêtement comprend une charge de surface positive sur une surface du revêtement conçue pour entrer en contact avec le site de traitement.

3. Dispositif selon la revendication 2, dans lequel la charge de surface positive est au moins l'une de : au moins 1 mV, plus de 1 mV, au moins 5 mV, au moins 10 mV, 10 mV à 50 mV, 20 mV à 50 mV, 10 mV à 40 mV, 30 mV à 40 mV, 20 mV à 30 mV, 20 mV à 40 mV et 25 mV à 35 mV.

4. Dispositif selon la revendication 1, dans lequel l'agent de liaison cationique est choisi parmi la polyarginine, la polyarginine 9-L-pArg, le DEAE-dextrane (diéthylaminoéthyl cellulose-dextrane), le DMAB (bromure de didodécyldiméthylammonium), la PEI (polyéthylèneimine), le TAB (bromure de tétradodécylammonium), ou le DMTAB (bromure de diméthylditétradécylammonium).
